# EUROPEAN PATENT APPLICATION

(11) **EP 4 151 752 A1**
(43) Date of publication of application: **22.03.2023**
(21) Application number: 21805028.4
(22) Date of filing: 14.05.2021
(51) Int. Cl.: C12Q 1/6832, G01N 33/50

(54) **METHOD FOR DETECTING PARKINSON'S DISEASE**

(30) Priority: 14.05.2020 JP 2020085430
(71) Applicant: Kao Corporation, Chuo-ku Tokyo 103-8210 (JP); Juntendo Educational Foundation, Tokyo 113-8421 (JP)
(72) Inventor: UEHARA, Yuya, Haga-gun, Tochigi 321-3497 (JP); INOUE, Takayoshi, Haga-gun, Tochigi 321-3497 (JP); HATTORI, Nobutaka, Tokyo 113-8421 (JP); SAIKI, Shinji, Tokyo 113-8421 (JP); UENO, Shin-Ichi, Tokyo 113-8421 (JP); TAKESHIGE, Haruka, Tokyo 113-8421 (JP)
(74) Representative: Vossius & Partner Patentanwälte Rechtsanwälte mbB
(86) International application number: PCT/JP2021/018511
(87) International publication number: WO 2021/230379

(57) **Abstract**

Provided are a marker gene for detecting Parkinson's disease, and a method for detecting Parkinson's disease by using the marker gene. The method for detecting Parkinson's disease in a test subject comprises a step of measuring an expression level of at least one gene selected from the group of 4 genes consisting of SNORA16A, SNORA24, SNORA50 and REXO1L2P or an expression product thereof in a biological sample collected from the test subject.

## Description

### Field of the Invention

The present invention relates to a method for detecting Parkinson's disease by using a Parkinson's disease marker.

### Background of the Invention

Parkinson's disease is pathologically a progressive neurodegenerative disease composed mainly of the formation of Lewy body having **α**-synuclein aggregates as a main component, the degeneration of dopaminergic neurons in the substantia nigra of the midbrain, and cell death, and is clinically a disease composed mainly of movement disorder such as muscle stiffness, tremor, hypokinesis, or gait disturbance.

Parkinson's disease is the second most common neurodegenerative disease after Alzheimer's disease. Its morbidity prevalence rate is 120 to 130 per 100,000 people, and it is estimated that there are approximately 140,000 patients in Japan.

At present, there exists no definitive therapy for Parkinson's disease. It is considered important for QOL maintenance to control symptoms by symptomatic therapy based on the supplementation of L-DOPA or the like.

However, subjective symptoms of movement disorder appear in an intermediate stage thereof or later. Thus, there is a demand for early diagnosis and early intervention of the disease.

For example, the detection of **α**-synuclein accumulation as well as the detection of microRNA derived from circulating serum (Patent Literature 1) and the measurement of the concentration ratio of tyrosine to phenylalanine in blood (Patent Literature 2) have been proposed as biomarkers for detecting Parkinson's disease. It has also been reported that: the formation of **α-**synuclein aggregates is observed in the skin, as in the brain, of Parkinson's disease patients (Non Patent Literature 1); and Parkinson's disease patients manifest skin diseases or symptoms such as seborrheic dermatitis, melanoma, bullous pemphigoid, or rosacea (Non Patent Literature 2). Although it is also considered that skin conditions are related in some way to Parkinson's disease, its scientific relation is totally unknown.

Meanwhile, techniques of examining current or future physiological states *in vivo* in humans by the analysis of nucleic acids such as DNA or RNA in biological samples have been developed. The analysis using nucleic acids has the advantages that: exhaustive analysis methods have already been established and abundant information can be obtained by one analysis; and the functional connection of analysis results is easily performed on the basis of many research reports on single-nucleotide polymorphism, RNA functions, and the like. Nucleic acids derived from a biological origin can be extracted from body fluids such as blood, secretions, tissues, and the like. It has recently been reported that: RNA contained in skin surface lipids (SSL) can be used as a biological sample for analysis; and marker genes of the epidermis, the sweat gland, the hair follicle and the sebaceous gland can be detected from SSL (Patent Literature 3).
(Patent Literature 1) JP-A-2019-506183
(Patent Literature 2) JP-A-2016-75644
(Patent Literature 3) WO 2018/008319
(Non Patent Literature 1) Rodriguez-Leyva I et al. Ann Clin Transl Neurol. 2014 (modified)
(Non Patent Literature 2) Ravn AH et al. Clin Cosmet Investig Dermatol. 2017

### Summary of the Invention

The present invention relates to the following 1) to 3) .
1) A method for detecting Parkinson's disease in a test subject, comprising a step of measuring an expression level of at least one gene selected from the group of 4 genes consisting of SNORA16A, SNORA24, SNORA50 and REXO1L2P or an expression product thereof in a biological sample collected from the test subject.
2) A test kit for detecting Parkinson's disease, the kit being used in a method according to 1), and comprising an oligonucleotide which specifically hybridizes to the gene, or an antibody which recognizes an expression product of the gene.
3) A marker for detecting Parkinson's disease comprising at least one gene selected from the groups of genes shown in Tables 3-1 to 3-4 and Tables 6-1 and 6-2 or an expression product thereof.

### Brief Description of Drawing

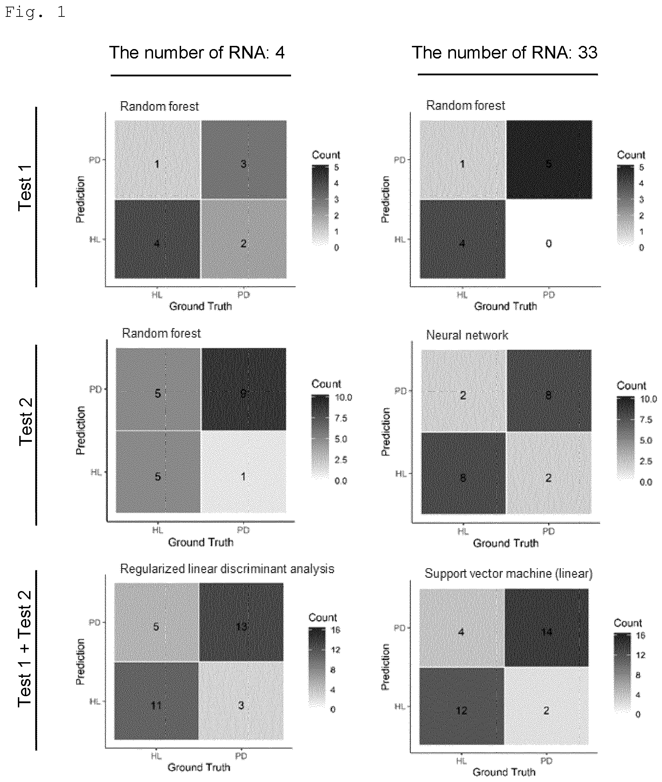

[Figure 1] Figure 1 shows confusion matrix in which predictive values in the optimum prediction model and actually measured values were plotted in test data.

### Detailed Description of the Invention

The present invention relates to a provision of a marker for detecting Parkinson's disease and a method for detecting Parkinson's disease by using the marker.

The present inventors collected SSL from the skin of Parkinson's disease patients and healthy subjects and exhaustively analyzed the expression state of RNA contained in the SSL as sequence information, and consequently found that the expression levels of particular genes significantly differ therebetween and Parkinson's disease can be detected on the basis of this index.

The present invention enables Parkinson's disease to be conveniently and noninvasively detected in an early stage with high accuracy, sensitivity and specificity.

All patent literatures, non patent literatures, and other publications cited herein are incorporated herein by reference in their entirety.

In the present invention, the term "nucleic acid" or "polynucleotide" means DNA or RNA. The DNA includes all of cDNA, genomic DNA, and synthetic DNA. The "RNA" includes all of total RNA, mRNA, rRNA, tRNA, non-coding RNA and synthetic RNA.

In the present invention, the "gene" encompasses double-stranded DNA including human genomic DNA as well as single-stranded DNA including cDNA (positive strand), single-stranded DNA having a sequence complementary to the positive strand (complementary strand), and their fragments, and means matter containing some biological information in sequence information on bases constituting DNA.

The "gene" encompasses not only a "gene" represented by a particular nucleotide sequence but a nucleic acid encoding a congener (i.e., a homolog or an ortholog), a variant such as gene polymorphism, and a derivative thereof.

The names of genes disclosed herein follow Official Symbol described in NCBI ([www.ncbi.nlm.nih.gov/]). Meanwhile, gene ontology (GO) follows Pathway ID. described in String ([string-db.org/]).

In the present invention, the "expression product" of a gene conceptually encompasses a transcription product and a translation product of the gene. The "transcription product" is RNA resulting from the transcription of the gene (DNA), and the "translation product" means a protein which is encoded by the gene and translationally synthesized on the basis of the RNA.

In the present invention, the "Parkinson's disease" means an idiopathic and progressive disease which has the degeneration of dopaminergic neurons in the substantia nigra pars compacta as a main lesion and manifests three motor symptoms (tremor at rest, rigidity, and bradykinesia or akinesia) in a slowly progressive manner.

In the present invention, the "detection" of Parkinson's disease means to elucidate the presence or absence of Parkinson's disease and may be used interchangeably with the term "test", "measurement", "determination", "evaluation" or "assistance of evaluation". In the present specification, the term "determination" or "evaluation" does not include determination or evaluation by a physician.

The 4 genes consisting of SNORA16A, SNORA24, SNORA50 and REXO1L2P according to the present invention are genes selected from the 33 genes described in Table A given below for which the expression level of SSL-derived RNA was found to be significantly increased (UP) or decreased (DOWN) in Parkinson's disease patients compared with healthy subjects, as shown in Examples mentioned later. The 4 genes are genes whose relation to Parkinson's disease has previously been unknown (indicated by boldface in the table).

**[Table A]**

| Symbol | p value (Test 1) | p value (Test 2) | Regulation |
|---|---|---|---|
| ANKRD12 | 0.010769 | 0.022801 | DOWN |
| C10orf116 | 0.027915 | 0.039587 | UP |
| CCL3 | 0.008678 | 0.028353 | DOWN |
| CCNI | 0.004032 | 0.027019 | DOWN |
| CD83 | 0.041752 | 0.029159 | DOWN |
| CNFN | 0.024347 | 1.85E-05 | UP |
| CNN2 | 0.023711 | 0.045042 | DOWN |
| CSF2RB | 0.020573 | 0.047037 | DOWN |
| CXCR4 | 0.000244 | 0.020358 | DOWN |
| EGR2 | 0.005989 | 0.033983 | DOWN |
| EMP1 | 0.010452 | 0.000953 | UP |
| ITGAX | 0.027931 | 0.014582 | DOWN |
| KCNQ10T1 | 0.0414 | 0.015544 | UP |
| LCE3D | 0.01773 | 0.000578 | UP |
| LITAF | 0.014086 | 0.02915 | DOWN |
| NDUFA4L2 | 0.047011 | 2.72E-05 | UP |
| NDUFS5 | 0.028286 | 0.011341 | UP |
| POLR2L | 0.005102 | 0.0376 | UP |
| **REXO1L2P** | 0.021096 | 0.016022 | UP |
| RHOA | 0.003152 | 0.004939 | DOWN |
| RNASEK | 0.030621 | 0.046581 | DOWN |
| RPL7A | 0.040024 | 0.003107 | UP |
| RPS26 | 0.020174 | 0.015282 | UP |
| SERINC1 | 0.046063 | 0.011959 | DOWN |
| SERP1 | 0.033858 | 0.027307 | DOWN |
| SERPINB4 | 0.048165 | 0.009405 | UP |
| SLC25A3 | 0.040817 | 0.031602 | UP |
| **SNORA16A** | 0.005217 | 3.37E-05 | UP |
| **SNORA24** | 0.001017 | 0.00062 | UP |
| **SNORA50** | 0.010607 | 0.004445 | UP |
| SNRPG | 0.002506 | 0.003904 | UP |
| SRRM2 | 0.036848 | 0.010131 | DOWN |
| UQCRH | 0.01058 | 0.030619 | UP |

33 genes shown in Table A were obtained by converting data (read count values) on the expression level of RNA extracted from SSL of test subjects of two tests (Test 1: 15 healthy subjects and 15 Parkinson's disease patients, Test 2: 50 healthy subjects and 50 Parkinson's disease patients) to RPM values which normalize the read count values for difference in the total number of reads among samples, identifying RNA (Test 1: 111 genes with increased expression and 68 genes with decreased expression (a total of 179 gene, Tables 1-1 to 1-5), Test 2: 565 genes with increased expression and 294 genes with decreased expression (a total of 859 gene, Tables 1-6 to 1-27) which attained a p value of 0.05 or less in Student's t-test in Parkinson's disease patients compared with healthy subjects on the basis of values obtained by the conversion of the RPM values to logarithmic values to base 2 (Log₂RPM values), and selecting common genes with increased expression (18 genes) and genes with decreased expression (15 genes) between Test 1 and Test 2.

Thus, a gene selected from the group consisting of the 179 genes and the 859 genes (a total of 1,005 genes except for duplication) or an expression product thereof is capable of serving as a Parkinson's disease marker for detecting Parkinson's disease. Among them, a gene selected from the group consisting of 33 genes shown in Table A or an expression product thereof is a preferred Parkinson's disease marker.

In Table A and Table 1 mentioned later, the "p value" refers to the probability of observing extreme statistics based on statistics actually calculated from data under null hypothesis in a statistical test. Thus, a smaller "p value" can be regarded as more significant difference between objects to be compared.

Genes represented by "UP" are genes whose expression level is increased in Parkinson's disease patients, and genes represented by "DOWN" are genes whose expression level is decreased in Parkinson's disease patients.

The group of the differentially expressed genes described above was found to include genes related to Parkinson's disease (hsa05012) in search for a biological process (BP) and a KEGG pathway by gene ontology (GO) enrichment analysis (see Table 2 mentioned later). Meanwhile, in the group of the differentially expressed genes described above, genes shown in Tables 3-1 to 3-4 mentioned later are genes whose relation to Parkinson's disease has not been reported so far. Thus, at least one gene selected from the group consisting of these genes or an expression product thereof is a novel Parkinson's disease marker for detecting Parkinson's disease. Particularly, at least one gene selected from the group consisting of SNORA16A, SNORA24, SNORA50 and REXO1L2P which are common between Test 1 and Test 2, or an expression product thereof is preferred as a novel Parkinson's disease marker. Two or more genes selected from the group are more preferred, three or more genes selected therefrom are further more preferred, and all of the four genes are even more preferred. It is also preferred to include at least SNORA24, which is included in common in Table A described above and Table B mentioned later.

Differentially expressed RNA may be identified from data (read count values) on the expression level of RNA by using normalized count values obtained by using, for example, DESeq2 (Love MI et al., Genome Biol. 2014) or logarithmic values to base 2 of the count value plus integer 1 (Log₂(count + 1) value).

For example, RNA which attains a corrected p value (FDR) of 0.25 or less in a likelihood ratio test in Parkinson's disease patients compared with healthy subjects is identified by using normalized count values as data on the expression level of RNA extracted from SSL of test subjects of the two tests mentioned above. As a result, 74 genes with increased expression, 209 genes with decreased expression, and a total of 283 genes (Tables 4-1 to 4-8) are obtained in Test 1, and 151 genes with increased expression, 308 genes with decreased expression, and a total of 459 genes (Tables 4-9 to 4-20) are obtained in Test 2. The expression of 7 genes is increased in common between Test 1 and Test 2 (ANXA1, AQP3, EMP1, KRT16, POLR2L, SERPINB4, and SNORA24), and the expression of 10 genes is decreased in common therebetween (ATP6V0C, BHLHE40, CCL3, CCNI, CXCR4, EGR2, GABARAPL1, RHOA, RNASEK, and SERINC1) (a total of 17 genes, Table B).

Thus, a gene selected from the group consisting of the 283 genes and the 459 genes (a total of 725 genes except for duplication) or an expression product thereof is capable of serving as a Parkinson's disease marker for detecting Parkinson's disease. Among them, a gene selected from the group consisting of the 17 genes shown in Table B or an expression product thereof is a preferred Parkinson's disease marker. Among them, a gene selected from the group consisting of 11 genes shown in Table C mentioned later, which are common with the genes shown in Table A described above, or an expression product thereof is a more preferred Parkinson's disease marker.

In the group of the differentially expressed genes described above, genes shown in Tables 6-1 and 6-2 mentioned later are genes whose relation to Parkinson's disease has not been reported so far. Thus, at least one gene selected from the group consisting of these genes or an expression product thereof is a novel Parkinson's disease marker for detecting Parkinson's disease. Particularly, SNORA24 (indicated by boldface in the table) which is common between Test 1 and Test 2 or an expression product thereof is preferred as a novel Parkinson's disease marker.

**[Table B]**

| Symbol | FDR (Test 1) | FDR (Test 2) | Regulation |
|---|---|---|---|
| ANXA1 | 0.032013 | 0.014395 | UP |
| AQP3 | 0.207454 | 0.197196 | UP |
| ATP6V0C | 0.142105 | 0.029799 | DOWN |
| BHLHE40 | 0.003239 | 0.189294 | DOWN |
| CCL3 | 0.022303 | 0.019217 | DOWN |
| CCNI | 8.89E-05 | 0.191526 | DOWN |
| CXCR4 | 0.024085 | 0.097541 | DOWN |
| EGR2 | 0.166431 | 0.179929 | DOWN |
| EMP1 | 0.060302 | 0.00062 | UP |
| GABARAPL1 | 0.060302 | 0.028215 | DOWN |
| KRT16 | 0.157035 | 0.203917 | UP |
| POLR2L | 0.205453 | 0.070687 | UP |
| RHOA | 0.166431 | 0.114613 | DOWN |
| RNASEK | 0.134092 | 0.189824 | DOWN |
| SERINC1 | 0.073126 | 0.233337 | DOWN |
| SERPINB4 | 0.093219 | 0.142882 | UP |
| **SNORA24** | 0.022726 | 0.249405 | UP |

The gene capable of serving as a Parkinson's disease marker (hereinafter, also referred to as a "target gene") also encompasses a gene having a nucleotide sequence substantially identical to the nucleotide sequence of DNA constituting the gene, as long as the gene is capable of serving as a biomarker for detecting Parkinson's disease. In this context, the nucleotide sequence substantially identical means a nucleotide sequence having 90% or higher, preferably 95% or higher, more preferably 98% or higher, further more preferably 99% or higher identity to the nucleotide sequence of DNA constituting the gene, for example, when searched by using homology calculation algorithm NCBI BLAST under conditions of expectation value = 10; gap accepted; filtering = ON; match score = 1; and mismatch score = -3.

The method for detecting Parkinson's disease according to the present invention includes a step of measuring an expression level of a target gene, which is in one aspect, at least one gene selected from the group consisting of SNORA16A, SNORA24, SNORA50 and REXO1L2P or an expression product thereof in a biological sample collected from a test subject.

In the method for detecting Parkinson's disease according to the present invention, examples of the test subject from which the biological sample is collected include mammals including humans and nonhuman mammals. A human is preferred. When the test subject is a human, the human is not particularly limited by sex, age, race, and the like thereof and can include infants to elderly people. Preferably, the test subject is a human who needs or desires detection of Parkinson's disease. The test subject is, for example, a human suspected of developing Parkinson's disease or a human having a genetic predisposition to develop Parkinson's disease.

The biological sample used in the present invention can be a tissue or a biomaterial in which the expression of the gene of the present invention varies with the onset or progression of Parkinson's disease. Examples thereof specifically include organs, skin, blood, urine, saliva, sweat, stratum corneum, skin surface lipids (SSL), body fluids such as tissue exudates, serum, plasma and others prepared from blood, feces, and hair, and preferably include the skin, the stratum corneum and skin surface lipids (SSL), more preferably skin surface lipids (SSL). Examples of the site of the skin from which SSL is collected include, but are not particularly limited to, the skin at an arbitrary site of the body, such as the head, the face, the neck, the body trunk, and the limbs. The skin at a site with high sebum secretion, for example, the skin of the head or the face, is preferred, and facial skin is more preferred.

In this context, the "skin surface lipids (SSL)" refer to a lipid-soluble fraction present on skin surface, and is also referred to as sebum. In general, SSL mainly contains secretion secreted from the exocrine gland such as the sebaceous gland in the skin, and is present on skin surface in the form of a thin layer that covers the skin surface. SSL contains RNA expressed in skin cells (see Patent Literature 3 described above). In the present specification, the "skin" is a generic name for regions containing tissues such as the stratum corneum, the epidermis, the dermis, and the hair follicle as well as the sweat gland, the sebaceous gland and other glands, unless otherwise specified.

Any approach for use in the recovery or removal of SSL from the skin can be adopted for the collection of SSL from the skin of a test subject. Preferably, an SSL-absorbent material or an SSL-adhesive material mentioned later, or a tool for scraping off SSL from the skin can be used. The SSL-absorbent material or the SSL-adhesive material is not particularly limited as long as the material has affinity for SSL. Examples thereof include polypropylene and pulp. More detailed examples of the procedure of collecting SSL from the skin include a method of allowing SSL to be absorbed to a sheet-like material such as an oil blotting paper or an oil blotting film, a method of allowing SSL to adhere to a glass plate, a tape, or the like, and a method of recovering SSL by scraping with a spatula, a scraper, or the like. In order to improve the adsorbability of SSL, an SSL-absorbent material impregnated in advance with a solvent having high lipid solubility may be used. On the other hand, the SSL-absorbent material preferably has a low content of a solvent having high water solubility or water because the adsorption of SSL to a material containing the solvent having high water solubility or water is inhibited. The SSL-absorbent material is preferably used in a dry state. Examples of the site of the skin from which SSL is collected include, but are not particularly limited to, the skin at an arbitrary site of the body, such as the head, the face, the neck, the body trunk, and the limbs. A site having high secretion of sebum, for example, the facial skin, is preferred.

The RNA-containing SSL collected from the test subject may be preserved for a given period. The collected SSL is preferably preserved under low-temperature conditions as rapidly as possible after collection in order to minimize the degradation of contained RNA. The temperature conditions for the preservation of RNA-containing SSL according to the present invention can be 0°C or lower and are preferably from -20 ± 20°C to -80 ± 20°C, more preferably from -20 ± 10°C to -80 ± 10°C, further more preferably from -20 ± 20°C to -40 ± 20°C, further more preferably from -20 ± 10°C to -40 ± 10°C, further more preferably -20 ± 10°C, further more preferably -20 ± 5°C. The period of preservation of the RNA-containing SSL under the low-temperature conditions is not particularly limited and is preferably 12 months or shorter, for example, 6 hours or longer and 12 months or shorter, more preferably 6 months or shorter, for example, 1 day or longer and 6 months or shorter, further more preferably 3 months or shorter, for example, 3 days or longer and 3 months or shorter.

In the present invention, examples of the measurement object for the expression level of a target gene or an expression product thereof include cDNA artificially synthesized from RNA, DNA encoding the RNA, a protein encoded by the RNA, a molecule which interacts with the protein, a molecule which interacts with the RNA, and a molecule which interacts with the DNA. In this context, examples of the molecule which interacts with the RNA, the DNA or the protein include DNA, RNA, proteins, polysaccharides, oligosaccharides, monosaccharides, lipids, fatty acids, and their phosphorylation products, alkylation products, and sugar adducts, and complexes of any of them. The expression level comprehensively means the expression level or activity of the gene or the expression product.

In a preferred aspect, in the method of the present invention, SSL is used as a biological sample. In this case, the expression level of RNA contained in SSL is analyzed. Specifically, RNA is converted to cDNA through reverse transcription, followed by the measurement of the cDNA or an amplification product thereof.

In the extraction of RNA from SSL, a method which is usually used in RNA extraction or purification from a biological sample, for example, phenol/chloroform method, AGPC (acid guanidinium thiocyanate-phenol-chloroform extraction) method, a method using a column such as TRIzol(R), RNeasy(R), or QIAzol(R), a method using special magnetic particles coated with silica, a method using magnetic particles for solid phase reversible immobilization, or extraction with a commercially available RNA extraction reagent such as ISOGEN can be used.

In the reverse transcription, primers which target particular RNA to be analyzed may be used, and random primers are preferably used for more comprehensive nucleic acid preservation and analysis. In the reverse transcription, common reverse transcriptase or reverse transcription reagent kit can be used. Highly accurate and efficient reverse transcriptase or reverse transcription reagent kit is suitably used. Examples thereof include M-MLV reverse transcriptase and its modified forms, and commercially available reverse transcriptase or reverse transcription reagent kits, for example, PrimeScript(R) Reverse Transcriptase series (Takara Bio Inc.) and Superscript(R) Reverse Transcriptase series (Thermo Fisher Scientific, Inc.). Superscript(R) III Reverse Transcriptase, Superscript(R) VILO cDNA Synthesis kit (both from Thermo Fisher Scientific, Inc.), and the like are preferably used.

The temperature of extension reaction in the reverse transcription is adjusted to preferably 42°C ± 1°C, more preferably 42°C ± 0.5°C, further more preferably 42°C ± 0.25°C, while its reaction time is adjusted to preferably 60 minutes or longer, more preferably from 80 to 120 minutes.

In the case of using RNA, cDNA or DNA as a measurement object, the method for measuring the expression level can be selected from nucleic acid amplification methods typified by PCR using DNA primers which hybridize thereto, real-time RT-PCR, multiplex PCR, SmartAmp, and LAMP, hybridization using a nucleic acid probe which hybridizes thereto (DNA chip, DNA microarray, dot blot hybridization, slot blot hybridization, Northern blot hybridization, and the like), a method of determining a nucleotide sequence (sequencing), and combined methods thereof.

In PCR, only particular DNA to be analyzed may be amplified by using a primer pair which targets the particular DNA, or a plurality of DNAs may be amplified by using a plurality of primer pairs. Preferably, the PCR is multiplex PCR. The multiplex PCR is a method of amplifying a plurality of gene regions at the same time by using a plurality of primer pairs at the same time in a PCR reaction system. The multiplex PCR can be carried out by using a commercially available kit (e.g., Ion AmpliSeq Transcriptome Human Gene Expression Kit; Life Technologies Japan Ltd.).

The temperature of annealing and extension reaction in the PCR depends on the primers used and therefore cannot be generalized. In the case of using the multiplex PCR kit described above, the temperature is preferably 62°C ± 1°C, more preferably 62°C ± 0.5°C, further more preferably 62°C ± 0.25°C. Thus, preferably, the annealing and the extension reaction are performed by one step in the PCR. The time of the step of the annealing and the extension reaction can be adjusted depending on the size of DNA to be amplified, and the like, and is preferably from 14 to 18 minutes. Conditions for denaturation reaction in the PCR can be adjusted depending on the DNA to be amplified, and are preferably from 95 to 99°C and from 10 to 60 seconds. The reverse transcription and the PCR using the temperatures and the times as described above can be carried out by using a thermal cycler which is generally used for PCR.

The reaction product obtained by the PCR is preferably purified by the size separation of the reaction product. By the size separation, the PCR reaction product of interest can be separated from the primers and other impurities contained in the PCR reaction solution. The size separation of DNA can be performed by using, for example, a size separation column, a size separation chip, or magnetic beads which can be used in size separation. Preferred examples of the magnetic beads which can be used in size separation include magnetic beads for solid phase reversible immobilization (SPRI) such as Ampure XP.

The purified PCR reaction product may be subjected to further treatment necessary for conducting subsequent quantitative analysis. For example, for DNA sequencing, the purified PCR reaction product may be prepared into an appropriate buffer solution, the PCR primer regions contained in DNA amplified by PCR may be cleaved, and an adaptor sequence may be further added to the amplified DNA. For example, the purified PCR reaction product can be prepared into a buffer solution, and the removal of the PCR primer sequences and adaptor ligation can be performed for the amplified DNA. If necessary, the obtained reaction product can be amplified to prepare a library for quantitative analysis. These operations can be performed, for example, by using 5 × VILO RT Reaction Mix attached to Superscript(R) VILO cDNA Synthesis kit (Life Technologies Japan Ltd.), 5 × Ion AmpliSeq HiFi Mix attached to Ion AmpliSeq Transcriptome Human Gene Expression Kit (Life Technologies Japan Ltd.), and Ion AmpliSeq Transcriptome Human Gene Expression Core Panel according to a protocol attached to each kit.

In the case of measuring the expression level of a target gene or a nucleic acid derived therefrom by use of Northern blot hybridization, examples thereof include a method in which; probe DNA is first labeled with a radioisotope, a fluorescent material, or the like. Subsequently, the obtained labeled DNA is allowed to hybridize to biological sample-derived RNA transferred to a nylon membrane or the like in accordance with a routine method. Then, the formed duplex of the labeled DNA and the RNA can be measured by detecting a signal derived from the label.

In the case of measuring the expression level of a target gene or a nucleic acid derived therefrom by use of RT-PCR, for example, cDNA is first prepared from biological sample-derived RNA in accordance with a routine method. This cDNA is used as a template, and a pair of primers (a positive strand which binds to the cDNA (- strand) and an opposite strand which binds to a + strand) prepared so as to be able to amplify the target gene of the present invention is allowed to hybridize thereto. Then, PCR is performed in accordance with a routine method, and the obtained amplified double-stranded DNA is detected. In the detection of the amplified double-stranded DNA, for example, a method of detecting labeled double-stranded DNA produced by the PCR by using primers labeled in advance with RI, a fluorescent material, or the like can be used.

In the case of measuring the expression level of a target gene or a nucleic acid derived therefrom by use of a DNA microarray, for example, an array in which at least one nucleic acid (cDNA or DNA) derived from the target gene of the present invention is immobilized on a support is used. Labeled cDNA or cRNA prepared from mRNA is allowed to bind onto the microarray, and the expression level of the mRNA can be measured by detecting the label on the microarray.

The nucleic acid to be immobilized in the array can be a nucleic acid which specifically (i.e., substantially only to the nucleic acid of interest) hybridizes under stringent conditions, and may be, for example, a nucleic acid having the whole sequence of the target gene of the present invention or may be a nucleic acid consisting of a partial sequence thereof. In this context, examples of the "partial sequence" include nucleic acids consisting of at least 15 to 25 bases. In this context, examples of the stringent conditions can usually include washing conditions on the order of "1 × SSC, 0.1% SDS, and 37°C". Examples of the more stringent hybridization conditions can include conditions on the order of "0.5 × SSC, 0.1% SDS, and 42°C". Examples of the much more stringent hybridization conditions can include conditions on the order of "0.1 × SSC, 0.1% SDS, and 65°C". The hybridization conditions are described in, for example, J. Sambrook et al., Molecular Cloning: A Laboratory Manual, Third Edition, Cold Spring Harbor Laboratory Press (2001).

In the case of measuring the expression level of a target gene or a nucleic acid derived therefrom by sequencing, examples thereof include analysis using a next-generation sequencer (e.g., Ion S5/XL system, Life Technologies Japan Ltd.). RNA expression can be quantified on the basis of the number of reads (read count) prepared by the sequencing.

The probe or the primers for use in the measurement described above, which correspond to the primers for specifically recognizing and amplifying the target gene of the present invention or a nucleic acid derived therefrom, or the probe for specifically detecting the RNA or the nucleic acid derived therefrom, can be designed on the basis of a nucleotide sequence constituting the target gene. In this context, the phrase "specifically recognize" means that a detected product or an amplification product can be confirmed to be the gene or the nucleic acid derived therefrom in such a way that, for example, substantially only the target gene of the present invention or the nucleic acid derived therefrom can be detected in Northern blot, or, for example, substantially only the nucleic acid is amplified in RT-PCR.

Specifically, an oligonucleotide containing a given number of nucleotides complementary to DNA consisting of a nucleotide sequence constituting the target gene of the present invention, or a complementary strand thereof can be used. In this context, the "complementary strand" refers to one strand of double-stranded DNA consisting of A:T (U for RNA) and/or G:C base pairs with respect to the other strand. The term "complementary" is not limited by the case of being a completely complementary sequence in a region with the given number of consecutive nucleotides, and can have preferably 80% or higher, more preferably 90% or higher, further more preferably 95% or higher identity of the nucleotide sequence. The identity of the nucleotide sequence can be determined by algorithm such as BLAST described above.

For use as a primer, the oligonucleotide can achieve specific annealing and strand extension. Examples thereof usually include oligonucleotides having a strand length of 10 or more bases, preferably 15 or more bases, more preferably 20 or more bases, and 100 or less bases, preferably 50 or less bases, more preferably 35 or less bases. For use as a probe, the oligonucleotide can achieve specific hybridization. An oligonucleotide can be used which has at least a portion or the whole of the sequence of DNA (or a complementary strand thereof) consisting of a nucleotide sequence constituting the target gene of the present invention, and has a strand length of, for example, 10 or more bases, preferably 15 or more bases, and, for example, 100 or less bases, preferably 50 or less bases, more preferably 25 or less bases.

In this context, the "oligonucleotide" can be DNA or RNA and may be synthetic or natural. The probe for use in hybridization is usually labeled for use.

In the case of measuring a translation product (protein) of the target gene of the present invention, a molecule which interacts with the protein, a molecule which interacts with the RNA, or a molecule which interacts with the DNA, a method such as protein chip analysis, immunoassay (e.g., ELISA), mass spectrometry (e.g., LC-MS/MS and MALDI-TOF/MS), one-hybrid method (PNAS 100, 12271-12276 (2003)), or two-hybrid method (Biol. Reprod. 58, 302-311 (1998)) can be used and can be appropriately selected depending on the measurement object.

For example, in the case of using the protein as a measurement object, the measurement may be carried out by contacting an antibody against the expression product of the present invention with a biological sample, detecting a polypeptide in the sample bound to the antibody, and measuring the level thereof. For example, according to Western blot, the antibody described above is used as a primary antibody, and an antibody which binds to the primary antibody and which is labeled with, for example, a radioisotope, a fluorescent material or an enzyme is used as a secondary antibody to label the primary antibody therewith, followed by the measurement of a signal derived from such a labeling material using a radiation meter, a fluorescence detector, or the like.

The antibody against the translation product may be a polyclonal antibody or a monoclonal antibody. These antibodies can be produced in accordance with a method known in the art. Specifically, the polyclonal antibody may be produced by using a protein which has been expressed in *E. coli* or the like and purified in accordance with a routine method, or synthesizing a partial polypeptide of the protein in accordance with a routine method, and immunizing a nonhuman animal such as a house rabbit therewith, followed by obtainment from the serum of the immunized animal in accordance with a routine method.

Meanwhile, the monoclonal antibody can be obtained from hybridoma cells prepared by immunizing a nonhuman animal such as a mouse with a protein which has been expressed in *E. coli* or the like and purified in accordance with a routine method, or a partial polypeptide of the protein, and fusing the obtained spleen cells with myeloma cells. Alternatively, the monoclonal antibody may be prepared by use of phage display (Griffiths, A.D.; Duncan, A.R., Current Opinion in Biotechnology, Volume 9, Number 1, February 1998, pp. 102-108 (7)).

In this way, the expression level of the target gene of the present invention or the expression product thereof in a biological sample collected from a test subject is measured, and Parkinson's disease is detected on the basis of the expression level. The detection is specifically performed by comparing the measured expression level of the target gene of the present invention or the expression product thereof with a control level.

In the case of analyzing expression levels of a plurality of target genes by sequencing, as described above, read count values which are data on expression levels, RPM values which normalize the read count values for difference in the total number of reads among samples, values obtained by the conversion of the RPM values to logarithmic values to base 2 (Log₂RPM values), or normalized count values obtained by using DESeq2 or logarithmic values to base 2 of the count value plus integer 1 (Log₂(count + 1) values) are preferably used as an index. Also, values calculated by, for example, fragments per kilobase of exon per million reads mapped (FPKM), reads per kilobase of exon per million reads mapped (RPKM), or transcripts per million (TPM) which are general quantitative values of RNA-seq may be used. Alternatively, signal values obtained by microarray method or corrected values thereof may be used. In the case of analyzing only a particular target gene by RT-PCR or the like, an analysis method of converting the expression level of the target gene to a relative expression level with respect to the expression level of a housekeeping gene as a standard, or a method of analyzing a copy number obtained by absolute quantification using a plasmid containing a region of the target gene is preferred. A copy number obtained by digital PCR may be used.

In this context, examples of the "control level" include an expression level of the target gene or the expression product thereof in a healthy person. The expression level of the healthy person may be a statistic (e.g., a mean) of the expression level of the gene or the expression product thereof measured from a healthy person population. For a plurality of target genes, it is preferred to determine a standard expression level of each individual gene or expression product thereof.

The detection of Parkinson's disease according to the present invention may be performed through an increase and/or decrease in the expression level of the target gene of the present invention or the expression product thereof. In this case, the expression level of the target gene or the expression product thereof in a biological sample derived from a test subject is compared with a cutoff value (reference value) of each gene or the expression product thereof. The cutoff value can be appropriately determined on the basis of a statistical numeric value, such as a mean or standard deviation, of the expression level based on the expression level of the target gene or expression product thereof in a healthy subject obtained as a standard data.

A discriminant (prediction model) which discriminates between a Parkinson's disease patient and a healthy person is constructed by using measurement values of an expression level of the target gene or the expression product thereof derived from a Parkinson's disease patient and an expression level of the target gene or the expression product thereof derived from a healthy person, and Parkinson's disease can be detected through the use of the discriminant. Specifically, a discriminant (prediction model) which discriminates between a Parkinson's disease patient and a healthy person is constructed by using measurement values of an expression level of a target gene or an expression product thereof derived from a Parkinson's disease patient and an expression level of the target gene or the expression product thereof derived from a healthy subject as teacher samples, and a cutoff value (reference value) which discriminates between the Parkinson's disease patient and the healthy person is determined on the basis of the discriminant. In the preparation of the discriminant, dimensional compression is performed by principal component analysis (PCA), and a principal component can be used as an explanatory variable.

The presence or absence of Parkinson's disease in a test subject can be evaluated by similarly measuring a level of the target gene or the expression product thereof from a biological sample collected from the test subject, substituting the obtained measurement value into the discriminant, and comparing the results obtained from the discriminant with the reference value.

In this context, algorithm known in the art such as algorithm for use in machine learning can be used as the algorithm in the construction of the discriminant. Examples of the machine learning algorithm include random forest, linear kernel support vector machine (SVM linear), rbf kernel support vector machine (SVM rbf), neural network, generalized linear model, regularized linear discriminant analysis, and regularized logistic regression. A predictive value is calculated by inputting data for the verification of the constructed prediction model, and a model which attains the predictive value most compatible with an actually measured value, for example, recall, precision, and an F value which is a harmonic mean thereof are calculated from a predictive value and an actually measured value, and a model having the largest F value can be selected as the optimum prediction model.

The method for determining the cutoff value (reference value) is not particularly limited, and the value can be determined in accordance with an approach known in the art. The value can be determined from, for example, an ROC (receiver operating characteristic) curve prepared by using the discriminant. In the ROC curve, the probability (%) of producing positive results in positive patients (sensitivity) is plotted on the ordinate against a value (false positive rate) of 1 minus the probability (%) of producing negative results in negative patients (specificity) on the abscissa. As for "true positive (sensitivity)" and "false positive (1 - specificity)" shown in the ROC curve, a value at which "true positive (sensitivity)" - "false positive (1 - specificity)" is maximized (Youden index) can be used as the cutoff value (reference value).

As shown in Examples mentioned later, prediction models were constructed by use of machine learning algorithm by using a value of each principal component obtained from expression level data (Log₂RPM values) on target genes shown in Table A (33 genes or 4 genes selected therefrom) as an explanatory variable, and the healthy subjects and the Parkinson's disease patients as objective variables. As a result, Parkinson's disease was found predictable with the model by using the 4 genes SNORA16A, SNORA24, SNORA50, and REXO1L2P. Also, Parkinson's disease was found predictable more accurately with the model by using the 33 genes.

Thus, in the case of preparing the discriminant which discriminates between a Parkinson's disease patient group and a healthy person group, a discriminant which exhibits high recall and precision can be prepared by appropriately adding, to expression data on the 4 target genes SNORA16A, SNORA24, SNORA50 and REXO1L2P, expression data on at least one gene selected from the group consisting of the remaining 29 genes shown in Table A or an expression product thereof as a target gene, preferably adding thereto an appropriate number of genes with high variable importance based on variable importance shown in Table 8 mentioned later. Thus, Parkinson's disease can be detected with higher accuracy. Specifically, addition of 8 genes EGR2, RHOA, CCNI, RNASEK, CSF2RB, SERP1, ANKRD12, and SLC25A3 are preferred. Further, addition of 12 genes consisting of these 8 genes and 4 genes CD83, CXCR4, ITGAX, and UQCRH are preferred, and addition of 18 genes consisting of these 12 genes and 6 genes KCNQ1OT1, CCL3, C10orf116, SERPINB4, LCE3D, and CNFN are preferred. It is preferred to add all of the 29 genes.

Alternatively, expression data on at least one gene, except for SNORA24, selected from the group consisting of 11 genes which are shown as differentially expressed genes in both Table A and Table B described above, and shown in Table C given below, or an expression product thereof may be appropriately added as a target gene to the 4 target genes SNORA16A, SNORA24, SNORA50 and REXO1L2P.

**[Table C]**

| Symbol | Regulation |
|---|---|
| CCL3 | DOWN |
| CCNI | DOWN |
| CXCR4 | DOWN |
| EGR2 | DOWN |
| EMP1 | UP |
| POLR2L | UP |
| RHOA | DOWN |
| RNASEK | DOWN |
| SERINC1 | DOWN |
| SERPINB4 | UP |
| SNORA24 | UP |

Expression data on at least one gene selected from the group consisting of genes shown in Table B or an expression product thereof may be used as a target gene for use in preparing the discriminant which discriminates between a Parkinson's disease patient group and a healthy person group. Preferably, SNORA24 as well as at least one of the other genes is used. More preferably, expression data on genes shown in Table C or expression products thereof is used. Further more preferably, expression data on all the genes shown in Table B or expression products thereof is used.

The test kit for detecting Parkinson's disease according to the present invention contains a test reagent for measuring an expression level of the target gene of the present invention or an expression product thereof in a biological sample separated from a patient. Specific examples thereof include a reagent for nucleic acid amplification and hybridization containing an oligonucleotide (e.g., a primer for PCR) which specifically binds (hybridizes) to the target gene of the present invention or a nucleic acid derived therefrom, and a reagent for immunoassay containing an antibody which recognizes an expression product (protein) of the target gene of the present invention. The oligonucleotide, the antibody, or the like contained in the kit can be obtained by a method known in the art as mentioned above.

The test kit may contain, in addition to the antibody or the nucleic acid, a labeling reagent, a buffer solution, a chromogenic substrate, a secondary antibody, a blocking agent, an instrument necessary for a test, a control, a tool for collecting a biological sample (e.g., an oil blotting film for collecting SSL), and the like.

Aspects and preferred embodiments of the present invention will be given below.
<1> A method for detecting Parkinson's disease in a test subject, comprising a step of measuring an expression level of at least one gene selected from the group of 4 genes consisting of SNORA16A, SNORA24, SNORA50 and REXO1L2P or an expression product thereof in a biological sample collected from the test subject.
<2> The method for detecting Parkinson's disease according to <1>, wherein the method at least comprises measuring an expression level of SNORA24 gene or an expression product thereof.
<3> The method according to <1> or <2>, wherein the expression level of the gene or the expression product thereof is measured as an expression level of mRNA.
<4> The method according to any of <1> to <3>, wherein the gene or the expression product thereof is RNA contained in skin surface lipids of the test subject.
<5> The method according to any of <1> to <4>, wherein the presence or absence of Parkinson's disease is evaluated by comparing the measurement value of the expression level with a reference value of the gene or the expression product thereof.
<6> The method according to any of <1> to <4>, wherein the presence or absence of Parkinson's disease in the test subject is evaluated by the following steps: preparing a discriminant which discriminates between the Parkinson's disease patient and a healthy person by using measurement values of an expression level of the gene or the expression product thereof derived from a Parkinson's disease patient and an expression level of the gene or the expression product thereof derived from a healthy subject as teacher samples; substituting the measurement value of the expression level of the gene or the expression product thereof obtained from the biological sample collected from the test subject into the discriminant; and comparing the obtained results with a reference value.
<7> The method according to <6>, wherein expression levels of all the genes of the group of 4 genes or expression products thereof are measured.
<8> The method according to <6> or <7>, wherein expression levels of the at least one gene selected from the group of 4 genes as well as at least one gene selected from the following group of 29 genes or expression products thereof are measured:
   ANKRD12, C10orf116, CCL3, CCNI, CD83, CNFN, CNN2, CSF2RB, CXCR4, EGR2, EMP1, ITGAX, KCNQ1OT1, LCE3D, LITAF, NDUFA4L2, NDUFS5, POLR2L, RHOA, RNASEK, RPL7A, RPS26, SERINC1, SERP1, SERPINB4, SLC25A3, SNRPG, SRRM2, and UQCRH.
<9> The method according to <8>, wherein expression levels of the at least one gene selected from the group of 4 genes as well as at least one gene selected from the following group of 10 genes or expression products thereof are measured:
   CCL3, CCNI, CXCR4, EGR2, EMP1, POLR2L, RHOA, RNASEK, SERINC1, and SERPINB4.
<10> The method according to <6> or <7>, wherein expression levels of the at least one gene selected from the group of 4 genes as well as at least one gene selected from the following group of 16 genes or expression products thereof are measured:
   ANXA1, AQP3, ATP6V0C, BHLHE40, CCL3, CCNI, CXCR4, EGR2, EMP1, GABARAPL1, KRT16, POLR2L, RHOA, RNASEK, SERINC1, and SERPINB4.
<11> The method according to <6> or <7>, wherein expression levels of the at least one gene selected from the group of 4 genes as well as at least one gene selected from the groups of genes shown in Tables 3-1 to 3-4 mentioned later and Tables 6-1 and 6-2 mentioned later (except for the 4 genes) or expression products thereof are measured.
<12> The method according to <6> or <7>, wherein expression levels of the at least one gene selected from the group of 4 genes as well as at least one gene selected from the groups of 1,005 genes shown in Tables 1-1 to 1-27 mentioned later and 725 genes shown in Tables 4-1 to 4-20 mentioned later except for the 4 genes or expression products thereof are measured.
<13> A test kit for detecting Parkinson's disease, the kit being used in a method according to any of <1> to <10>, and comprising an oligonucleotide which specifically hybridizes to the gene or a nucleic acid derived therefrom, or an antibody which recognizes an expression product of the gene.
<14> Use of at least one gene selected from the groups of genes shown in Tables 3-1 to 3-4 mentioned later and Tables 6-1 and 6-2 mentioned later or an expression product thereof as a marker for detecting Parkinson's disease.
<15> Use of at least one gene selected from the group of 4 genes consisting of SNORA16A, SNORA24, SNORA50 and REXO1L2P or an expression product thereof as a marker for detecting Parkinson's disease.
<16> A marker for detecting Parkinson's disease comprising at least one gene selected from the groups of genes shown in Tables 3-1 to 3-4 mentioned later and Tables 6-1 and 6-2 mentioned later or an expression product thereof.
<17> The marker for detecting Parkinson's disease according to <16>, wherein the detection marker comprises at least one gene selected from the group of 4 genes consisting of SNORA16A, SNORA24, SNORA50 and REXO1L2P or an expression product thereof.

### Examples

Hereinafter, the present invention will be described in more detail with reference to Examples. However, the present invention is not limited by these examples.

Example 1 Detection of Parkinson's disease by using RNA extracted from SSL

### 1) SSL collection

Two tests were conducted as the following Test 1 and Test 2.
Test 1: 15 healthy subjects (from 40 to 89 years old, male and female) and 15 Parkinson's disease patients (PD) (from 40 to 89 years old, male and female) were selected as test subjects.
Test 2: 50 healthy subjects (from 40 to 89 years old, male) and 50 PD (from 40 to 89 years old, male) were selected as test subjects.

PD was diagnosed in advance as Parkinson's disease (Hoehn & Yahr stage I or II) by a neurologist. Sebum was recovered from the whole face of each test subject by using an oil blotting film (5 × 8 cm, made of polypropylene, 3M Company). Then, the oil blotting film was transferred to a vial and preserved at -80°C for approximately 1 month until use in RNA extraction.

### 2) RNA preparation and sequencing

The oil blotting film of the above section 1) was cut into an appropriate size, and RNA was extracted by using QIAzol Lysis Reagent (Qiagen N.V.) in accordance with the attached protocol. On the basis of the extracted RNA, cDNA was synthesized through reverse transcription at 42°C for 90 minutes by using Superscript VILO cDNA Synthesis kit (Life Technologies Japan Ltd.). The primers used for reverse transcription reaction were random primers attached to the kit. A library containing DNA derived from 20802 genes was prepared by multiplex PCR from the obtained cDNA. The multiplex PCR was performed by using Ion AmpliSeq Transcriptome Human Gene Expression Kit (Life Technologies Japan Ltd.) under conditions of [99°C, 2 min -> (99°C, 15 sec -> 62°C, 16 min) × 20 cycles -> 4°C, hold]. The obtained PCR product was purified with Ampure XP (Beckman Coulter Inc.), followed by buffer reconstitution, primer sequence digestion, adaptor ligation, purification, and amplification to prepare a library. The prepared library was loaded on Ion 540 Chip and sequenced by using Ion S5/XL system (Life Technologies Japan Ltd.).

### 3) Data analysis

### i) RNA expression analysis - 1

In the data (read count values) on the expression level of RNA derived from the test subjects measured in the above section 2), data with a read count of less than 10 was treated as missing values. After conversion to RPM values which normalized the read count values for difference in the total number of reads among samples, the missing values were compensated for by use of an approach called singular value decomposition (SVD) imputation. However, only genes which produced expression level data without missing values in 80% or more sample test subjects in the expression level data on the test subjects in all the samples were used in analysis given below. In the analysis, converted RPM values, logarithmic values of the RPM values of the read counts to base 2 (Log₂RPM values) were used in order to approximate the RPM values, which followed negative binominal distribution, to normal distribution.

Differentially expressed RNA which attained a p value of 0.05 or less in Student's t-test in PD compared with the healthy subjects was identified on the basis of the SSL-derived RNA expression levels (Log2RPM values) of the healthy subjects and PD described above. In Test 1, the expression of 111 RNAs was increased in PD compared with the healthy subjects (Tables 1-1 to 1-3), and the expression of 68 RNAs was decreased therein (Tables 1-4 to 1-5). Meanwhile, in Test 2, the expression of 565 RNAs was increased (Tables 1-6 to 1-19), and the expression of 294 RNAs was decreased (Tables 1-20 to 1-27). The expression of 18 RNAs was increased in common between Test 1 and Test 2, and the expression of 15 RNAs was decreased in common therebetween (genes indicated by boldface in the tables).

**[Table 1-1]**

| Test | Symbol | Fold change | p value | Regulation |
|---|---|---|---|---|
| Test 1 | ADRM1 | 0.609210571 | 0.043833254 | UP |
| Test 1 | ARF5 | 0.699874992 | 0.026663956 | UP |
| Test 1 | ARHGEF5 | 1.274057568 | 0.048234993 | UP |
| Test 1 | BCKDK | 1.080907515 | 0.000291562 | UP |
| Test 1 | **C10orf116** | **1.346318684** | **0.027914601** | **UP** |
| Test 1 | C11orf10 | 1.167882659 | 9.62E-05 | UP |
| Test 1 | C14orf2 | 0.627373658 | 0.013138616 | UP |
| Test 1 | CEBPA | 1.625049985 | 0.001261191 | UP |
| Test 1 | CHAC1 | 1.615056048 | 0.017192687 | UP |
| Test 1 | CHCHD2 | 0.867580331 | 0.009229477 | UP |
| Test 1 | CMIP | 0.555282243 | 0.042367806 | UP |
| Test 1 | **CNFN** | **1.272119089** | **0.024347366** | **UP** |
| Test 1 | COPE | 0.889122418 | 0.006852965 | UP |
| Test 1 | COPS8 | 1.11272686 | 0.033587705 | UP |
| Test 1 | COX8A | 0.614359496 | 0.037782725 | UP |
| Test 1 | CSDA | 1.34167409 | 0.00133884 | UP |
| Test 1 | CTBP2 | 0.962196486 | 0.024818568 | UP |
| Test 1 | CTDNEP1 | 0.961690787 | 0.019367259 | UP |
| Test 1 | CYFIP1 | 1.075131825 | 0.044852257 | UP |
| Test 1 | DAD1 | 0.860983925 | 0.020003683 | UP |
| Test 1 | DNASE1L2 | 1.461274581 | 0.021696734 | UP |
| Test 1 | DUX4L4 | 1.821703773 | 0.016937626 | UP |
| Test 1 | EDF1 | 0.634696647 | 0.006062601 | UP |
| Test 1 | EIF3E | 0.85054249 | 0.023427635 | UP |
| Test 1 | EIF4G1 | 0.874582442 | 0.008300118 | UP |
| Test 1 | **EMP1** | **1.428292097** | **0.010451584** | **UP** |
| Test 1 | FAM129B | 0.906162295 | 0.03314269 | UP |
| Test 1 | FAM83G | 1.692703352 | 0.005470943 | UP |
| Test 1 | FEM1B | 1.475450334 | 0.00508158 | UP |
| Test 1 | G6PD | 0.767453922 | 0.046885133 | UP |
| Test 1 | GPBP1L1 | 0.943160553 | 0.030787333 | UP |
| Test 1 | GPR157 | 1.399710796 | 0.001639686 | UP |
| Test 1 | GPX3 | 1.106976877 | 0.011928668 | UP |
| Test 1 | HECA | 0.551678309 | 0.021073296 | UP |
| Test 1 | HIPK1 | 1.10213078 | 0.008842706 | UP |
| Test 1 | HIST2H2BE | 0.639444381 | 0.048606096 | UP |
| Test 1 | HLA.DQB2 | 1.515233848 | 0.034824195 | UP |
| Test 1 | HMGCS1 | 0.852395823 | 0.033976968 | UP |
| Test 1 | HSPA1A | 1.559694806 | 0.002025704 | UP |
| Test 1 | IQSEC1 | 1.33654119 | 0.01105081 | UP |
| Test 1 | **KCNQ1OT1** | **1.644199329** | **0.04140012** | **UP** |

**[Table 1-2]**

| | | | | |
|---|---|---|---|---|
| Test 1 | KCTD11 | 1.172625927 | 0.028996311 | UP |
| Test 1 | KIAA0930 | 0.802565628 | 0.040645349 | UP |
| Test 1 | KLHDC3 | 1.162575929 | 0.029124485 | UP |
| Test 1 | **LCE3D** | **1.525057902** | **0.017729736** | **UP** |
| Test 1 | LOC100093631 | 1.202317044 | 0.018702745 | UP |
| Test 1 | LOC100506888 | 2.345734653 | 0.005026134 | UP |
| Test 1 | LOC349196 | 2.155586353 | 0.025593893 | UP |
| Test 1 | LOC401321 | 1.818317716 | 0.043316136 | UP |
| Test 1 | LPIN1 | 1.342620463 | 0.022057267 | UP |
| Test 1 | MAP2K2 | 0.96303756 | 0.018140774 | UP |
| Test 1 | METRNL | 0.495872438 | 0.034081797 | UP |
| Test 1 | MGLL | 1.12786717 | 0.031415315 | UP |
| Test 1 | NDUFA13 | 1.028288472 | 0.002609224 | UP |
| Test 1 | **NDUFA4L2** | **1.469853745** | **0.047011103** | **UP** |
| Test 1 | NDUFB11 | 1.178953409 | 0.015560192 | UP |
| Test 1 | **NDUFS5** | **1.173366098** | **0.028285636** | **UP** |
| Test 1 | N R4A3 | 1.062128975 | 0.018538 | UP |
| Test 1 | OAZ1 | 0.46509906 | 0.019553364 | UP |
| Test 1 | OR4F3 | 1.980298573 | 0.013883164 | UP |
| Test 1 | PKP3 | 1.051268637 | 0.017270523 | UP |
| Test 1 | POLD4 | 0.727728607 | 0.026390396 | UP |
| Test 1 | **POLR2L** | **1.288069119** | **0.005102443** | **UP** |
| Test 1 | PPA1 | 0.749279023 | 0.03206103 | UP |
| Test 1 | PQLC1 | 0.790392011 | 0.038455602 | UP |
| Test 1 | PRELID1 | 0.895128216 | 0.036353629 | UP |
| Test 1 | PSMB7 | 1.198078264 | 0.010069291 | UP |
| Test 1 | PSMC1 | 0.921786669 | 0.002813666 | UP |
| Test 1 | PSMD4 | 1.162613293 | 0.000301746 | UP |
| Test 1 | PURB | 1.294350134 | 0.005492975 | UP |
| Test 1 | RAP2B | 0.712661882 | 0.007752025 | UP |
| Test 1 | RASAL1 | 1.533954936 | 0.007931264 | UP |
| Test 1 | **REXO1L2P** | **2.258334633** | **0.021096388** | **UP** |
| Test 1 | **RPL7A** | **0.799765552** | **0.040024088** | **UP** |
| Test 1 | **RPS26** | **1.048925589** | **0.020173699** | **UP** |
| Test 1 | RRAD | 1.551857659 | 0.009621785 | UP |
| Test 1 | RRAGA | 1.21815067 | 0.01109446 | UP |
| Test 1 | SEC61A1 | 1.061101156 | 0.045353369 | UP |
| Test 1 | **SERPINB4** | **1.73450959** | **0.048165225** | **UP** |
| Test 1 | SFXN3 | 0.967423668 | 0.026897044 | UP |

**[Table 1-3]**

| | | | | |
|---|---|---|---|---|
| Test 1 | **SLC25A3** | **0.683663369** | **0.040816858** | **UP** |
| Test 1 | SNF8 | 0.92398189 | 0.031993765 | UP |
| Test 1 | **SNORA16A** | **1.233214856** | **0.005217419** | **UP** |
| Test 1 | **SNORA24** | **1.397191537** | **0.001016782** | **UP** |
| Test 1 | SNORA43 | 1.274218723 | 0.007468774 | UP |
| Test 1 | **SNORA50** | **1.299388426** | **0.010607324** | **UP** |
| Test 1 | SNORA8 | 1.005454688 | 0.028833348 | UP |
| Test 1 | **SNRPG** | **1.989577925** | **0.002505629** | **UP** |
| Test 1 | SPINT1 | 1.207565409 | 0.034516318 | UP |
| Test 1 | SQRDL | 0.766969993 | 0.00457474 | UP |
| Test 1 | SRXN1 | 0.873833451 | 0.018173048 | UP |
| Test 1 | STAT6 | 1.011794726 | 0.007483321 | UP |
| Test 1 | STIP1 | 0.85103506 | 0.043705392 | UP |
| Test 1 | TALD01 | 0.536260527 | 0.045700866 | UP |
| Test 1 | TCEB3CL | 2.366983401 | 0.006731453 | UP |
| Test 1 | TCIRG1 | 1.57092833 | 0.027056141 | UP |
| Test 1 | TEX264 | 1.236719119 | 0.014308363 | UP |
| Test 1 | TMEM183A | 1.156156624 | 0.03501219 | UP |
| Test 1 | TRMT112 | 0.867005398 | 0.033782415 | UP |
| Test 1 | TTC9 | 0.883705752 | 0.014677092 | UP |
| Test 1 | TYMP | 1.201700537 | 0.0171455 | UP |
| Test 1 | UQCRB | 0.761955105 | 0.036349839 | UP |
| Test 1 | UQCRC1 | 1.021819204 | 0.000400463 | UP |
| Test 1 | **UQCRH** | **1.081064513** | **0.010579673** | **UP** |
| Test 1 | UQCRQ | 1.172007584 | 0.009792679 | UP |
| Test 1 | USP17L5 | 2.261956901 | 0.017602717 | UP |
| Test 1 | USP17L6P | 2.333811727 | 0.013336494 | UP |
| Test 1 | USP38 | 1.129519222 | 0.022350671 | UP |
| Test 1 | VEGFA | 0.958877042 | 0.010007452 | UP |
| Test 1 | ZNF33A | 1.458704981 | 0.009711608 | UP |
| Test 1 | ZNF410 | 0.936467529 | 0.017554463 | UP |

**[Table 1-4]**

| | | | | |
|---|---|---|---|---|
| Test 1 | ACSL1 | -1.314414944 | 0.021386968 | DOWN |
| Test 1 | ACSL4 | -1.040837462 | 0.017095652 | DOWN |
| Test 1 | **ANKRD12** | **-1.930754522** | **0.010768568** | **DOWN** |
| Test 1 | ARPC1B | -0.617322479 | 0.042888509 | DOWN |
| Test 1 | BRD4 | -1.024694066 | 0.02763023 | DOWN |
| Test 1 | BTG1 | -0.934321414 | 0.039327439 | DOWN |
| Test 1 | CALM2 | -0.91810275 | 0.009187675 | DOWN |
| Test 1 | **CCL3** | **-1.639111096** | **0.008678309** | **DOWN** |
| Test 1 | **CCNI** | **-1.932387295** | **0.00403203** | **DOWN** |
| Test 1 | **CD83** | **-1.066374053** | **0.04175246** | **DOWN** |
| Test 1 | CDC42 | -1.611732634 | 0.00284614 | DOWN |
| Test 1 | CHMP4B | -0.715746914 | 0.030901 | DOWN |
| Test 1 | CNBP | -1.045580294 | 0.00387558 | DOWN |
| Test 1 | **CNN2** | **-0.629754604** | **0.023710615** | **DOWN** |
| Test 1 | **CSF2RB** | **-1.104312619** | **0.020573046** | **DOWN** |
| Test 1 | **CXCR4** | **-2.033830014** | **0.00024412** | **DOWN** |
| Test 1 | DDX5 | -1.149683056 | 0.023786744 | DOWN |
| Test 1 | EEF1A1 | -0.734882964 | 0.008509423 | DOWN |
| Test 1 | EEF1B2 | -0.972404288 | 0.009011592 | DOWN |
| Test 1 | **EGR2** | **-0.997120306** | **0.005989411** | **DOWN** |
| Test 1 | EIF1 | -1.068314684 | 0.000455452 | DOWN |
| Test 1 | EPS15 | -1.047381411 | 0.006922926 | DOWN |
| Test 1 | GNG10 | -0.739434103 | 0.04672473 | DOWN |
| Test 1 | GRINA | -0.709755929 | 0.043785466 | DOWN |
| Test 1 | H3F3A | -0.484685703 | 0.042106784 | DOWN |
| Test 1 | HIF1A | -0.738592709 | 0.038912439 | DOWN |
| Test 1 | HNRNPA2B1 | -1.27064282 | 0.001794524 | DOWN |
| Test 1 | HNRNPU | -1.02784524 | 0.042720575 | DOWN |
| Test 1 | IFNGR2 | -1.000333459 | 0.013306532 | DOWN |
| Test 1 | IL1RN | -1.291864782 | 0.0026776 | DOWN |
| Test 1 | **ITGAX** | **-1.11377676** | **0.027930711** | **DOWN** |
| Test 1 | **LITAF** | **-0.831805644** | **0.014085655** | **DOWN** |
| Test 1 | LYN | -0.959021868 | 0.040941384 | DOWN |
| Test 1 | NEAT1 | -0.957011121 | 0.047894721 | DOWN |
| Test 1 | PABPC1 | -1.035504388 | 0.002341174 | DOWN |
| Test 1 | PAIP2 | -0.864545414 | 0.040428904 | DOWN |
| Test 1 | PGK1 | -0.963519428 | 0.011841674 | DOWN |
| Test 1 | PLXNC1 | -1.099466822 | 0.026428919 | DOWN |
| Test 1 | RABGEF1 | -0.993856958 | 0.037254884 | DOWN |
| Test 1 | RAP1A | -1.114744033 | 0.031169618 | DOWN |
| Test 1 | REL | -1.282793738 | 0.01153212 | DOWN |

**[Table 1-5]**

| | | | | |
|---|---|---|---|---|
| Test 1 | RGS2 | -0.995907178 | 0.045303026 | DOWN |
| Test 1 | **RHOA** | **-0.902566363** | **0.003151667** | **DOWN** |
| Test 1 | **RNASEK** | **-1.016194703** | **0.030620951** | **DOWN** |
| Test 1 | RPL10 | -2.025185976 | 5.92E-05 | DOWN |
| Test 1 | RPL15 | -1.54290515 | 0.000469071 | DOWN |
| Test 1 | RPL19 | -0.892202011 | 0.015862788 | DOWN |
| Test 1 | RPL21 | -0.625280627 | 0.036709641 | DOWN |
| Test 1 | RPL26 | -1.153562245 | 0.015851768 | DOWN |
| Test 1 | RPL28 | -1.000169058 | 0.030081325 | DOWN |
| Test 1 | RPL3 | -1.077830298 | 0.003267945 | DOWN |
| Test 1 | RPL30 | -0.660396387 | 0.033024638 | DOWN |
| Test 1 | RPL35 | -0.700317983 | 0.029053156 | DOWN |
| Test 1 | RPL5 | -1.081758489 | 0.0300877 | DOWN |
| Test 1 | RPL6 | -1.573868621 | 0.025108045 | DOWN |
| Test 1 | RPS20 | -1.311993027 | 0.023283488 | DOWN |
| Test 1 | RPS25 | -0.913868434 | 0.022273799 | DOWN |
| Test 1 | S100A11 | -1.226240532 | 0.001687759 | DOWN |
| Test 1 | SCARNA9 | -1.045209104 | 0.029905065 | DOWN |
| Test 1 | **SERINC1** | **-0.651103256** | **0.046063301** | **DOWN** |
| Test 1 | **SERP1** | **-0.82729507** | **0.033858187** | **DOWN** |
| Test 1 | SNORA53 | -1.226150595 | 0.046365671 | DOWN |
| Test 1 | **SRRM2** | **-0.752261071** | **0.036848008** | **DOWN** |
| Test 1 | STK24 | -1.185703307 | 0.03897646 | DOWN |
| Test 1 | TMEM127 | -0.800780218 | 0.025357034 | DOWN |
| Test 1 | TNIP1 | -1.01072003 | 0.008782635 | DOWN |
| Test 1 | TPM4 | -0.629827116 | 0.033794869 | DOWN |
| Test 1 | TPT1 | -0.672287453 | 0.035475508 | DOWN |

**[Table 1-6]**

| | | | | |
|---|---|---|---|---|
| Test 2 | A2ML1 | 1.221533613 | 0.000112481 | UP |
| Test 2 | ABRACL | 0.595825415 | 0.002340761 | UP |
| Test 2 | ACBD3 | 0.480880204 | 0.019490426 | UP |
| Test 2 | ACOT13 | 0.435902885 | 0.027959053 | UP |
| Test 2 | ACSS3 | 0.639119766 | 0.023601116 | UP |
| Test 2 | ADAP2 | 0.538749812 | 0.018924703 | UP |
| Test 2 | ADPRHL2 | 0.404739489 | 0.045086548 | UP |
| Test 2 | ADSL | 0.4588524 | 0.038972385 | UP |
| Test 2 | ADSS | 0.651140057 | 0.002197834 | UP |
| Test 2 | AHCY | 0.802441979 | 0.000607218 | UP |
| Test 2 | AIF1L | 1.055021255 | 0.000495159 | UP |
| Test 2 | AIM1L | 0.62944081 | 0.040629376 | UP |
| Test 2 | AK1 | 0.491622387 | 0.029441855 | UP |
| Test 2 | AK4 | 0.474128267 | 0.041622944 | UP |
| Test 2 | ALDH1A3 | 0.535322936 | 0.025214627 | UP |
| Test 2 | ALDOC | 0.471800562 | 0.013096482 | UP |
| Test 2 | AMBRA1 | 0.359110063 | 0.04974087 | UP |
| Test 2 | ANP32B | 0.397177548 | 0.03932888 | UP |
| Test 2 | ANP32E | 0.494174712 | 0.011258347 | UP |
| Test 2 | ANXA1 | 0.54435181 | 0.008220099 | UP |
| Test 2 | AP4S1 | 0.553991355 | 0.012146838 | UP |
| Test 2 | ARFGAP2 | 0.459611753 | 0.018201769 | UP |
| Test 2 | ARHGAP29 | 0.914208609 | 0.003531249 | UP |
| Test 2 | ARL1 | 0.408729114 | 0.044051381 | UP |
| Test 2 | ASS1 | 0.643120811 | 0.006574816 | UP |
| Test 2 | ATP5B | 0.249687247 | 0.039711327 | UP |
| Test 2 | ATP5E | 0.284815562 | 0.033935216 | UP |
| Test 2 | ATP5G1 | 0.599572218 | 0.003312854 | UP |
| Test 2 | ATP5I | 0.537663746 | 0.00267032 | UP |
| Test 2 | ATP50 | 0.39709147 | 0.002208756 | UP |
| Test 2 | ATPIF1 | 0.382294733 | 0.01863468 | UP |
| Test 2 | BAG3 | 0.716644595 | 0.005145384 | UP |
| Test 2 | BCAS1 | 0.948535572 | 0.005105851 | UP |
| Test 2 | BCAS2 | 0.440711713 | 0.003048003 | UP |
| Test 2 | BCL2L13 | 0.458803119 | 0.037434143 | UP |
| Test 2 | BCL7C | 0.443147949 | 0.039568486 | UP |
| Test 2 | BMP2 | 0.68056004 | 0.032195249 | UP |
| Test 2 | **C10orf116** | **0.529752336** | **0.039587014** | **UP** |
| Test 2 | C11orf31 | 0.358444195 | 0.020591887 | UP |
| Test 2 | C1orf52 | 0.395191044 | 0.049654449 | UP |
| Test 2 | C1orf63 | 0.45668822 | 0.026824533 | UP |

**[Table 1-7]**

| | | | | |
|---|---|---|---|---|
| Test 2 | C22orf32 | 0.509669614 | 0.03126071 | UP |
| Test 2 | C2orf49 | 0.601493547 | 0.000740502 | UP |
| Test 2 | C5orf43 | 0.343779718 | 0.040727567 | UP |
| Test 2 | C5orf46 | 0.745587247 | 0.009981759 | UP |
| Test 2 | C8orf33 | 0.451060206 | 0.025475738 | UP |
| Test 2 | CACYBP | 0.426591512 | 0.016609089 | UP |
| Test 2 | CALM1 | 0.330924338 | 0.004677453 | UP |
| Test 2 | CARHSP1 | 0.784681465 | 0.000175932 | UP |
| Test 2 | CASK | 0.599582959 | 0.01003174 | UP |
| Test 2 | CASP14 | 0.632903218 | 0.027505259 | UP |
| Test 2 | CAST | 0.350973694 | 0.030849173 | UP |
| Test 2 | CCDC6 | 0.696273484 | 0.003551549 | UP |
| Test 2 | CCNE1 | 0.555426503 | 0.037173127 | UP |
| Test 2 | CCT2 | 0.427873529 | 0.031204993 | UP |
| Test 2 | CCT3 | 0.352019271 | 0.042821173 | UP |
| Test 2 | CCT4 | 0.414581271 | 0.048774852 | UP |
| Test 2 | CCT8 | 0.3837055 | 0.049670658 | UP |
| Test 2 | CDC16 | 0.57034355 | 0.009643665 | UP |
| Test 2 | CDSN | 0.644348354 | 0.010053349 | UP |
| Test 2 | CGA | 1.091914746 | 0.000462077 | UP |
| Test 2 | CGNL1 | 0.9992731 | 0.000612501 | UP |
| Test 2 | CHI3L2 | 0.57424439 | 0.020046259 | UP |
| Test 2 | CHIC2 | 0.410002803 | 0.04762423 | UP |
| Test 2 | CHMP4A | 0.52527036 | 0.004336838 | UP |
| Test 2 | CIZ1 | 0.498982985 | 0.039336744 | UP |
| Test 2 | CKB | 0.683086969 | 0.018305412 | UP |
| Test 2 | CLIC3 | 0.74263737 | 0.020412012 | UP |
| Test 2 | CLIP1 | 0.435971364 | 0.019001211 | UP |
| Test 2 | CNDP2 | 0.281021946 | 0.048982715 | UP |
| Test 2 | **CNFN** | **0.990121666** | **1.85E-05** | **UP** |
| Test 2 | CNIH4 | 0.457328621 | 0.02164633 | UP |
| Test 2 | CNN3 | 0.624509347 | 0.016593952 | UP |
| Test 2 | CNNM4 | 0.561756946 | 0.049794296 | UP |
| Test 2 | COA1 | 0.635059866 | 0.00138502 | UP |
| Test 2 | COA3 | 0.55836372 | 0.010330091 | UP |
| Test 2 | COMT | 0.329211212 | 0.046136915 | UP |
| Test 2 | COX4I1 | 0.298413672 | 0.00394488 | UP |
| Test 2 | COX5B | 0.236438786 | 0.036474931 | UP |
| Test 2 | CPEB2 | 0.875753539 | 0.003788596 | UP |
| Test 2 | CPNE3 | 0.532838867 | 0.015475427 | UP |
| Test 2 | CRABP2 | 0.766900027 | 0.000735812 | UP |

**[Table 1-8]**

| | | | | |
|---|---|---|---|---|
| Test 2 | CRELD2 | 0.685045483 | 0.009010899 | UP |
| Test 2 | CRIPT | 0.623088661 | 0.000802774 | UP |
| Test 2 | CRNN | 1.401884112 | 0.001214875 | UP |
| Test 2 | CST6 | 0.589966531 | 0.016466862 | UP |
| Test 2 | CSTA | 0.784255116 | 0.002502729 | UP |
| Test 2 | CUL4A | 0.489558405 | 0.013487958 | UP |
| Test 2 | CUTA | 0.585987127 | 0.001016471 | UP |
| Test 2 | CYB5A | 0.641939407 | 0.009668198 | UP |
| Test 2 | CYB5B | 0.544680118 | 0.005232354 | UP |
| Test 2 | DANCR | 0.43126336 | 0.041709971 | UP |
| Test 2 | DCAF12 | 0.545454648 | 0.011615314 | UP |
| Test 2 | DDRGK1 | 0.372347748 | 0.044309125 | UP |
| Test 2 | DDT | 0.472760004 | 0.010925051 | UP |
| Test 2 | DEGS1 | 0.545984689 | 0.037107489 | UP |
| Test 2 | DENND2C | 0.502288792 | 0.047224257 | UP |
| Test 2 | DHPS | 0.518845683 | 0.012866877 | UP |
| Test 2 | DHX29 | 0.682106105 | 0.006066935 | UP |
| Test 2 | DHX32 | 0.506509259 | 0.033864568 | UP |
| Test 2 | DHX40 | 0.396573604 | 0.015540946 | UP |
| Test 2 | DNAJA1 | 0.252552372 | 0.019713754 | UP |
| Test 2 | DNAJA4 | 0.483045351 | 0.044641278 | UP |
| Test 2 | DNAJC13 | 0.470362936 | 0.029093404 | UP |
| Test 2 | DNAJC15 | 0.469211563 | 0.013979287 | UP |
| Test 2 | DNAJC21 | 0.452709072 | 0.022814459 | UP |
| Test 2 | DNAJC7 | 0.319676387 | 0.022220543 | UP |
| Test 2 | DNAJC9 | 0.575126954 | 0.012161694 | UP |
| Test 2 | DOCK6 | 0.545719783 | 0.03676478 | UP |
| Test 2 | DOCK9 | 0.621757986 | 0.012261459 | UP |
| Test 2 | DPH1 | 1.158039818 | 6.72E-05 | UP |
| Test 2 | DPY30 | 0.398317757 | 0.02828779 | UP |
| Test 2 | DRG1 | 0.581253641 | 0.004984247 | UP |
| Test 2 | DSG1 | 0.567399218 | 0.037732972 | UP |
| Test 2 | DUSP11 | 0.473325618 | 0.006136292 | UP |
| Test 2 | DYM | 0.816178513 | 0.00274631 | UP |
| Test 2 | DYNC1LI1 | 0.583242858 | 0.0067388 | UP |
| Test 2 | DYNLL1 | 0.374636406 | 0.035010075 | UP |
| Test 2 | DYNLRB1 | 0.330053674 | 0.027194242 | UP |
| Test 2 | ECHS1 | 0.374219263 | 0.043974114 | UP |
| Test 2 | EFNB2 | 0.661019693 | 0.019887237 | UP |
| Test 2 | EIF1AX | 0.600523864 | 0.00135969 | UP |
| Test 2 | EIF2S2 | 0.666962534 | 0.008564954 | UP |

**[Table 1-9]**

| | | | | |
|---|---|---|---|---|
| Test 2 | EIF3K | 0.47571023 | 0.000332146 | UP |
| Test 2 | EIF4EBP1 | 0.509037765 | 0.036582232 | UP |
| Test 2 | ELOVL7 | 0.663055469 | 0.029361928 | UP |
| Test 2 | **EMP1** | **0.948607145** | **0.000952753** | **UP** |
| Test 2 | ENDOD1 | 0.83064568 | 0.003155686 | UP |
| Test 2 | EPHB6 | 0.996531897 | 0.000172709 | UP |
| Test 2 | EPHX3 | 0.957706717 | 0.001357233 | UP |
| Test 2 | ERBB3 | 0.668574797 | 0.018620686 | UP |
| Test 2 | ER01L | 0.595297117 | 0.005178102 | UP |
| Test 2 | EXOC4 | 0.717282266 | 0.003430647 | UP |
| Test 2 | EXOC5 | 0.555371778 | 0.003548179 | UP |
| Test 2 | EXOC6B | 0.472278499 | 0.047161361 | UP |
| Test 2 | F13A1 | 0.700312885 | 0.033387875 | UP |
| Test 2 | FABP4 | 1.470499552 | 2.97E-05 | UP |
| Test 2 | FABP9 | 1.368332459 | 2.70E-05 | UP |
| Test 2 | FAM108B1 | 0.629521772 | 0.011810697 | UP |
| Test 2 | FAM135A | 0.588280986 | 0.032230354 | UP |
| Test 2 | FAM210B | 0.545271557 | 0.037981153 | UP |
| Test 2 | FAM25B | 0.522599819 | 0.047412373 | UP |
| Test 2 | FAM3C | 0.615760366 | 0.004593036 | UP |
| Test 2 | FAM45A | 0.552544411 | 0.016999174 | UP |
| Test 2 | FAM46B | 0.791235807 | 0.003269448 | UP |
| Test 2 | FBXO45 | 0.651269976 | 0.015000916 | UP |
| Test 2 | FCHSD1 | 0.505831668 | 0.048614621 | UP |
| Test 2 | FIG4 | 0.441222277 | 0.010793265 | UP |
| Test 2 | FKBP1A | 0.163295342 | 0.048754238 | UP |
| Test 2 | FKBP3 | 0.635315923 | 0.011710114 | UP |
| Test 2 | FLG | 0.8220595 | 0.030652941 | UP |
| Test 2 | FOXQ1 | 0.739538694 | 0.019924301 | UP |
| Test 2 | FRMD6 | 0.680800391 | 0.007307527 | UP |
| Test 2 | FTSJ1 | 0.66863706 | 0.005309815 | UP |
| Test 2 | FUNDC2 | 0.499543676 | 0.016301607 | UP |
| Test 2 | FYN | 0.464677144 | 0.036203854 | UP |
| Test 2 | GBAS | 0.702529543 | 0.002848186 | UP |
| Test 2 | GGCT | 0.642905928 | 0.026758206 | UP |
| Test 2 | GHITM | 0.25443743 | 0.032592325 | UP |
| Test 2 | GLOD4 | 0.533853822 | 0.013415796 | UP |
| Test 2 | GNL3 | 0.584900087 | 0.009442293 | UP |
| Test 2 | GPSM2 | 0.739454628 | 0.002548872 | UP |
| Test 2 | GRHL3 | 0.572937195 | 0.024076507 | UP |
| Test 2 | GRPEL1 | 0.471178948 | 0.007047655 | UP |

**[Table 1-10]**

| | | | | |
|---|---|---|---|---|
| Test 2 | GTF2A2 | 0.330372647 | 0.040423351 | UP |
| Test 2 | GTF2E2 | 0.534855078 | 0.004830931 | UP |
| Test 2 | GTF2H5 | 0.611879208 | 0.000741758 | UP |
| Test 2 | GTF3C5 | 0.388985663 | 0.032287492 | UP |
| Test 2 | GTF3C6 | 0.562851294 | 0.008550842 | UP |
| Test 2 | H1FX | 0.38289824 | 0.039887459 | UP |
| Test 2 | HADH | 0.596886384 | 0.031698277 | UP |
| Test 2 | HBEGF | 0.35824757 | 0.029353225 | UP |
| Test 2 | HDAC1 | 0.412996826 | 0.019794177 | UP |
| Test 2 | HDDC2 | 0.481028865 | 0.038549638 | UP |
| Test 2 | HEATR5A | 0.541675769 | 0.004141004 | UP |
| Test 2 | HEXB | 0.48326638 | 0.016112958 | UP |
| Test 2 | HIBADH | 0.491409911 | 0.028149152 | UP |
| Test 2 | HIBCH | 0.588943801 | 0.025448145 | UP |
| Test 2 | HIST1H1E | 0.436334476 | 0.040849694 | UP |
| Test 2 | HIST1H2AE | 0.472022185 | 0.032486571 | UP |
| Test 2 | HIST1H2AG | 0.554952196 | 0.026912916 | UP |
| Test 2 | HIST1H2AI | 0.53748617 | 0.034833553 | UP |
| Test 2 | HIST1H2AM | 0.505465922 | 0.015356542 | UP |
| Test 2 | HIST1H2BN | 0.547150364 | 0.019828836 | UP |
| Test 2 | HIST1H3B | 1.061476948 | 0.000400823 | UP |
| Test 2 | HIST1H3I | 0.601309393 | 0.011107396 | UP |
| Test 2 | HIST1H4B | 0.839544468 | 0.00079634 | UP |
| Test 2 | HIST1H4E | 0.778335085 | 0.00020329 | UP |
| Test 2 | HIST1H4F | 0.551175791 | 0.032237462 | UP |
| Test 2 | HIST1H4H | 0.715081702 | 0.000190121 | UP |
| Test 2 | HMOX2 | 0.375124592 | 0.032277484 | UP |
| Test 2 | HNRNPA0 | 0.43012224 | 0.023849018 | UP |
| Test 2 | HOMER1 | 0.572056122 | 0.027336542 | UP |
| Test 2 | HOOK1 | 0.689647412 | 0.000609804 | UP |
| Test 2 | HPGD | 0.531461662 | 0.034525209 | UP |
| Test 2 | HRSP12 | 0.748163886 | 0.00429738 | UP |
| Test 2 | HSD17B10 | 0.525580431 | 0.005390788 | UP |
| Test 2 | HSP90AA1 | 0.450671514 | 0.012853222 | UP |
| Test 2 | HSPD1 | 0.353524268 | 0.038337878 | UP |
| Test 2 | HYPK | 0.495508732 | 0.000812946 | UP |
| Test 2 | IDE | 0.561486404 | 0.030266606 | UP |
| Test 2 | IDH3A | 0.715741483 | 0.000740982 | UP |
| Test 2 | IFI27 | 1.088718271 | 0.000166105 | UP |
| Test 2 | IL32 | 0.635464648 | 0.022927371 | UP |
| Test 2 | IL36A | 1.193557169 | 0.000147742 | UP |

**[Table 1-11]**

| | | | | |
|---|---|---|---|---|
| Test 2 | ILKAP | 0.498704265 | 0.018877961 | UP |
| Test 2 | IP05 | 0.524135485 | 0.004351655 | UP |
| Test 2 | IQCG | 0.517533726 | 0.033045998 | UP |
| Test 2 | ITGB1BP1 | 0.592037248 | 0.005471131 | UP |
| Test 2 | ITPA | 0.428949095 | 0.023930788 | UP |
| Test 2 | ITPRIPL2 | 0.522695359 | 0.014016549 | UP |
| Test 2 | IVL | 1.113959428 | 4.72E-05 | UP |
| Test 2 | KAN K1 | 0.722652018 | 0.016004319 | UP |
| Test 2 | **KCNQ1OT1** | **0.517120259** | **0.015543571** | **UP** |
| Test 2 | KIAA0240 | 0.431683501 | 0.018876409 | UP |
| Test 2 | KIAA1143 | 0.346950172 | 0.046415853 | UP |
| Test 2 | KLF5 | 0.500232931 | 0.027794858 | UP |
| Test 2 | KLK13 | 0.65799937 | 0.017034932 | UP |
| Test 2 | KLK7 | 0.744388154 | 0.007376759 | UP |
| Test 2 | KLK8 | 0.597855011 | 0.024365979 | UP |
| Test 2 | KRT14 | 0.471660604 | 0.041821956 | UP |
| Test 2 | KRT16 | 0.438210052 | 0.041610329 | UP |
| Test 2 | KRT25 | 1.299645558 | 5.17E-05 | UP |
| Test 2 | KRT26 | 0.707262572 | 0.008809601 | UP |
| Test 2 | KRT27 | 1.207014606 | 5.40E-05 | UP |
| Test 2 | KRT5 | 0.757037273 | 0.027824563 | UP |
| Test 2 | KRT6A | 0.454023277 | 0.038422784 | UP |
| Test 2 | KRT6C | 0.78340478 | 0.023768169 | UP |
| Test 2 | KRT71 | 1.159098826 | 8.74E-05 | UP |
| Test 2 | KRT72 | 1.230166904 | 6.27E-05 | UP |
| Test 2 | KRT74 | 1.095061209 | 0.00590623 | UP |
| Test 2 | KRT78 | 0.737035195 | 0.034619014 | UP |
| Test 2 | KRTAP1.5 | 1.60464646 | 0.000819038 | UP |
| Test 2 | KRTAP12.1 | 1.229397362 | 0.000287135 | UP |
| Test 2 | KRTAP12.2 | 0.938688052 | 0.001073077 | UP |
| Test 2 | KRTAP19.1 | 0.844471097 | 0.018428031 | UP |
| Test 2 | KRTAP3.1 | 2.122465083 | 7.02E-05 | UP |
| Test 2 | KRTAP3.3 | 1.394541092 | 0.001179985 | UP |
| Test 2 | KRTAP5.3 | 1.432908956 | 6.99E-05 | UP |
| Test 2 | KRTAP5.7 | 1.447966369 | 3.78E-05 | UP |
| Test 2 | KRTDAP | 0.702409287 | 0.003662818 | UP |
| Test 2 | KTN1 | 0.381285498 | 0.039123884 | UP |
| Test 2 | LCE2A | 0.582131055 | 0.026199523 | UP |
| Test 2 | LCE2C | 0.619920884 | 0.015817916 | UP |
| Test 2 | LCE2D | 0.621960127 | 0.021384422 | UP |
| Test 2 | **LCE3D** | **0.843482517** | **0.000577787** | **UP** |

**[Table 1-12]**

| | | | | |
|---|---|---|---|---|
| Test 2 | LCE3E | 0.836563972 | 0.000810209 | UP |
| Test 2 | LCMT1 | 0.601842869 | 0.025859542 | UP |
| Test 2 | LCN2 | 0.73325772 | 0.003321 | UP |
| Test 2 | LEMD3 | 0.440994015 | 0.016865308 | UP |
| Test 2 | LEPROTL1 | 0.377879515 | 0.038317178 | UP |
| Test 2 | LINC00675 | 0.573523306 | 0.034073972 | UP |
| Test 2 | LLPH | 0.484998299 | 0.007188702 | UP |
| Test 2 | LMBR1 | 0.665083353 | 0.00191151 | UP |
| Test 2 | LNX1 | 0.952713443 | 0.000293549 | UP |
| Test 2 | LOC100505738 | 0.477053745 | 0.024409 | UP |
| Test 2 | LOC550643 | 0.634672437 | 0.002533558 | UP |
| Test 2 | LOC646862 | 0.747572165 | 0.025405318 | UP |
| Test 2 | LRBA | 0.529280351 | 0.038783597 | UP |
| Test 2 | LRRC15 | 0.906456672 | 0.002321669 | UP |
| Test 2 | LSM10 | 0.508507242 | 0.013416263 | UP |
| Test 2 | LSM2 | 0.675878285 | 0.004242908 | UP |
| Test 2 | LSM7 | 0.570617764 | 0.005193872 | UP |
| Test 2 | LTF | 0.717042662 | 0.012011178 | UP |
| Test 2 | LY6D | 0.638909247 | 0.038220601 | UP |
| Test 2 | LYNX1 | 1.006012262 | 0.002091327 | UP |
| Test 2 | MAFA | 0.646509839 | 0.018661569 | UP |
| Test 2 | MAL | 1.157393695 | 0.00203966 | UP |
| Test 2 | MALL | 1.082853546 | 0.000258882 | UP |
| Test 2 | MAOA | 0.489452289 | 0.017793881 | UP |
| Test 2 | MAP4K3 | 0.567499535 | 0.022681249 | UP |
| Test 2 | MAP7 | 0.597783019 | 0.034525239 | UP |
| Test 2 | MCCC1 | 0.677783016 | 0.008565533 | UP |
| Test 2 | MCTS1 | 0.499448675 | 0.013734219 | UP |
| Test 2 | MICALCL | 0.519128213 | 0.00748038 | UP |
| Test 2 | MNF1 | 0.448890025 | 0.045325106 | UP |
| Test 2 | MPHOSPH6 | 0.431463962 | 0.044290704 | UP |
| Test 2 | MPV17 | 0.462010792 | 0.022209637 | UP |
| Test 2 | MRPL11 | 0.444169953 | 0.041198724 | UP |
| Test 2 | MRPL12 | 0.459260738 | 0.023664309 | UP |
| Test 2 | MRPL24 | 0.49423042 | 0.034251269 | UP |
| Test 2 | MRPL32 | 0.570527545 | 0.004685957 | UP |
| Test 2 | MRPL47 | 0.522250156 | 0.004855696 | UP |
| Test 2 | MRPS11 | 0.723572233 | 0.000171238 | UP |
| Test 2 | MRPS18B | 0.606642285 | 0.003402311 | UP |
| Test 2 | MRPS24 | 0.424610103 | 0.027109976 | UP |
| Test 2 | MT1X | 0.913147816 | 0.000517578 | UP |

**[Table 1-13]**

| | | | | |
|---|---|---|---|---|
| Test 2 | MTMR12 | 0.60532463 | 0.015109514 | UP |
| Test 2 | MUT | 0.529761761 | 0.005829175 | UP |
| Test 2 | MY010 | 0.937118688 | 6.65E-05 | UP |
| Test 2 | MZT2A | 0.830391158 | 0.000900666 | UP |
| Test 2 | NCBP2 | 0.513307919 | 0.007048167 | UP |
| Test 2 | NCK1 | 0.49374477 | 0.015371978 | UP |
| Test 2 | NDRG2 | 0.354843551 | 0.048575543 | UP |
| Test 2 | NDUFA12 | 0.737096619 | 0.000119869 | UP |
| Test 2 | NDUFA2 | 0.432617464 | 0.013454569 | UP |
| Test 2 | **NDUFA4L2** | **1.063393782** | **2.72E-05** | **UP** |
| Test 2 | NDUFB1 | 0.287495056 | 0.035078556 | UP |
| Test 2 | **NDUFS5** | **0.457090069** | **0.011340643** | **UP** |
| Test 2 | NDUFS6 | 0.590046428 | 6.11E-05 | UP |
| Test 2 | NEDD4L | 0.511884957 | 0.049453884 | UP |
| Test 2 | NFU1 | 0.65767548 | 0.002449743 | UP |
| Test 2 | NHP2 | 0.454032668 | 0.040871766 | UP |
| Test 2 | NIN | 0.52183109 | 0.010001048 | UP |
| Test 2 | NIPAL3 | 0.577629247 | 0.046987766 | UP |
| Test 2 | NIPAL4 | 0.897027632 | 0.005310037 | UP |
| Test 2 | NOSIP | 0.427938626 | 0.020360295 | UP |
| Test 2 | NRIP3 | 0.506284789 | 0.020884863 | UP |
| Test 2 | NSMCE1 | 0.577791701 | 0.00730131 | UP |
| Test 2 | NUDC | 0.694341809 | 0.014762563 | UP |
| Test 2 | NUMA1 | 0.36191125 | 0.020606246 | UP |
| Test 2 | NUP214 | 0.47743027 | 0.049271574 | UP |
| Test 2 | NUPL1 | 0.335741644 | 0.047223087 | UP |
| Test 2 | OFD1 | 0.563849491 | 0.004983374 | UP |
| Test 2 | OLA1 | 0.393966653 | 0.029910839 | UP |
| Test 2 | ORMDL3 | 0.439255773 | 0.034802361 | UP |
| Test 2 | PABPN1 | 0.350869528 | 0.02211862 | UP |
| Test 2 | PADI1 | 0.67070604 | 0.019434864 | UP |
| Test 2 | PADI3 | 1.215749009 | 7.35E-05 | UP |
| Test 2 | PAK4 | 0.509965072 | 0.042007684 | UP |
| Test 2 | PAPL | 0.529409941 | 0.037223187 | UP |
| Test 2 | PCCB | 0.709869935 | 0.001272684 | UP |
| Test 2 | PDCD5 | 0.377965927 | 0.044465853 | UP |
| Test 2 | PDDC1 | 0.503218985 | 0.028228175 | UP |
| Test 2 | PDE12 | 0.396547298 | 0.041406947 | UP |
| Test 2 | PDHA1 | 0.492613289 | 0.011811977 | UP |
| Test 2 | PDZD8 | 0.455694983 | 0.039833582 | UP |
| Test 2 | PDZK1IP1 | 0.748234961 | 0.004749239 | UP |

**[Table 1-14]**

| | | | | |
|---|---|---|---|---|
| Test 2 | PEPD | 0.468814718 | 0.026975661 | UP |
| Test 2 | PFDN2 | 0.4710158 | 0.021859005 | UP |
| Test 2 | PFDN5 | 0.220335663 | 0.049147318 | UP |
| Test 2 | PFDN6 | 0.494260171 | 0.011519195 | UP |
| Test 2 | PHAX | 0.508298395 | 0.018098917 | UP |
| Test 2 | PHF13 | 0.460509877 | 0.034666845 | UP |
| Test 2 | PHPT1 | 0.548135369 | 0.005002792 | UP |
| Test 2 | PICK1 | 0.4635477 | 0.02617828 | UP |
| Test 2 | PINLYP | 0.783929639 | 0.006215794 | UP |
| Test 2 | PITRM1 | 0.39895903 | 0.039173248 | UP |
| Test 2 | PKP1 | 0.644729487 | 0.011720844 | UP |
| Test 2 | PLCD1 | 0.737870521 | 0.005536531 | UP |
| Test 2 | PLD2 | 0.508558382 | 0.012373652 | UP |
| Test 2 | PLS3 | 0.658472726 | 0.007445336 | UP |
| Test 2 | POF1B | 0.848689586 | 0.009264505 | UP |
| Test 2 | POLR2D | 0.4992488 | 0.001983556 | UP |
| Test 2 | POLR2G | 0.598027214 | 0.010404429 | UP |
| Test 2 | **POLR2L** | **0.3357497** | **0.037600455** | **UP** |
| Test 2 | POLR2M | 0.411455592 | 0.045454349 | UP |
| Test 2 | PPFIBP2 | 0.449798477 | 0.042506262 | UP |
| Test 2 | PPID | 0.382456948 | 0.041407629 | UP |
| Test 2 | PPIL4 | 0.40788823 | 0.035051455 | UP |
| Test 2 | PPL | 1.165630089 | 0.000661664 | UP |
| Test 2 | PPP1R13B | 0.707903942 | 0.012009575 | UP |
| Test 2 | PPP2R2A | 0.37767129 | 0.046357843 | UP |
| Test 2 | PPP5C | 0.814821426 | 0.002376737 | UP |
| Test 2 | PPWD1 | 0.580223957 | 0.004372047 | UP |
| Test 2 | PRDX3 | 0.409111767 | 0.024794403 | UP |
| Test 2 | PRDX6 | 0.310519039 | 0.032277997 | UP |
| Test 2 | PREP | 0.416055344 | 0.048166689 | UP |
| Test 2 | PRKRA | 0.45076589 | 0.028570612 | UP |
| Test 2 | PROM2 | 0.821663343 | 0.016405122 | UP |
| Test 2 | PRPF40A | 0.457875591 | 0.027986636 | UP |
| Test 2 | PRPF4B | 0.537856846 | 0.002990068 | UP |
| Test 2 | PRR9 | 1.012665648 | 0.000286425 | UP |
| Test 2 | PRSS3 | 0.753484738 | 0.001895958 | UP |
| Test 2 | PSMC2 | 0.35148635 | 0.040165519 | UP |
| Test 2 | PSORS1C2 | 0.946759355 | 0.006100019 | UP |
| Test 2 | PTPN3 | 0.543446291 | 0.035596652 | UP |
| Test 2 | PVRL4 | 0.80686893 | 0.004930709 | UP |
| Test 2 | QKI | 0.277755845 | 0.031937093 | UP |

**[Table 1-15]**

| | | | | |
|---|---|---|---|---|
| Test 2 | RAB38 | 0.748487957 | 0.001035725 | UP |
| Test 2 | RABIF | 0.438505415 | 0.007298243 | UP |
| Test 2 | RANBP1 | 1.04696268 | 1.36E-05 | UP |
| Test 2 | RANBP10 | 0.552977796 | 0.026238115 | UP |
| Test 2 | RARRES1 | 0.620687381 | 0.019855473 | UP |
| Test 2 | RBM10 | 0.425725495 | 0.023147642 | UP |
| Test 2 | RBMS2 | 0.706576019 | 0.001389874 | UP |
| Test 2 | **REXO1L2P** | **0.730041651** | **0.016022131** | **UP** |
| Test 2 | RHCG | 1.163040682 | 9.35E-05 | UP |
| Test 2 | RMRP | 0.605574492 | 0.000163029 | UP |
| Test 2 | RNASE7 | 0.718897169 | 0.019766232 | UP |
| Test 2 | RNF121 | 0.440046558 | 0.016046961 | UP |
| Test 2 | RNF20 | 0.673608418 | 0.00223791 | UP |
| Test 2 | ROM01 | 0.325128448 | 0.038508067 | UP |
| Test 2 | RPA1 | 0.436121484 | 0.045123305 | UP |
| Test 2 | RPIA | 0.74921433 | 0.000111086 | UP |
| Test 2 | RPL10A | 0.283649048 | 0.039968527 | UP |
| Test 2 | RPL18 | 0.301532043 | 0.024095949 | UP |
| Test 2 | RPL21 | 0.266798928 | 0.03678764 | UP |
| Test 2 | RPL26L1 | 0.674511114 | 0.001010617 | UP |
| Test 2 | RPL30 | 0.231637085 | 0.039116984 | UP |
| Test 2 | RPL32 | 0.357709343 | 0.004112163 | UP |
| Test 2 | RPL36 | 0.311893187 | 0.018234435 | UP |
| Test 2 | RPL36A | 0.336279436 | 0.007725217 | UP |
| Test 2 | RPL37A | 0.415682354 | 0.003350758 | UP |
| Test 2 | RPL38 | 0.285259201 | 0.027892997 | UP |
| Test 2 | RPL7 | 0.355361637 | 0.006023648 | UP |
| Test 2 | **RPL7A** | **0.370261967** | **0.003107308** | **UP** |
| Test 2 | RPLP0 | 0.397845736 | 0.002167296 | UP |
| Test 2 | RPLP1 | 0.422223519 | 0.001209864 | UP |
| Test 2 | RPS12 | 0.45913525 | 0.000751779 | UP |
| Test 2 | RPS15 | 0.302865045 | 0.029280518 | UP |
| Test 2 | RPS15A | 0.260829012 | 0.042894741 | UP |
| Test 2 | RPS18 | 0.549381525 | 0.00019412 | UP |
| Test 2 | **RPS26** | **0.423684057** | **0.015281796** | **UP** |
| Test 2 | RPS28 | 0.376721184 | 0.01216883 | UP |
| Test 2 | RPS29 | 0.98999607 | 0.001861427 | UP |
| Test 2 | RPS3 | 0.438411389 | 0.005266268 | UP |
| Test 2 | RPS4X | 0.381013466 | 0.004861978 | UP |
| Test 2 | RPS5 | 0.351386701 | 0.014125176 | UP |
| Test 2 | RPS6 | 0.278963462 | 0.044007702 | UP |

**[Table 1-16]**

| | | | | |
|---|---|---|---|---|
| Test 2 | RPS6KA2 | 0.517206191 | 0.04372271 | UP |
| Test 2 | RPS6KB1 | 0.413890467 | 0.04890732 | UP |
| Test 2 | RPTN | 0.792988732 | 0.039812579 | UP |
| Test 2 | S100A14 | 0.735444547 | 0.007361184 | UP |
| Test 2 | S100A7 | 0.511938094 | 0.039234587 | UP |
| Test 2 | S100A7A | 0.780884027 | 0.006392561 | UP |
| Test 2 | S100A8 | 0.480554943 | 0.005565746 | UP |
| Test 2 | S100A9 | 0.541890412 | 0.001827719 | UP |
| Test 2 | SBDS | 0.501382901 | 0.004032371 | UP |
| Test 2 | SBF1 | 0.450478584 | 0.026502631 | UP |
| Test 2 | SBSN | 0.514431135 | 0.03450677 | UP |
| Test 2 | SCARNA12 | 0.47613506 | 0.013100706 | UP |
| Test 2 | SCARNA16 | 0.950996515 | 4.89E-06 | UP |
| Test 2 | SCARNA17 | 0.480107492 | 0.005539693 | UP |
| Test 2 | SCARNA6 | 0.69159554 | 0.000174868 | UP |
| Test 2 | SCARNA7 | 0.39276757 | 0.018164776 | UP |
| Test 2 | SCGB2A2 | 0.605410508 | 0.04509638 | UP |
| Test 2 | SCNN1B | 0.790814471 | 0.006130053 | UP |
| Test 2 | SCNN1G | 0.659327124 | 0.033550311 | UP |
| Test 2 | SDR16C5 | 0.803745452 | 0.005092806 | UP |
| Test 2 | SDR9C7 | 0.562729748 | 0.023941492 | UP |
| Test 2 | SEC23A | 0.381494906 | 0.048213143 | UP |
| Test 2 | SERPINA9 | 0.745391039 | 0.009476658 | UP |
| **Test 2** | **SERPINB4** | **0.740104652** | **0.009405167** | **UP** |
| Test 2 | SERPINB5 | 0.545369808 | 0.01702846 | UP |
| Test 2 | SERPINB7 | 0.996392198 | 0.001276768 | UP |
| Test 2 | SF3B14 | 0.322347433 | 0.03198073 | UP |
| Test 2 | SF3B3 | 0.374175675 | 0.042316392 | UP |
| Test 2 | SH3GL3 | 0.536171647 | 0.014877267 | UP |
| Test 2 | SLC10A6 | 0.70193016 | 0.012397218 | UP |
| Test 2 | SLC25A20 | 0.506425898 | 0.031631881 | UP |
| **Test 2** | **SLC25A3** | **0.266515461** | **0.031602198** | **UP** |
| Test 2 | SLC25A5 | 0.348945458 | 0.046829004 | UP |
| Test 2 | SLC26A9 | 0.875604042 | 0.003342367 | UP |
| Test 2 | SLC5A1 | 0.69571361 | 0.019710856 | UP |
| Test 2 | SLC6A14 | 1.057914573 | 0.000130014 | UP |
| Test 2 | SLC6A8 | 0.649208632 | 0.00790547 | UP |
| Test 2 | SLFN5 | 0.484567716 | 0.031313993 | UP |
| Test 2 | SLM02 | 0.422924252 | 0.044372872 | UP |
| Test 2 | SLURP1 | 1.144320913 | 0.003648741 | UP |
| Test 2 | SMAD7 | 0.460254587 | 0.028410104 | UP |

**[Table 1-17]**

| | | | | |
|---|---|---|---|---|
| Test 2 | SMC3 | 0.424518141 | 0.025409105 | UP |
| Test 2 | SMEK2 | 0.365851287 | 0.025427854 | UP |
| Test 2 | SMIM5 | 0.578361256 | 0.035681779 | UP |
| Test 2 | SNHG1 | 0.574921517 | 0.043671047 | UP |
| Test 2 | SNHG16 | 0.452555179 | 0.016900832 | UP |
| Test 2 | SNHG6 | 0.544935945 | 0.015263998 | UP |
| Test 2 | SNHG9 | 0.482721161 | 0.016394879 | UP |
| Test 2 | SNIP1 | 0.530290658 | 0.003401929 | UP |
| Test 2 | SNORA10 | 0.500134561 | 0.002159221 | UP |
| Test 2 | SNORA14B | 0.45085888 | 0.041534114 | UP |
| Test 2 | **SNORA16A** | **0.800445194** | **3.37E-05** | **UP** |
| Test 2 | SNORA21 | 0.715146141 | 0.000691404 | UP |
| Test 2 | SNORA23 | 0.496231233 | 0.003562425 | UP |
| Test 2 | **SNORA24** | **0.62246595** | **0.000620204** | **UP** |
| Test 2 | SNORA33 | 0.438137876 | 0.02535746 | UP |
| Test 2 | SNORA34 | 0.656213162 | 0.000524629 | UP |
| Test 2 | SNORA38 | 0.634158916 | 0.003980579 | UP |
| Test 2 | SNORA49 | 0.391208768 | 0.046528283 | UP |
| Test 2 | **SNORA50** | **0.501154595** | **0.004445349** | **UP** |
| Test 2 | SNORA52 | 0.691692913 | 0.000529232 | UP |
| Test 2 | SNORA57 | 0.670436835 | 0.000193375 | UP |
| Test 2 | SNORA6 | 0.370693768 | 0.043296102 | UP |
| Test 2 | SNORA62 | 0.442287413 | 0.013819155 | UP |
| Test 2 | SNORA63 | 0.532547036 | 0.003373425 | UP |
| Test 2 | SNORA65 | 0.448397229 | 0.026519056 | UP |
| Test 2 | SNORA67 | 0.441883998 | 0.017176358 | UP |
| Test 2 | SNORA68 | 0.857238018 | 4.58E-06 | UP |
| Test 2 | SNORA71A | 0.77835002 | 6.58E-05 | UP |
| Test 2 | SNORA71B | 0.529565961 | 0.004265337 | UP |
| Test 2 | SNORA71C | 0.495048141 | 0.007860449 | UP |
| Test 2 | SNORA71D | 0.435120855 | 0.018190672 | UP |
| Test 2 | SNORA74A | 0.610355277 | 0.004621969 | UP |
| Test 2 | SNORA74B | 0.645103623 | 0.004736561 | UP |
| Test 2 | SNORA7B | 0.492973554 | 0.010228164 | UP |
| Test 2 | SNORA84 | 0.625894973 | 0.001979651 | UP |
| Test 2 | SNORA9 | 0.497227196 | 0.007127047 | UP |
| Test 2 | SNORD15A | 0.637610126 | 0.001369934 | UP |
| Test 2 | SNORD15B | 0.59085556 | 0.00058311 | UP |
| Test 2 | SNORD17 | 0.357567591 | 0.045043368 | UP |
| Test 2 | SNORD94 | 0.826225768 | 6.63E-05 | UP |
| Test 2 | SNRPD1 | 0.511252623 | 0.028348085 | UP |

**[Table 1-18]**

| | | | | |
|---|---|---|---|---|
| Test 2 | SNRPE | 0.569496878 | 0.00533683 | UP |
| Test 2 | SNRPF | 0.723093533 | 0.002784753 | UP |
| Test 2 | **SNRPG** | **0.533113185** | **0.003903621** | **UP** |
| Test 2 | SOS1 | 0.510197893 | 0.008126315 | UP |
| Test 2 | SPINK5 | 0.679695473 | 0.013783683 | UP |
| Test 2 | SPINK7 | 0.833537404 | 0.008934427 | UP |
| Test 2 | SPRED1 | 0.448131411 | 0.045688393 | UP |
| Test 2 | SPRR1A | 0.588650149 | 0.016152579 | UP |
| Test 2 | SPRR1B | 0.56105932 | 0.012468229 | UP |
| Test 2 | SPRR2D | 1.026630516 | 1.40E-05 | UP |
| Test 2 | SPRR2E | 0.812347856 | 0.000312759 | UP |
| Test 2 | SPRR2F | 0.6818015 | 0.01623415 | UP |
| Test 2 | SPRR3 | 1.262348143 | 0.010954072 | UP |
| Test 2 | SPTLC1 | 0.676124476 | 0.00023443 | UP |
| Test 2 | SPTLC2 | 0.466842018 | 0.021700887 | UP |
| Test 2 | SRD5A1 | 0.384823816 | 0.048171711 | UP |
| Test 2 | SRSF10 | 0.559315822 | 0.003436841 | UP |
| Test 2 | SSBP1 | 0.362932978 | 0.040546721 | UP |
| Test 2 | SSBP3 | 0.477696067 | 0.030247231 | UP |
| Test 2 | STAP2 | 0.595061813 | 0.020857904 | UP |
| Test 2 | SUMF2 | 0.520831633 | 0.008329324 | UP |
| Test 2 | SYBU | 0.795756793 | 0.010109319 | UP |
| Test 2 | TADA2B | 0.650173529 | 0.00745433 | UP |
| Test 2 | TCEA1 | 0.41056745 | 0.030342599 | UP |
| Test 2 | TCHH | 1.178806419 | 8.10E-05 | UP |
| Test 2 | TCHHL1 | 1.300728249 | 5.53E-05 | UP |
| Test 2 | TFAP2C | 0.503894804 | 0.049079738 | UP |
| Test 2 | TFIP11 | 0.475273026 | 0.013898256 | UP |
| Test 2 | TGM3 | 1.042385225 | 0.004077755 | UP |
| Test 2 | THOC7 | 0.413919226 | 0.049112511 | UP |
| Test 2 | TIA1 | 0.490818268 | 0.006610946 | UP |
| Test 2 | TM4SF1 | 0.476103934 | 0.034341158 | UP |
| Test 2 | TM4SF19 | 0.971617642 | 0.000218413 | UP |
| Test 2 | TMEM179B | 0.389161719 | 0.035487717 | UP |
| Test 2 | TMEM45B | 0.547241184 | 0.03914833 | UP |
| Test 2 | TMEM60 | 0.408255653 | 0.033088543 | UP |
| Test 2 | TPRG1 | 0.86840816 | 0.011100172 | UP |
| Test 2 | TRAF4 | 0.577432006 | 0.023791558 | UP |
| Test 2 | TRAK2 | 0.711752054 | 0.000495145 | UP |
| Test 2 | TRAPPC2L | 0.909322771 | 1.74E-05 | UP |
| Test 2 | TRMT6 | 0.549132145 | 0.002369651 | UP |

**[Table 1-19]**

| | | | | |
|---|---|---|---|---|
| Test 2 | TRPT1 | 0.438418122 | 0.021480307 | UP |
| Test 2 | TSC2 | 0.397964507 | 0.021089712 | UP |
| Test 2 | TSPO | 0.345346267 | 0.025202467 | UP |
| Test 2 | TSR1 | 0.558146659 | 0.014222557 | UP |
| Test 2 | TTPAL | 0.459459863 | 0.034963074 | UP |
| Test 2 | TUBB2A | 0.525704007 | 0.020268788 | UP |
| Test 2 | TWF1 | 0.373452154 | 0.047899723 | UP |
| Test 2 | TXNDC17 | 0.714879216 | 0.000129111 | UP |
| Test 2 | TXNRD1 | 1.099101729 | 0.001183883 | UP |
| Test 2 | UBE2L3 | 0.526673618 | 0.000211555 | UP |
| Test 2 | UBL3 | 0.474834314 | 0.007289944 | UP |
| Test 2 | UBL5 | 0.376245303 | 0.005590702 | UP |
| Test 2 | UCHL3 | 0.538513933 | 0.012719666 | UP |
| Test 2 | UGP2 | 0.412622582 | 0.011054428 | UP |
| Test 2 | UNC50 | 0.424239923 | 0.033163478 | UP |
| Test 2 | UQCR10 | 0.337398522 | 0.0413842 | UP |
| Test 2 | **UQCRH** | **0.346746063** | **0.030618555** | **UP** |
| Test 2 | UTP6 | 0.477781959 | 0.037563094 | UP |
| Test 2 | VASN | 0.580611332 | 0.028789273 | UP |
| Test 2 | VPS4A | 0.509642105 | 0.028041104 | UP |
| Test 2 | VSIG8 | 0.679661792 | 0.024720039 | UP |
| Test 2 | WDR60 | 0.685964377 | 0.004520911 | UP |
| Test 2 | WDR61 | 0.451460615 | 0.04499615 | UP |
| Test 2 | WFDC12 | 0.911124303 | 0.015496389 | UP |
| Test 2 | WFDC5 | 0.630799027 | 0.035863131 | UP |
| Test 2 | WIBG | 0.471704215 | 0.039582096 | UP |
| Test 2 | WWTR1 | 0.715620692 | 0.004105629 | UP |
| Test 2 | XPOT | 0.468281083 | 0.045788227 | UP |
| Test 2 | YTHDF1 | 0.386322627 | 0.038012885 | UP |
| Test 2 | YTHDF2 | 0.62946844 | 0.002443494 | UP |
| Test 2 | ZFAND2A | 0.450586185 | 0.018177494 | UP |
| Test 2 | ZNF259 | 0.481902886 | 0.003455703 | UP |

**[Table 1-20]**

| | | | | |
|---|---|---|---|---|
| Test 2 | ABTB1 | -0.546411505 | 0.018144091 | DOWN |
| Test 2 | ADAM8 | -0.513306321 | 0.035447376 | DOWN |
| Test 2 | ADORA2A | -0.81885306 | 0.002331507 | DOWN |
| Test 2 | AGTRAP | -0.538277133 | 0.006210892 | DOWN |
| Test 2 | AGXT2L2 | -0.484250819 | 0.016167126 | DOWN |
| Test 2 | AHCYL1 | -0.414076682 | 0.031287665 | DOWN |
| Test 2 | ALPL | -0.732971219 | 0.037662124 | DOWN |
| Test 2 | **ANKRD12** | **-0.584058465** | **0.022801499** | **DOWN** |
| Test 2 | ANKRD17 | -0.405544382 | 0.007522217 | DOWN |
| Test 2 | ANKRD27 | -0.457996379 | 0.047461771 | DOWN |
| Test 2 | AP1G1 | -0.353067009 | 0.020214307 | DOWN |
| Test 2 | APH1A | -0.525725945 | 0.014352743 | DOWN |
| Test 2 | ARF1 | -0.23559285 | 0.007275376 | DOWN |
| Test 2 | ARF5 | -0.359483978 | 0.007087883 | DOWN |
| Test 2 | ARHGAP30 | -0.671059506 | 0.003502527 | DOWN |
| Test 2 | ARHGEF2 | -0.365713515 | 0.046292884 | DOWN |
| Test 2 | ARID3A | -0.5595524 | 0.020763141 | DOWN |
| Test 2 | ARL5B | -0.374255491 | 0.032148928 | DOWN |
| Test 2 | ARPC2 | -0.301380741 | 0.007856807 | DOWN |
| Test 2 | ATG2A | -0.491971315 | 0.004958653 | DOWN |
| Test 2 | ATHL1 | -0.839020018 | 0.002709554 | DOWN |
| Test 2 | ATP13A3 | -0.343948111 | 0.034155817 | DOWN |
| Test 2 | ATP6V0C | -0.528286893 | 0.000408234 | DOWN |
| Test 2 | ATP6V0D1 | -0.287114942 | 0.039585219 | DOWN |
| Test 2 | AURKAIP1 | -0.502619683 | 0.00958826 | DOWN |
| Test 2 | BAK1 | -0.487024115 | 0.040014771 | DOWN |
| Test 2 | BAP1 | -0.504388246 | 0.01853754 | DOWN |
| Test 2 | BMP2K | -0.61467808 | 0.039213696 | DOWN |
| Test 2 | BRD2 | -0.365257538 | 0.006933573 | DOWN |
| Test 2 | BSDC1 | -0.374730519 | 0.046154207 | DOWN |
| Test 2 | C15orf38 | -0.715339518 | 0.016230138 | DOWN |
| Test 2 | C17orf107 | -0.638711636 | 0.031899208 | DOWN |
| Test 2 | C22orf13 | -0.556229527 | 0.002139299 | DOWN |
| Test 2 | CAMK1D | -0.363332672 | 0.048425091 | DOWN |
| Test 2 | CANT1 | -0.619432538 | 0.005612133 | DOWN |
| Test 2 | CASP9 | -0.391150283 | 0.046236587 | DOWN |
| Test 2 | CCDC28A | -0.325084738 | 0.039941927 | DOWN |
| Test 2 | CCDC9 | -0.381074881 | 0.032466986 | DOWN |
| Test 2 | **CCL3** | **-0.647010847** | **0.028352908** | **DOWN** |
| Test 2 | **CCNI** | **-0.335231908** | **0.027018957** | **DOWN** |
| Test 2 | CCRL2 | -0.588173323 | 0.040593791 | DOWN |

**[Table 1-21]**

| | | | | |
|---|---|---|---|---|
| Test 2 | CD63 | -0.445497418 | 0.004230269 | DOWN |
| Test 2 | **CD83** | **-0.526594744** | **0.029159029** | **DOWN** |
| Test 2 | CD97 | -0.76193437 | 0.005030014 | DOWN |
| Test 2 | CDC42SE1 | -0.371810977 | 0.009492497 | DOWN |
| Test 2 | CDKN1A | -0.409759913 | 0.000877116 | DOWN |
| Test 2 | CFL1 | -0.235920095 | 0.045298064 | DOWN |
| Test 2 | CHD2 | -0.534365582 | 0.01031488 | DOWN |
| Test 2 | CIC | -0.780568746 | 0.001746158 | DOWN |
| Test 2 | **CNN2** | **-0.478206967** | **0.045041795** | **DOWN** |
| Test 2 | CRLF3 | -0.470829152 | 0.018171553 | DOWN |
| Test 2 | CSF1 | -0.859593671 | 0.001648257 | DOWN |
| Test 2 | **CSF2RB** | **-0.537088027** | **0.047037042** | **DOWN** |
| Test 2 | CSRNP1 | -0.702210165 | 0.000255859 | DOWN |
| Test 2 | CTBP2 | -0.482865997 | 0.039884842 | DOWN |
| Test 2 | CTDSP2 | -0.549610429 | 0.000270136 | DOWN |
| Test 2 | **CXCR4** | **-0.628204085** | **0.020358444** | **DOWN** |
| Test 2 | CYTH1 | -0.39211801 | 0.021273331 | DOWN |
| Test 2 | DBNL | -0.361312286 | 0.009931476 | DOWN |
| Test 2 | DCAF11 | -0.6326978 | 0.001569843 | DOWN |
| Test 2 | DENND5A | -0.563683623 | 0.011198898 | DOWN |
| Test 2 | DESI1 | -0.476146449 | 0.025976354 | DOWN |
| Test 2 | DGAT1 | -0.486566263 | 0.018563147 | DOWN |
| Test 2 | DNM2 | -0.613664304 | 0.034363766 | DOWN |
| Test 2 | DOT1L | -0.437381988 | 0.033054977 | DOWN |
| Test 2 | DUSP1 | -0.456302054 | 0.039191808 | DOWN |
| Test 2 | DUSP2 | -0.511851781 | 0.011855554 | DOWN |
| Test 2 | DUSP3 | -0.539021616 | 0.003981359 | DOWN |
| Test 2 | ECD | -0.44764865 | 0.027268857 | DOWN |
| Test 2 | EFHD2 | -0.472250359 | 0.032124386 | DOWN |
| Test 2 | EFR3A | -0.357748504 | 0.035446993 | DOWN |
| Test 2 | **EGR2** | **-0.299185803** | **0.033982561** | **DOWN** |
| Test 2 | EGR3 | -0.600613853 | 0.002514735 | DOWN |
| Test 2 | EIF2C4 | -0.507646506 | 0.040582559 | DOWN |
| Test 2 | EIF4EBP2 | -0.415388743 | 0.02944828 | DOWN |
| Test 2 | ELF1 | -0.414700395 | 0.046718101 | DOWN |
| Test 2 | EMP3 | -0.56727553 | 0.012074994 | DOWN |
| Test 2 | EPS15L1 | -0.43465134 | 0.021095706 | DOWN |
| Test 2 | FAM100B | -0.396320013 | 0.007456757 | DOWN |
| Test 2 | FAM193B | -0.816752521 | 0.006550877 | DOWN |
| Test 2 | FAM210A | -0.415999641 | 0.043646384 | DOWN |
| Test 2 | FAM32A | -0.441434954 | 0.00711492 | DOWN |

**[Table 1-22]**

| | | | | |
|---|---|---|---|---|
| Test 2 | FAM53C | -0.414646652 | 0.043215387 | DOWN |
| Test 2 | FBXO11 | -0.587567686 | 0.033095048 | DOWN |
| Test 2 | FCGRT | -0.593104023 | 0.019455764 | DOWN |
| Test 2 | FGR | -0.573604518 | 0.025328892 | DOWN |
| Test 2 | FLNA | -0.503978457 | 0.020310777 | DOWN |
| Test 2 | FNIP1 | -0.559259947 | 0.024530856 | DOWN |
| Test 2 | FOSB | -1.091622363 | 0.000150044 | DOWN |
| Test 2 | FOSL2 | -0.741546633 | 0.000377548 | DOWN |
| Test 2 | FOXN3 | -0.354637174 | 0.046745405 | DOWN |
| Test 2 | FOXO4 | -0.4667223 | 0.043783003 | DOWN |
| Test 2 | FURIN | -0.459105715 | 0.001341881 | DOWN |
| Test 2 | FZR1 | -0.364147622 | 0.028337243 | DOWN |
| Test 2 | GABARAPL1 | -0.55597523 | 0.006898537 | DOWN |
| Test 2 | GADD45B | -0.470481527 | 0.001471104 | DOWN |
| Test 2 | GAPVD1 | -0.410369844 | 0.017202036 | DOWN |
| Test 2 | GATAD2A | -0.427073771 | 0.023639602 | DOWN |
| Test 2 | GGA1 | -0.396427118 | 0.011108895 | DOWN |
| Test 2 | GLA | -0.432386163 | 0.046129953 | DOWN |
| Test 2 | GMIP | -0.439255443 | 0.025650159 | DOWN |
| Test 2 | GNB1 | -0.31847551 | 0.015581144 | DOWN |
| Test 2 | GNB2 | -0.319721149 | 0.049773636 | DOWN |
| Test 2 | GPR108 | -0.441903322 | 0.042000281 | DOWN |
| Test 2 | GPX1 | -0.419015476 | 0.012872784 | DOWN |
| Test 2 | GRAMD1A | -0.932263643 | 1.44E-05 | DOWN |
| Test 2 | GRK6 | -0.654615424 | 0.008370268 | DOWN |
| Test 2 | GRN | -0.551500985 | 0.014350263 | DOWN |
| Test 2 | GTPBP1 | -0.403503204 | 0.015278622 | DOWN |
| Test 2 | HEXIM1 | -0.365986502 | 0.049415504 | DOWN |
| Test 2 | HIPK3 | -0.463202659 | 0.018014847 | DOWN |
| Test 2 | HLA.A | -1.236792406 | 0.020861464 | DOWN |
| Test 2 | HLX | -0.624323089 | 0.020911612 | DOWN |
| Test 2 | HSPA4 | -0.59623271 | 0.022837699 | DOWN |
| Test 2 | IDS | -0.240881411 | 0.028746962 | DOWN |
| Test 2 | IER3 | -0.287520838 | 0.017217201 | DOWN |
| Test 2 | IMPDH1 | -0.610405695 | 0.010620152 | DOWN |
| Test 2 | IN080D | -0.37547378 | 0.007394184 | DOWN |
| Test 2 | INPP5K | -0.423372501 | 0.028673174 | DOWN |
| Test 2 | IQSEC1 | -0.390427136 | 0.017062257 | DOWN |
| Test 2 | IRAK2 | -0.658169882 | 0.010698571 | DOWN |
| Test 2 | IRS2 | -0.420496894 | 0.042158917 | DOWN |
| Test 2 | ISCU | -0.287869125 | 0.027433296 | DOWN |

**[Table 1-23]**

| | | | | |
|---|---|---|---|---|
| Test 2 | ISG20L2 | -0.294870703 | 0.042571274 | DOWN |
| Test 2 | ITGA5 | -0.468532816 | 0.03498499 | DOWN |
| Test 2 | ITGAM | -0.527588792 | 0.029840274 | DOWN |
| Test 2 | **ITGAX** | **-0.64770333** | **0.014582029** | **DOWN** |
| Test 2 | JARID2 | -0.490688966 | 0.018106974 | DOWN |
| Test 2 | JUNB | -0.293143391 | 0.04364956 | DOWN |
| Test 2 | KAT5 | -0.418414536 | 0.014457718 | DOWN |
| Test 2 | KDM6B | -0.692508948 | 0.021431857 | DOWN |
| Test 2 | KIAA0232 | -0.381744969 | 0.033575438 | DOWN |
| Test 2 | KIAA0513 | -0.533596569 | 0.029885073 | DOWN |
| Test 2 | KLF2 | -0.643760892 | 0.043067951 | DOWN |
| Test 2 | KLF6 | -0.548841797 | 0.000827261 | DOWN |
| Test 2 | KLHL2 | -0.943508712 | 0.003900525 | DOWN |
| Test 2 | LATS2 | -0.464918389 | 0.023816082 | DOWN |
| Test 2 | LILRB2 | -0.517795974 | 0.044488235 | DOWN |
| Test 2 | LIMS1 | -0.49409161 | 0.012867015 | DOWN |
| Test 2 | **LITAF** | **-0.329270813** | **0.029150473** | **DOWN** |
| Test 2 | LOC283070 | -0.442909512 | 0.028945909 | DOWN |
| Test 2 | LPAR2 | -0.547607501 | 0.027604213 | DOWN |
| Test 2 | LPCAT1 | -0.832260386 | 0.002421822 | DOWN |
| Test 2 | LSP1 | -0.601135718 | 0.002264273 | DOWN |
| Test 2 | LTBR | -0.485771016 | 0.02744313 | DOWN |
| Test 2 | MAF1 | -0.526440437 | 0.015395944 | DOWN |
| Test 2 | MAN2A1 | -0.652423245 | 0.045009558 | DOWN |
| Test 2 | MAP4K4 | -0.364791966 | 0.026304596 | DOWN |
| Test 2 | MAP7D1 | -0.445039844 | 0.012145517 | DOWN |
| Test 2 | MAPKAPK2 | -0.32646322 | 0.018751928 | DOWN |
| Test 2 | MECP2 | -0.759034568 | 0.000179172 | DOWN |
| Test 2 | MEF2D | -0.48601618 | 0.004466755 | DOWN |
| Test 2 | METRNL | -0.31396947 | 0.014421188 | DOWN |
| Test 2 | MGEA5 | -0.417769836 | 0.00311289 | DOWN |
| Test 2 | MIDN | -0.412523462 | 0.032312233 | DOWN |
| Test 2 | MKNK2 | -0.468504375 | 0.006063396 | DOWN |
| Test 2 | MLF2 | -0.522610118 | 0.00717405 | DOWN |
| Test 2 | MLLT6 | -0.562801463 | 0.012870027 | DOWN |
| Test 2 | MMP25 | -0.607156567 | 0.040654743 | DOWN |
| Test 2 | MTHFS | -0.620132887 | 0.008704266 | DOWN |
| Test 2 | MTMR14 | -0.500904907 | 0.027113371 | DOWN |
| Test 2 | MYADM | -0.532524278 | 0.032770779 | DOWN |
| Test 2 | MY09B | -0.465760356 | 0.012683393 | DOWN |
| Test 2 | NAA50 | -0.333033539 | 0.024091716 | DOWN |

**[Table 1-24]**

| | | | | |
|---|---|---|---|---|
| Test 2 | NAB1 | -0.41631867 | 0.034705696 | DOWN |
| Test 2 | NAGK | -0.400938958 | 0.039418511 | DOWN |
| Test 2 | NCF1B | -0.632988161 | 0.032521317 | DOWN |
| Test 2 | NCF1C | -0.564958434 | 0.023648103 | DOWN |
| Test 2 | NCOA1 | -0.35268749 | 0.025504935 | DOWN |
| Test 2 | NFKB2 | -0.686225906 | 0.006490871 | DOWN |
| Test 2 | NFKBIB | -0.417375331 | 0.020054211 | DOWN |
| Test 2 | NFKBID | -0.579020216 | 0.039512351 | DOWN |
| Test 2 | NINJ1 | -0.666521399 | 0.007758421 | DOWN |
| Test 2 | NLRC5 | -0.518289968 | 0.04615466 | DOWN |
| Test 2 | NOTCH2NL | -0.380526931 | 0.002073988 | DOWN |
| Test 2 | NRIP1 | -1.322958378 | 0.002632999 | DOWN |
| Test 2 | NUMB | -0.494870767 | 0.00364152 | DOWN |
| Test 2 | OGFR | -0.457666083 | 0.021935407 | DOWN |
| Test 2 | OS9 | -0.472649391 | 0.045293803 | DOWN |
| Test 2 | PAN3 | -0.490759714 | 0.037403044 | DOWN |
| Test 2 | PATL1 | -0.425494161 | 0.039431793 | DOWN |
| Test 2 | PCBP1 | -0.176849095 | 0.0308842 | DOWN |
| Test 2 | PDPK1 | -0.351764848 | 0.030720043 | DOWN |
| Test 2 | PER1 | -0.520214927 | 0.038720114 | DOWN |
| Test 2 | PFKFB3 | -0.371937997 | 0.012048698 | DOWN |
| Test 2 | PHF1 | -0.509490418 | 0.018640047 | DOWN |
| Test 2 | PIK3AP1 | -0.630445334 | 0.004184868 | DOWN |
| Test 2 | PIK3R5 | -0.612446475 | 0.004720621 | DOWN |
| Test 2 | PIM3 | -0.467577174 | 0.002878904 | DOWN |
| Test 2 | PITPNA | -0.474470422 | 0.00241514 | DOWN |
| Test 2 | PLAU | -0.65031011 | 0.029875395 | DOWN |
| Test 2 | PLEKHB2 | -0.305583054 | 0.044277802 | DOWN |
| Test 2 | PLEKHM3 | -0.368794416 | 0.029647876 | DOWN |
| Test 2 | PLIN5 | -0.676960181 | 0.015080446 | DOWN |
| Test 2 | PPP1R15A | -0.418072337 | 0.005793369 | DOWN |
| Test 2 | PPP1R18 | -0.506261932 | 0.019385963 | DOWN |
| Test 2 | PPP2R5C | -0.471507643 | 0.029204209 | DOWN |
| Test 2 | PPP4R1 | -0.578649371 | 0.006631286 | DOWN |
| Test 2 | PRR14 | -0.46051795 | 0.0377872 | DOWN |
| Test 2 | PRR24 | -0.39469883 | 0.038397986 | DOWN |
| Test 2 | PRRC2C | -0.383243267 | 0.047022553 | DOWN |
| Test 2 | PTGER4 | -0.467431527 | 0.024894507 | DOWN |
| Test 2 | PTK2B | -0.429404802 | 0.005990901 | DOWN |
| Test 2 | PTTG1IP | -0.481590933 | 0.044232468 | DOWN |
| Test 2 | RAB11FIP1 | -0.22457918 | 0.041085596 | DOWN |

**[Table 1-25]**

| | | | | |
|---|---|---|---|---|
| Test 2 | RAB20 | -0.640257296 | 0.027879149 | DOWN |
| Test 2 | RAB5C | -0.399368933 | 0.007878983 | DOWN |
| Test 2 | RALGDS | -0.524141036 | 0.022754244 | DOWN |
| Test 2 | RAP2C | -0.429682327 | 0.044030988 | DOWN |
| Test 2 | RBCK1 | -0.576800376 | 0.038892289 | DOWN |
| Test 2 | RBM39 | -0.42348233 | 0.016771793 | DOWN |
| Test 2 | RBM4 | -0.319253158 | 0.022873847 | DOWN |
| Test 2 | RELA | -0.484272048 | 0.003538465 | DOWN |
| Test 2 | RGS19 | -0.569964421 | 0.00660173 | DOWN |
| Test 2 | RHBDD2 | -0.351071124 | 0.041709589 | DOWN |
| Test 2 | RHEB | -0.372307356 | 0.006704257 | DOWN |
| Test 2 | **RHOA** | **-0.299449889** | **0.004939206** | **DOWN** |
| Test 2 | RHOB | -0.626515052 | 0.00782575 | DOWN |
| Test 2 | RILPL2 | -0.751957556 | 0.011595227 | DOWN |
| Test 2 | **RNASEK** | **-0.203072703** | **0.046581317** | **DOWN** |
| Test 2 | RNF13 | -0.445901036 | 0.045657953 | DOWN |
| Test 2 | RNF41 | -0.405181229 | 0.01043338 | DOWN |
| Test 2 | RTN4 | -0.45443723 | 0.003045171 | DOWN |
| Test 2 | RXRA | -0.438666828 | 0.003686277 | DOWN |
| Test 2 | RYBP | -0.450501153 | 0.006086994 | DOWN |
| Test 2 | SBN02 | -0.549309601 | 0.010378319 | DOWN |
| Test 2 | SCYL1 | -0.410832924 | 0.012148609 | DOWN |
| Test 2 | SDE2 | -0.345790438 | 0.046190193 | DOWN |
| Test 2 | SEC22B | -0.255374419 | 0.036042427 | DOWN |
| Test 2 | SEMA6B | -0.5268738 | 0.041383614 | DOWN |
| Test 2 | **SERINC1** | **-0.54365295** | **0.011959311** | **DOWN** |
| Test 2 | **SERP1** | **-0.296323781** | **0.027306968** | **DOWN** |
| Test 2 | SF3B2 | -0.309131592 | 0.04838585 | DOWN |
| Test 2 | SH3BP5 | -0.457133655 | 0.025096704 | DOWN |
| Test 2 | SHISA5 | -0.703515786 | 0.03999053 | DOWN |
| Test 2 | SIPA1 | -0.534045003 | 0.043975734 | DOWN |
| Test 2 | SIRPA | -0.367127888 | 0.004462404 | DOWN |
| Test 2 | SLC11A1 | -0.583530702 | 0.045040558 | DOWN |
| Test 2 | SLC15A3 | -0.482099344 | 0.041303655 | DOWN |
| Test 2 | SLC16A3 | -0.590518437 | 0.027963972 | DOWN |
| Test 2 | SLC25A6 | -0.401411655 | 0.018627665 | DOWN |
| Test 2 | SLC3A2 | -0.593512339 | 0.004761774 | DOWN |
| Test 2 | SLC43A2 | -0.717518005 | 0.003148231 | DOWN |
| Test 2 | SLC44A2 | -0.366045357 | 0.026415807 | DOWN |
| Test 2 | SLC6A6 | -0.696190042 | 0.0043814 | DOWN |
| Test 2 | SLC9A8 | -0.589473162 | 0.029051064 | DOWN |

**[Table 1-26]**

| | | | | |
|---|---|---|---|---|
| Test 2 | SLED1 | -0.693849284 | 0.028188168 | DOWN |
| Test 2 | SMG1P1 | -0.574995261 | 0.020839424 | DOWN |
| Test 2 | SPHK1 | -0.61543386 | 0.004539302 | DOWN |
| Test 2 | SQSTM1 | -0.257715842 | 0.046959382 | DOWN |
| Test 2 | SREBF2 | -0.69315397 | 0.006195022 | DOWN |
| Test 2 | **SRRM2** | **-0.44624329** | **0.010131156** | **DOWN** |
| Test 2 | SRXN1 | -0.393283821 | 0.048331481 | DOWN |
| Test 2 | STK40 | -0.443882447 | 0.001414866 | DOWN |
| Test 2 | STX11 | -0.537977782 | 0.004822597 | DOWN |
| Test 2 | STX3 | -0.52789925 | 0.015799785 | DOWN |
| Test 2 | STX6 | -0.616930664 | 0.036164799 | DOWN |
| Test 2 | STXBP2 | -0.3689503 | 0.021350599 | DOWN |
| Test 2 | SUPT6H | -0.353757744 | 0.027853669 | DOWN |
| Test 2 | TAF10 | -0.442411568 | 0.003533838 | DOWN |
| Test 2 | TANK | -0.545432207 | 0.031687959 | DOWN |
| Test 2 | TCF25 | -0.409012121 | 0.024330453 | DOWN |
| Test 2 | TCIRG1 | -0.614475203 | 0.007539619 | DOWN |
| Test 2 | TM9SF4 | -0.38535586 | 0.04829699 | DOWN |
| Test 2 | TMBIM6 | -0.297865078 | 0.008470387 | DOWN |
| Test 2 | TMEM123 | -0.343267776 | 0.009094089 | DOWN |
| Test 2 | TMEM167B | -0.359258554 | 0.007761458 | DOWN |
| Test 2 | TMEM183A | -0.34494503 | 0.03937267 | DOWN |
| Test 2 | TMEM66 | -0.271219311 | 0.031611284 | DOWN |
| Test 2 | TMX4 | -0.900650969 | 0.002108645 | DOWN |
| Test 2 | TNFAIP2 | -0.568352841 | 0.021862206 | DOWN |
| Test 2 | TNFAIP3 | -0.665510696 | 0.007064788 | DOWN |
| Test 2 | TNFRSF14 | -0.543798981 | 0.014449198 | DOWN |
| Test 2 | TOM1 | -0.352436371 | 0.011291364 | DOWN |
| Test 2 | TP531NP2 | -0.423916841 | 0.049618263 | DOWN |
| Test 2 | TRAPPC5 | -0.329107904 | 0.045571636 | DOWN |
| Test 2 | TSPAN13 | -0.440929311 | 0.032283555 | DOWN |
| Test 2 | TTYH3 | -0.4874383 | 0.043267217 | DOWN |
| Test 2 | UBAP2L | -0.542649397 | 0.002387811 | DOWN |
| Test 2 | UBE2D3 | -0.456380993 | 2.47E-05 | DOWN |
| Test 2 | UBR4 | -0.760894686 | 0.002561835 | DOWN |
| Test 2 | UCP2 | -0.552795075 | 0.002082641 | DOWN |
| Test 2 | UPF1 | -0.336863796 | 0.026989745 | DOWN |
| Test 2 | USB1 | -0.406612823 | 0.033709698 | DOWN |
| Test 2 | USF2 | -0.486370326 | 0.00456431 | DOWN |
| Test 2 | WBP2 | -0.50639192 | 0.002504203 | DOWN |
| Test 2 | WDR82 | -0.410119457 | 0.031724832 | DOWN |

**[Table 1-27]**

| | | | | |
|---|---|---|---|---|
| Test 2 | XPO6 | -0.597709348 | 0.046102314 | DOWN |
| Test 2 | YPEL5 | -0.287506377 | 0.038298209 | DOWN |
| Test 2 | ZC3H12A | -0.511217461 | 0.009065486 | DOWN |
| Test 2 | ZFP36 | -0.468506172 | 0.020859393 | DOWN |
| Test 2 | ZMIZ1 | -0.651337052 | 0.00341487 | DOWN |
| Test 2 | ZNFX1 | -0.447727612 | 0.044337198 | DOWN |
| Test 2 | ZZEF1 | -0.356247435 | 0.015261504 | DOWN |

A biological process (BP) and a KEGG pathway were searched for by gene ontology (GO) enrichment analysis by using the public database STRING. As a result, 30 and 39 KEGG pathways related to the gene group with increased or decreased expression in the PD patients were obtained in Test 1 and Test 2, respectively, and the term hsa05012 (Parkinson's disease) which indicates Parkinson's disease was found to be included in both the tests (Tables 2-1 and 2-2).

**[Table 2-1]**

| Test | Regulation | ID | Description | FDR |
|---|---|---|---|---|
| Test 1 | UP | hsa00190 | Oxidative phosphorylation | 1.73E-08 |
| Test 1 | UP | hsa04932 | Non-alcoholic fatty liver disease (NAFL D) | 4.79E-07 |
| Test 1 | UP | hsa05012 | Parkinson's disease | 3.00E-06 |
| Test 1 | UP | hsa05016 | Huntington's disease | 3.00E-06 |
| Test 1 | UP | hsa05010 | Alzheimer's disease | 7.01E-06 |
| Test 1 | UP | hsa04714 | Thermogenesis | 7.19E-06 |
| Test 1 | UP | hsa01100 | Metabolic pathways | 0.00028 |
| Test 1 | UP | hsa04260 | Cardiac muscle contraction | 0.00092 |
| Test 1 | UP | hsa03050 | Proteasome | 0.0014 |
| Test 1 | UP | hsa04723 | Retrograde endocannabinoid signaling | 0.0142 |
| Test 1 | UP | hsa05219 | Bladder cancer | 0.0174 |
| Test 1 | UP | hsa05169 | Epstein-Barr virus infection | 0.0374 |
| Test 1 | DOWN | hsa03010 | Ribosome | 1.27E-13 |
| Test 1 | DOWN | hsa04062 | Chemokine signaling pathway | 0.00017 |
| Test 1 | DOWN | hsa04144 | Endocytosis | 0.0065 |
| Test 1 | DOWN | hsa05132 | Salmonella infection | 0.0065 |
| Test 1 | DOWN | hsa05203 | Viral carcinogenesis | 0.0091 |
| Test 1 | DOWN | hsa04670 | Leukocyte transendothelial migration | 0.0114 |
| Test 1 | DOWN | hsa00061 | Fatty acid biosynthesis | 0.0139 |
| Test 1 | DOWN | hsa04014 | Ras signaling pathway | 0.0139 |
| Test 1 | DOWN | hsa05130 | Pathogenic Escherichia coli infection | 0.0139 |
| Test 1 | DOWN | hsa05100 | Bacterial invasion of epithelial cells | 0.0191 |
| Test 1 | DOWN | hsa05200 | Pathways in cancer | 0.0191 |
| Test 1 | DOWN | hsa05211 | Renal cell carcinoma | 0.0191 |
| Test 1 | DOWN | hsa04360 | Axon guidance | 0.0249 |
| Test 1 | DOWN | hsa04666 | Fc gamma R-mediated phagocytosis | 0.029 |
| Test 1 | DOWN | hsa05205 | Proteoglycans in cancer | 0.0328 |
| Test 1 | DOWN | hsa04066 | HIF-1 signaling pathway | 0.0329 |
| Test 1 | DOWN | hsa04810 | Regulation of actin cytoskeleton | 0.0344 |
| Test 1 | DOWN | hsa04722 | Neurotrophin signaling pathway | 0.0461 |

**[Table 2-2]**

| | | | | |
|---|---|---|---|---|
| Test 2 | UP | hsa03010 | Ribosome | 4.70E-17 |
| Test 2 | UP | hsa04714 | Thermogenesis | 1.98E-05 |
| Test 2 | UP | hsa05016 | Huntington's disease | 0.00022 |
| Test 2 | UP | hsa00190 | Oxidative phosphorylation | 0.00034 |
| Test 2 | UP | hsa05010 | Alzheimer's disease | 0.00034 |
| Test 2 | UP | hsa05012 | Parkinson's disease | 0.00056 |
| Test 2 | UP | hsa00280 | Valine, leucine and isoleucine degradation | 0.003 |
| Test 2 | UP | hsa03040 | Spliceosome | 0.0094 |
| Test 2 | UP | hsa01100 | Metabolic pathways | 0.0188 |
| Test 2 | DOWN | hsa04142 | Lysosome | 0.0035 |
| Test 2 | DOWN | hsa05152 | Tuberculosis | 0.0035 |
| Test 2 | DOWN | hsa04072 | Phospholipase D signaling pathway | 0.0064 |
| Test 2 | DOWN | hsa04144 | Endocytosis | 0.0064 |
| Test 2 | DOWN | hsa04380 | Osteoclast differentiation | 0.0064 |
| Test 2 | DOWN | hsa05203 | Viral carcinogenesis | 0.0064 |
| Test 2 | DOWN | hsa05134 | Legionellosis | 0.0069 |
| Test 2 | DOWN | hsa04062 | Chemokine signaling pathway | 0.013 |
| Test 2 | DOWN | hsa05167 | Kaposi's sarcoma-associated herpesvirus i nfection | 0.013 |
| Test 2 | DOWN | hsa05223 | Non-small cell lung cancer | 0.0131 |
| Test 2 | DOWN | hsa04151 | PI3K-Akt signaling pathway | 0.0168 |
| Test 2 | DOWN | hsa05212 | Pancreatic cancer | 0.019 |
| Test 2 | DOWN | hsa05202 | Transcriptional misregulation in cancer | 0.0194 |
| Test 2 | DOWN | hsa04130 | SNARE interactions in vesicular transport | 0.0296 |
| Test 2 | DOWN | hsa05200 | Pathways in cancer | 0.0296 |
| Test 2 | DOWN | hsa05210 | Colorectal cancer | 0.0296 |
| Test 2 | DOWN | hsa05213 | Endometrial cancer | 0.0296 |
| Test 2 | DOWN | hsa04064 | NF-kappa B signaling pathway | 0.0316 |
| Test 2 | DOWN | hsa04140 | Autophagy - animal | 0.0316 |
| Test 2 | DOWN | hsa04218 | Cellular senescence | 0.0316 |
| Test 2 | DOWN | hsa04721 | Synaptic vesicle cycle | 0.0316 |
| Test 2 | DOWN | hsa05216 | Thyroid cancer | 0.0316 |
| Test 2 | DOWN | hsa05222 | Small cell lung cancer | 0.0316 |
| Test 2 | DOWN | hsa04068 | FoxO signaling pathway | 0.0342 |
| Test 2 | DOWN | hsa04371 | Apelin signaling pathway | 0.037 |
| Test 2 | DOWN | hsa04010 | MAPK signaling pathway | 0.0408 |
| Test 2 | DOWN | hsa05133 | Pertussis | 0.0456 |
| Test 2 | DOWN | hsa05220 | Chronic myeloid leukemia | 0.0488 |
| Test 2 | DOWN | hsa04145 | Phagosome | 0.0495 |
| Test 2 | DOWN | hsa05110 | Vibrio cholerae infection | 0.0495 |

Previously reported literatures were checked about the relation to Parkinson's disease of the genes shown in Tables 1-1 to 1-27 described above which were differentially expressed in at least either Test 1 or Test 2. As a result, 21 genes shown in Table 3-1 among the genes differentially expressed in Test 1 and 92 genes shown in Tables 3-2 to 3-4 among the genes differentially expressed in Test 2 had not been reported so far on their relation to Parkinson's disease, demonstrating that these genes are capable of serving as novel markers for detecting Parkinson's disease. Genes indicated by boldface in the tables are common genes between Test 1 and Test 2.

**[Table 3-1]**

| Test | Symbol | Regulation |
|---|---|---|
| Test 1 | DUX4L4 | UP |
| Test 1 | GPBP1L1 | UP |
| Test 1 | KIAA0930 | UP |
| Test 1 | LOC100093631 | UP |
| Test 1 | LOC100506888 | UP |
| Test 1 | LOC349196 | UP |
| Test 1 | LOC401321 | UP |
| Test 1 | OR4F3 | UP |
| Test 1 | PQLC1 | UP |
| Test 1 | **REXO1L2P** | UP |
| Test 1 | **SNORA16A** | UP |
| Test 1 | **SNORA24** | UP |
| Test 1 | SNORA43 | UP |
| Test 1 | **SNORA50** | UP |
| Test 1 | SNORA8 | UP |
| Test 1 | TCEB3CL | UP |
| Test 1 | TTC9 | UP |
| Test 1 | USP17L5 | UP |
| Test 1 | USP17L6P | UP |
| Test 1 | ZNF33A | UP |
| Test 1 | SNORA53 | DOWN |

**[Table 3-2]**

| Test | Symbol | Regulation |
|---|---|---|
| Test 2 | ACSS3 | UP |
| Test 2 | C1orf52 | UP |
| Test 2 | C5orf43 | UP |
| Test 2 | COA1 | UP |
| Test 2 | FAM210B | UP |
| Test 2 | FAM25B | UP |
| Test 2 | FAM45A | UP |
| Test 2 | GTF3C6 | UP |
| Test 2 | HEATR5A | UP |
| Test 2 | IQCG | UP |
| Test 2 | ITPRIPL2 | UP |
| Test 2 | KIAA0240 | UP |
| Test 2 | KIAA1143 | UP |
| Test 2 | KRTAP1.5 | UP |
| Test 2 | KRTAP12.1 | UP |
| Test 2 | KRTAP12.2 | UP |
| Test 2 | KRTAP3.1 | UP |
| Test 2 | KRTAP5.3 | UP |
| Test 2 | LINC00675 | UP |
| Test 2 | LOC100505738 | UP |
| Test 2 | LOC550643 | UP |
| Test 2 | LOC646862 | UP |
| Test 2 | LRRC15 | UP |
| Test 2 | MICALCL | UP |
| Test 2 | PDE12 | UP |
| Test 2 | PINLYP | UP |
| Test 2 | **REXO1L2P** | UP |
| Test 2 | SCARNA12 | UP |
| Test 2 | SCARNA16 | UP |
| Test 2 | SCARNA6 | UP |
| Test 2 | SCARNA7 | UP |
| Test 2 | SF3B14 | UP |
| Test 2 | SLFN5 | UP |
| Test 2 | SLMO2 | UP |
| Test 2 | SMIM5 | UP |
| Test 2 | SNHG9 | UP |
| Test 2 | SNORA10 | UP |
| Test 2 | SNORA14B | UP |
| Test 2 | **SNORA16A** | UP |
| Test 2 | SNORA21 | UP |
| Test 2 | SNORA23 | UP |

**[Table 3-3]**

| | | |
|---|---|---|
| Test 2 | **SNORA24** | UP |
| Test 2 | SNORA33 | UP |
| Test 2 | SNORA34 | UP |
| Test 2 | SNORA49 | UP |
| Test 2 | **SNORA50** | UP |
| Test 2 | SNORA52 | UP |
| Test 2 | SNORA57 | UP |
| Test 2 | SNORA6 | UP |
| Test 2 | SNORA63 | UP |
| Test 2 | SNORA65 | UP |
| Test 2 | SNORA67 | UP |
| Test 2 | SNORA68 | UP |
| Test 2 | SNORA71A | UP |
| Test 2 | SNORA71B | UP |
| Test 2 | SNORA71C | UP |
| Test 2 | SNORA71D | UP |
| Test 2 | SNORA74B | UP |
| Test 2 | SNORA7B | UP |
| Test 2 | SNORA84 | UP |
| Test 2 | SNORA9 | UP |
| Test 2 | SNORD15B | UP |
| Test 2 | SNORD17 | UP |
| Test 2 | TM4SF19 | UP |
| Test 2 | TMEM179B | UP |
| Test 2 | TMEM45B | UP |
| Test 2 | TRMT6 | UP |
| Test 2 | UTP6 | UP |
| Test 2 | VSIG8 | UP |
| Test 2 | WDR60 | UP |
| Test 2 | WDR61 | UP |
| Test 2 | WFDC12 | UP |
| Test 2 | WIBG | UP |
| Test 2 | ARHGAP30 | DOWN |
| Test 2 | C17orf107 | DOWN |
| Test 2 | C22orf13 | DOWN |
| Test 2 | FAM100B | DOWN |
| Test 2 | FAM193B | DOWN |
| Test 2 | FAM210A | DOWN |
| Test 2 | FAM53C | DOWN |
| Test 2 | GPR108 | DOWN |
| Test 2 | GRAMD1A | DOWN |

**[Table 3-4]**

| | | |
|---|---|---|
| Test 2 | INO80D | DOWN |
| Test 2 | KIAA0232 | DOWN |
| Test 2 | MAP7D1 | DOWN |
| Test 2 | MLLT6 | DOWN |
| Test 2 | NCF1B | DOWN |
| Test 2 | PRR24 | DOWN |
| Test 2 | SDE2 | DOWN |
| Test 2 | SLED1 | DOWN |
| Test 2 | SMG1P1 | DOWN |
| Test 2 | TMEM167B | DOWN |

### ii) RNA expression analysis - 2

Data (read count values) on the expression level of RNA derived from the test subjects measured in the above section 2) was normalized by use of an approach called DESeq2. However, a sample in which 4161 or more genes were not detected was excluded, and only genes which produced expression level data without missing values in 90% or more sample test subjects in the expression level data on the test subjects in all the samples after exclusion were used in analysis given below. In the analysis, normalized count values obtained by use of an approach called DESeq2 were used.

Differentially expressed RNA which attained a corrected p value (FDR) of 0.25 or less in the likelihood ratio test in PD compared with the healthy subjects was identified on the basis of the SSL-derived RNA expression levels (normalized count values) of the healthy subjects and PD described above. In Test 1, the expression of 74 RNAs was increased in PD compared with the healthy subjects (Tables 4-1 and 4-2), and the expression of 209 RNAs was decreased therein (Tables 4-3 to 4-8). Meanwhile, in Test 2, the expression of 151 RNAs was increased (Tables 4-9 to 4-12), and the expression of 308 RNAs was decreased (Tables 4-13 to 4-20). The expression of 7 RNAs was increased in common between Test 1 and Test 2, and the expression of 10 RNAs was decreased in common therebetween (genes indicated by boldface in the tables).

**[Table 4-1]**

| Test | Symbol | Fold change | FDR | Regulation |
|---|---|---|---|---|
| Test 1 | ACOT2 | 2.120830751 | 0.171477109 | UP |
| Test 1 | ACOX3 | 1.905155929 | 0.192395571 | UP |
| Test 1 | ACTG1 | 0.591044977 | 0.216495961 | UP |
| Test 1 | AKT1S1 | 1.576633081 | 0.14231193 | UP |
| Test 1 | AMZ2 | 1.243802404 | 0.166431417 | UP |
| Test 1 | **ANXA1** | **1.686938977** | **0.032012546** | **UP** |
| Test 1 | ANXA2 | 1.075474933 | 0.166431417 | UP |
| Test 1 | **AQP3** | **2.056781943** | **0.207453699** | **UP** |
| Test 1 | AREG | 1.282649037 | 0.067390396 | UP |
| Test 1 | ARF5 | 0.81934585 | 0.218559941 | UP |
| Test 1 | ATP5E | 0.935310816 | 0.02664718 | UP |
| Test 1 | BCKDK | 1.060110294 | 0.057912524 | UP |
| Test 1 | BCR | 1.172433365 | 0.201130825 | UP |
| Test 1 | BSG | 1.119059971 | 0.247784004 | UP |
| Test 1 | C14orf2 | 0.678497763 | 0.213710325 | UP |
| Test 1 | CEBPA | 1.320916354 | 0.11590529 | UP |
| Test 1 | CHCHD2 | 1.191349912 | 0.004169691 | UP |
| Test 1 | CHMP5 | 1.072325081 | 0.069334816 | UP |
| Test 1 | COPE | 0.836423589 | 0.222937414 | UP |
| Test 1 | CORO1A | 1.434117261 | 0.142104577 | UP |
| Test 1 | CSDA | 0.738859106 | 0.242957593 | UP |
| Test 1 | DYNLT1 | 1.409545405 | 0.245101281 | UP |
| Test 1 | EIF4A3 | 1.316806302 | 0.102589353 | UP |
| Test 1 | **EMP1** | **2.274143956** | **0.060301659** | **UP** |
| Test 1 | FLII | 1.575063373 | 0.212740547 | UP |
| Test 1 | GPR157 | 1.469120858 | 0.04751536 | UP |
| Test 1 | GPX3 | 1.455160573 | 0.081351393 | UP |
| Test 1 | HSPA1A | 1.401246844 | 0.128335102 | UP |
| Test 1 | **KRT16** | **1.904813057** | **0.157035049** | **UP** |
| Test 1 | LOC100216546 | 1.822186187 | 0.192395571 | UP |
| Test 1 | LOC100288069 | 3.001066333 | 0.046401197 | UP |
| Test 1 | MESDC1 | 2.292885941 | 0.004532329 | UP |
| Test 1 | MIEN1 | 1.165319054 | 0.149707426 | UP |
| Test 1 | MKNK2 | 1.30619845 | 0.080683082 | UP |
| Test 1 | MNDA | 1.634027585 | 0.157035049 | UP |
| Test 1 | NEDD8 | 1.635919219 | 0.003678702 | UP |
| Test 1 | OTUD1 | 1.438403588 | 0.172366481 | UP |
| Test 1 | PIR | 1.736471923 | 0.187849008 | UP |
| Test 1 | PNISR | 1.817249957 | 0.166431417 | UP |
| Test 1 | POLR2J3 | 1.890184932 | 0.140445468 | UP |

**[Table 4-2]**

| | | | | |
|---|---|---|---|---|
| Test 1 | **POLR2L** | **1.140600646** | **0.205453026** | **UP** |
| Test 1 | PQLC1 | 1.211887627 | 0.137092675 | UP |
| Test 1 | PRELID1 | 0.985264241 | 0.185048528 | UP |
| Test 1 | PRKAA1 | 1.063955765 | 0.247784004 | UP |
| Test 1 | PSMA7 | 0.928612269 | 0.191314244 | UP |
| Test 1 | PSMD4 | 1.210300048 | 0.016937899 | UP |
| Test 1 | PTGS2 | 1.716429174 | 0.165136091 | UP |
| Test 1 | RASAL1 | 1.378706419 | 0.240134481 | UP |
| Test 1 | RNASET2 | 1.690750811 | 0.221565223 | UP |
| Test 1 | RNF217 | 1.665796573 | 0.180188714 | UP |
| Test 1 | RPL13 | 1.656906532 | 0.004532329 | UP |
| Test 1 | S100A8 | 1.385197789 | 0.211636176 | UP |
| Test 1 | SDC4 | 1.764459258 | 0.186471701 | UP |
| Test 1 | **SERPINB4** | **2.405038672** | **0.093218948** | **UP** |
| Test 1 | SLC25A3 | 0.957786481 | 0.097706633 | UP |
| Test 1 | SLPI | 1.223952779 | 0.240134481 | UP |
| Test 1 | **SNORA24** | **1.41317214** | **0.022725658** | **UP** |
| Test 1 | SNORA50 | 2.364388841 | 0.035336192 | UP |
| Test 1 | SNORA57 | 2.930672887 | 0.004532329 | UP |
| Test 1 | SNORA8 | 1.396949079 | 0.142963515 | UP |
| Test 1 | SNORA9 | 1.523014565 | 0.142763401 | UP |
| Test 1 | SOCS3 | 1.228282723 | 0.240134481 | UP |
| Test 1 | TIMP1 | 1.378822071 | 0.165136091 | UP |
| Test 1 | TMCC3 | 1.173598961 | 0.209941538 | UP |
| Test 1 | TRMT44 | 1.881661647 | 0.11590529 | UP |
| Test 1 | TSPO | 1.290086722 | 0.008973844 | UP |
| Test 1 | TUBA1C | 1.180331715 | 0.067390396 | UP |
| Test 1 | UQCRB | 0.953505252 | 0.166431417 | UP |
| Test 1 | UQCRC1 | 1.171312279 | 0.032012546 | UP |
| Test 1 | UQCRFS1 | 1.118741793 | 0.157035049 | UP |
| Test 1 | VEGFA | 1.170135516 | 0.14231193 | UP |
| Test 1 | ZFP36L2 | 1.597906298 | 0.212740547 | UP |
| Test 1 | ZNF410 | 1.411824416 | 0.017419551 | UP |
| Test 1 | ZSWIM6 | 1.164512926 | 0.200368845 | UP |

**[Table 4-3]**

| | | | | |
|---|---|---|---|---|
| Test 1 | AATF | -1.772740768 | 0.028713164 | DOWN |
| Test 1 | ADRBK2 | -1.565594145 | 0.214520377 | DOWN |
| Test 1 | AHSA1 | -1.855551649 | 0.04751536 | DOWN |
| Test 1 | AIDA | -1.498618103 | 0.137671023 | DOWN |
| Test 1 | ANKRD12 | -3.218993782 | 0.000308536 | DOWN |
| Test 1 | ANXA3 | -2.160717204 | 0.198639769 | DOWN |
| Test 1 | AP3B1 | -2.069780342 | 0.01186505 | DOWN |
| Test 1 | APH1A | -1.601000274 | 0.044757805 | DOWN |
| Test 1 | API5 | -2.46515534 | 0.022303042 | DOWN |
| Test 1 | APLP2 | -1.302316687 | 0.209941538 | DOWN |
| Test 1 | ARID4B | -2.568474682 | 0.013052784 | DOWN |
| Test 1 | ARPC1A | -1.745179478 | 0.179294587 | DOWN |
| Test 1 | ARPC3 | -1.326436202 | 0.04751536 | DOWN |
| Test 1 | ATG12 | -1.485488983 | 0.201316711 | DOWN |
| Test 1 | ATP2A2 | -1.658130821 | 0.11590529 | DOWN |
| Test 1 | ATP5J2 | -0.961630702 | 0.153747759 | DOWN |
| Test 1 | ATP6AP2 | -1.690782229 | 0.028713164 | DOWN |
| Test 1 | **ATP6V0C** | **-0.92893591** | **0.142104577** | **DOWN** |
| Test 1 | ATP6V1G1 | -0.835824694 | 0.17928974 | DOWN |
| Test 1 | BAG1 | -1.308033408 | 0.154290596 | DOWN |
| Test 1 | **BHLHE40** | **-1.574553746** | **0.003238712** | **DOWN** |
| Test 1 | BTF3 | -0.962144231 | 0.166431417 | DOWN |
| Test 1 | BTG1 | -1.205115472 | 0.069804405 | DOWN |
| Test 1 | BUD31 | -1.680224231 | 0.140744395 | DOWN |
| Test 1 | C14orf178 | -1.827426054 | 0.079281961 | DOWN |
| Test 1 | CAPZA1 | -1.035082105 | 0.212749025 | DOWN |
| Test 1 | CAPZA2 | -2.593953142 | 0.000573479 | DOWN |
| Test 1 | CBFB | -1.809532507 | 0.149707426 | DOWN |
| Test 1 | CCDC93 | -2.428995887 | 0.027093818 | DOWN |
| Test 1 | **CCL3** | **-2.617993487** | **0.022303042** | **DOWN** |
| Test 1 | **CCNI** | **-2.705241728** | **8.8856E-05** | **DOWN** |
| Test 1 | CDC42 | -1.694231647 | 0.000238125 | DOWN |
| Test 1 | CHMP2A | -1.807256465 | 7.81525E-05 | DOWN |
| Test 1 | CHMP2B | -1.356411536 | 0.044486644 | DOWN |
| Test 1 | CHMP3 | -1.308675973 | 0.11590529 | DOWN |
| Test 1 | CIRBP | -1.490579305 | 0.028713164 | DOWN |
| Test 1 | CLIC4 | -2.178997823 | 0.004532329 | DOWN |
| Test 1 | CLIP1 | -1.655621373 | 0.157035049 | DOWN |
| Test 1 | CLK1 | -1.575308686 | 0.238174085 | DOWN |
| Test 1 | CLNS1A | -2.441026451 | 0.067390396 | DOWN |
| Test 1 | CNBP | -1.490648544 | 0.00052874 | DOWN |

**[Table 4-4]**

| | | | | |
|---|---|---|---|---|
| Test 1 | COPB2 | -1.508519088 | 0.201672143 | DOWN |
| Test 1 | CPA4 | -2.565357457 | 0.06078135 | DOWN |
| Test 1 | CPM | -3.185069888 | 0.004169691 | DOWN |
| Test 1 | CS | -1.412980886 | 0.155875067 | DOWN |
| Test 1 | CSF1 | -2.232962038 | 0.089756063 | DOWN |
| Test 1 | **CXCR4** | **-1.852473527** | **0.024085385** | **DOWN** |
| Test 1 | CYBB | -1.547680875 | 0.131595057 | DOWN |
| Test 1 | DCUN1D1 | -3.29687005 | 0.003016508 | DOWN |
| Test 1 | DDX21 | -1.953597404 | 0.17928974 | DOWN |
| Test 1 | DDX5 | -0.951202357 | 0.157035049 | DOWN |
| Test 1 | DICER1 | -2.438540315 | 0.053386182 | DOWN |
| Test 1 | DLD | -2.382342017 | 0.104358929 | DOWN |
| Test 1 | DNAJC15 | -1.735325528 | 0.212749025 | DOWN |
| Test 1 | DNAJC3 | -1.80993238 | 0.007533471 | DOWN |
| Test 1 | DR1 | -2.223672473 | 0.006574907 | DOWN |
| Test 1 | EEF1B2 | -1.013792824 | 0.153777482 | DOWN |
| Test 1 | **EGR2** | **-0.988003468** | **0.166431417** | **DOWN** |
| Test 1 | EIF2S2 | -1.424601763 | 0.192395571 | DOWN |
| Test 1 | EIF5A | -0.627832441 | 0.209941538 | DOWN |
| Test 1 | ELF1 | -1.71759088 | 0.02334994 | DOWN |
| Test 1 | EML4 | -3.139954556 | 0.000956919 | DOWN |
| Test 1 | EP300 | -2.931533156 | 0.003577618 | DOWN |
| Test 1 | EPS15 | -1.351127393 | 0.110751825 | DOWN |
| Test 1 | ERBB2IP | -0.983202956 | 0.157035049 | DOWN |
| Test 1 | ETF1 | -2.099561074 | 0.000858271 | DOWN |
| Test 1 | ETV6 | -1.217282765 | 0.161738652 | DOWN |
| Test 1 | EVL | -2.13743363 | 0.201316711 | DOWN |
| Test 1 | EZR | -1.337706169 | 0.104520242 | DOWN |
| Test 1 | FAM100A | -1.441515396 | 0.231685231 | DOWN |
| Test 1 | FAM126A | -3.771877733 | 0.026955947 | DOWN |
| Test 1 | FAM160A1 | -1.673651499 | 0.043287134 | DOWN |
| Test 1 | FNTA | -4.395569996 | 0.032461153 | DOWN |
| Test 1 | FUBP1 | -3.812291612 | 0.000308536 | DOWN |
| Test 1 | FYTTD1 | -1.74478099 | 0.186471701 | DOWN |
| Test 1 | G3BP2 | -1.313544933 | 0.187849008 | DOWN |
| Test 1 | GABARAP | -1.10082081 | 0.156429231 | DOWN |
| Test 1 | **GABARAPL1** | **-1.322693883** | **0.060301659** | **DOWN** |
| Test 1 | GLTP | -2.377100594 | 0.11590529 | DOWN |
| Test 1 | GLTSCR2 | -1.989132848 | 0.004532329 | DOWN |
| Test 1 | GOLGA8B | -1.533924827 | 0.213256993 | DOWN |
| Test 1 | GRB2 | -1.22550846 | 0.154290596 | DOWN |

**[Table 4-5]**

| | | | | |
|---|---|---|---|---|
| Test 1 | HBP1 | -1.691481895 | 0.02450771 | DOWN |
| Test 1 | HELZ | -2.689866366 | 0.003678702 | DOWN |
| Test 1 | HIF1A | -1.224827769 | 0.238174085 | DOWN |
| Test 1 | HINT1 | -1.453762692 | 0.04751536 | DOWN |
| Test 1 | HINT3 | -1.947152032 | 0.140445468 | DOWN |
| Test 1 | HIST1H1E | -1.670140606 | 0.103600407 | DOWN |
| Test 1 | HMGN1 | -2.063165682 | 0.073126006 | DOWN |
| Test 1 | HNRNPA2B1 | -1.274269915 | 0.142104577 | DOWN |
| Test 1 | HNRNPK | -1.96640437 | 0.000238125 | DOWN |
| Test 1 | HNRNPU | -1.703715606 | 0.009237092 | DOWN |
| Test 1 | IARS2 | -2.502578081 | 0.04751536 | DOWN |
| Test 1 | ICAM1 | -2.383130311 | 0.162465282 | DOWN |
| Test 1 | IDE | -1.862223274 | 0.11590529 | DOWN |
| Test 1 | IER3IP1 | -2.129040887 | 0.191902648 | DOWN |
| Test 1 | JAK1 | -2.478429677 | 0.04751536 | DOWN |
| Test 1 | JMY | -2.263496873 | 0.155218477 | DOWN |
| Test 1 | KAT2B | -1.550256904 | 0.157035049 | DOWN |
| Test 1 | KIAA1551 | -1.367628259 | 0.228182221 | DOWN |
| Test 1 | KIF16B | -1.712088316 | 0.170079315 | DOWN |
| Test 1 | KLF10 | -2.507920855 | 0.01186505 | DOWN |
| Test 1 | KLF3 | -2.671224065 | 0.011682374 | DOWN |
| Test 1 | LGALSL | -1.868246009 | 0.165136091 | DOWN |
| Test 1 | MARCH7 | -1.358142867 | 0.242073043 | DOWN |
| Test 1 | MBD2 | -1.966385172 | 0.008794332 | DOWN |
| Test 1 | MBD6 | -2.243104033 | 0.157035049 | DOWN |
| Test 1 | MDM2 | -2.174980192 | 0.067390396 | DOWN |
| Test 1 | MED13L | -1.63902893 | 0.104520242 | DOWN |
| Test 1 | MED19 | -3.581005956 | 0.007535427 | DOWN |
| Test 1 | MRPL15 | -2.386765875 | 0.094888262 | DOWN |
| Test 1 | NAPA | -1.420133442 | 0.067390396 | DOWN |
| Test 1 | N R4A2 | -1.256772697 | 0.231685231 | DOWN |
| Test 1 | NRBF2 | -0.871916325 | 0.124019241 | DOWN |
| Test 1 | NRBP1 | -1.122530881 | 0.242957593 | DOWN |
| Test 1 | NSFP1 | -1.167024718 | 0.104520242 | DOWN |
| Test 1 | OGFRL1 | -1.459613162 | 0.06499322 | DOWN |
| Test 1 | P4HB | -0.938800796 | 0.184113444 | DOWN |
| Test 1 | PAIP2 | -1.484416116 | 0.04751536 | DOWN |
| Test 1 | PDXK | -1.265283201 | 0.246917684 | DOWN |
| Test 1 | PGK1 | -0.921105178 | 0.135858235 | DOWN |
| Test 1 | PGRMC2 | -2.309010058 | 0.142104577 | DOWN |
| Test 1 | PHF20L1 | -2.098809369 | 0.18841032 | DOWN |

**[Table 4-6]**

| | | | | |
|---|---|---|---|---|
| Test 1 | PHF5A | -2.478924839 | 0.04751536 | DOWN |
| Test 1 | PIKFYVE | -2.246740668 | 0.247784004 | DOWN |
| Test 1 | PLA2G7 | -1.92588687 | 0.126138663 | DOWN |
| Test 1 | POLR2A | -1.007640822 | 0.245101281 | DOWN |
| Test 1 | PTPN12 | -2.409768904 | 0.003238712 | DOWN |
| Test 1 | QARS | -2.502319653 | 0.004169691 | DOWN |
| Test 1 | RAB14 | -2.991933128 | 0.001713645 | DOWN |
| Test 1 | RAB9A | -1.966991656 | 0.214127581 | DOWN |
| Test 1 | RABGEF1 | -2.346307336 | 0.004532329 | DOWN |
| Test 1 | RAP1A | -1.627957688 | 0.006574907 | DOWN |
| Test 1 | RAP1B | -0.938277209 | 0.128335102 | DOWN |
| Test 1 | **RHOA** | **-0.846384811** | **0.166431417** | **DOWN** |
| Test 1 | RIOK3 | -1.537528915 | 0.201316711 | DOWN |
| Test 1 | RMND5A | -1.727209574 | 0.104520242 | DOWN |
| Test 1 | **RNASEK** | **-0.803995199** | **0.134092229** | **DOWN** |
| Test 1 | RPL10 | -2.075102838 | 0.002320011 | DOWN |
| Test 1 | RPL13AP20 | -0.833469251 | 0.173586614 | DOWN |
| Test 1 | RPL15 | -1.780679733 | 0.00823501 | DOWN |
| Test 1 | RPL19 | -0.964036328 | 0.174007936 | DOWN |
| Test 1 | RPL24 | -1.300785402 | 0.131657412 | DOWN |
| Test 1 | RPL26 | -1.353489893 | 0.079440025 | DOWN |
| Test 1 | RPL28 | -0.831266984 | 0.215698645 | DOWN |
| Test 1 | RPL36AL | -1.835109641 | 0.005258055 | DOWN |
| Test 1 | RPL5 | -1.795729934 | 0.021525976 | DOWN |
| Test 1 | RPL6 | -2.49204435 | 0.003942597 | DOWN |
| Test 1 | RPS20 | -1.754918062 | 0.004169691 | DOWN |
| Test 1 | RPS25 | -1.28675117 | 0.01535602 | DOWN |
| Test 1 | RPS9 | -0.961456424 | 0.053716153 | DOWN |
| Test 1 | S100A10 | -1.295076872 | 0.166431417 | DOWN |
| Test 1 | S100A11 | -1.030462079 | 0.154290596 | DOWN |
| Test 1 | SCAF11 | -1.77369601 | 0.11590529 | DOWN |
| Test 1 | SCYL2 | -1.943402128 | 0.013505503 | DOWN |
| Test 1 | SDF4 | - 2 .085196384 | 0.170106979 | DOWN |
| Test 1 | SEC11C | -2.792373921 | 0.008933234 | DOWN |
| Test 1 | SEC24A | -3.07035687 | 0.038419833 | DOWN |
| Test 1 | SEPT11 | -2.357033916 | 0.153777482 | DOWN |
| Test 1 | SEPT2 | -1.74873368 | 0.004680722 | DOWN |
| Test 1 | **SERINC1** | **-1.248273951** | **0.073126006** | **DOWN** |
| Test 1 | SERINC3 | -1.030053705 | 0.025949689 | DOWN |
| Test 1 | SERPINA12 | -3.986279444 | 0.033389704 | DOWN |
| Test 1 | SERPINB9 | -1.250923726 | 0.209941538 | DOWN |

**[Table 4-7]**

| | | | | |
|---|---|---|---|---|
| Test 1 | SERTAD2 | -2.029830502 | 0.192395571 | DOWN |
| Test 1 | SET | -1.937444712 | 0.007533471 | DOWN |
| Test 1 | SH3BGRL3 | -0.663305735 | 0.067390396 | DOWN |
| Test 1 | SLMO2 | -1.81329058 | 0.137966894 | DOWN |
| Test 1 | SMS | -2.704517802 | 0.045626018 | DOWN |
| Test 1 | SNAP29 | -1.590085581 | 0.16114262 | DOWN |
| Test 1 | SNORA53 | -1.586512685 | 0.15598759 | DOWN |
| Test 1 | SNX13 | -3.004474961 | 0.000999599 | DOWN |
| Test 1 | SNX9 | -1.779958051 | 0.028713164 | DOWN |
| Test 1 | SREK1IP1 | -1.839855519 | 0.154290596 | DOWN |
| Test 1 | SRSF5 | -0.984113666 | 0.13064644 | DOWN |
| Test 1 | SSR2 | -1.749337253 | 0.146923279 | DOWN |
| Test 1 | SSU72 | -1.245325192 | 0.027093818 | DOWN |
| Test 1 | STK24 | -2.734918584 | 0.000596053 | DOWN |
| Test 1 | STT3B | -1.951800228 | 0.150544954 | DOWN |
| Test 1 | TAF10 | -1.32452647 | 0.094888262 | DOWN |
| Test 1 | TAOK1 | -1.927349024 | 0.030576015 | DOWN |
| Test 1 | TERF2IP | -1.792863603 | 0.084366841 | DOWN |
| Test 1 | TLK2 | -2.605906707 | 0.170106979 | DOWN |
| Test 1 | TMA7 | -1.367895195 | 0.028713164 | DOWN |
| Test 1 | TMEM106B | -1.86835998 | 0.247784004 | DOWN |
| Test 1 | TMEM127 | -1.190703794 | 0.044486644 | DOWN |
| Test 1 | TMEM167B | -1.796713966 | 0.116792089 | DOWN |
| Test 1 | TNFSF13B | -1.78814654 | 0.131657412 | DOWN |
| Test 1 | TPGS2 | -1.809874669 | 0.11590529 | DOWN |
| Test 1 | TRAM1 | -1.839546005 | 0.092035865 | DOWN |
| Test 1 | TRIP12 | -1.725435313 | 0.025949689 | DOWN |
| Test 1 | TRPM7 | -2.038357771 | 0.182595713 | DOWN |
| Test 1 | TSG101 | -1.005237989 | 0.209643258 | DOWN |
| Test 1 | TXNL1 | -1.549871273 | 0.032012546 | DOWN |
| Test 1 | UBE2A | -1.592829916 | 0.088783965 | DOWN |
| Test 1 | UBE2B | -1.436513364 | 0.078520181 | DOWN |
| Test 1 | UBE2H | -3.405818637 | 0.004532329 | DOWN |
| Test 1 | USMG5 | -1.046390149 | 0.136226208 | DOWN |
| Test 1 | USP22 | -1.181507483 | 0.174760541 | DOWN |
| Test 1 | USP53 | -3.761488613 | 0.006574907 | DOWN |
| Test 1 | USP6NL | -1.79126036 | 0.192642777 | DOWN |
| Test 1 | USP7 | -1.993708629 | 0.079281961 | DOWN |
| Test 1 | WIPF1 | -2.742465049 | 0.000134039 | DOWN |
| Test 1 | WTAP | -1.446170337 | 0.200942058 | DOWN |
| Test 1 | XBP1 | -1.326123865 | 0.14231193 | DOWN |

**[Table 4-8]**

| | | | | |
|---|---|---|---|---|
| Test 1 | YWHAQ | -3.230153729 | 0.000308536 | DOWN |
| Test 1 | ZCRB1 | -2.455139576 | 0.104520242 | DOWN |
| Test 1 | ZMAT2 | -1.635539488 | 0.104520242 | DOWN |
| Test 1 | ZNF148 | -2.237573981 | 0.088783965 | DOWN |

**[Table 4-9]**

| | | | | |
|---|---|---|---|---|
| Test 2 | ALOX12B | 0.723735806 | 0.167674326 | UP |
| Test 2 | **ANXA1** | **0.789867752** | **0.014394956** | **UP** |
| Test 2 | **AQP3** | **0.599212307** | **0.197195688** | **UP** |
| Test 2 | ATP12A | 0.431246438 | 0.191525939 | UP |
| Test 2 | ATP5B | 0.209786056 | 0.203917134 | UP |
| Test 2 | ATP5I | 0.542925568 | 0.018324394 | UP |
| Test 2 | ATP5O | 0.365101203 | 0.108586621 | UP |
| Test 2 | BAG3 | 0.698056003 | 0.041402036 | UP |
| Test 2 | C6orf132 | 0.427761744 | 0.221553842 | UP |
| Test 2 | CALM1 | 0.261573948 | 0.180087728 | UP |
| Test 2 | CASP14 | 0.575805082 | 0.189823772 | UP |
| Test 2 | CAST | 0.391443433 | 0.073143922 | UP |
| Test 2 | CDSN | 0.418002881 | 0.233357246 | UP |
| Test 2 | CLIC3 | 1.046049107 | 0.032990086 | UP |
| Test 2 | CNFN | 0.840234841 | 0.003993974 | UP |
| Test 2 | COX4I1 | 0.246033363 | 0.114612949 | UP |
| Test 2 | COX8A | 0.239739307 | 0.185751097 | UP |
| Test 2 | CRABP2 | 0.875558417 | 0.002325233 | UP |
| Test 2 | CST6 | 0.405606922 | 0.230255192 | UP |
| Test 2 | CTSC | 0.543171172 | 0.248489765 | UP |
| Test 2 | DNAJA1 | 0.280489771 | 0.204723138 | UP |
| Test 2 | DYNLL1 | 0.264998709 | 0.248133155 | UP |
| Test 2 | EEF1B2 | 0.323291572 | 0.113394678 | UP |
| Test 2 | EIF1AX | 0.484502482 | 0.057323067 | UP |
| Test 2 | EIF3K | 0.451680409 | 0.021385054 | UP |
| Test 2 | ELF3 | 0.673774244 | 0.148695977 | UP |
| Test 2 | **EMP1** | **1.53672252** | **0.000620279** | **UP** |
| Test 2 | EPHX3 | 0.986214766 | 0.023042585 | UP |
| Test 2 | FABP9 | 1.266189045 | 0.001096741 | UP |
| Test 2 | GNB2L1 | 0.271603302 | 0.203917134 | UP |
| Test 2 | GRHL3 | 0.482949482 | 0.207332199 | UP |
| Test 2 | HIST1H4E | 0.685060675 | 0.03912317 | UP |
| Test 2 | HIST1H4H | 0.770040391 | 0.004197223 | UP |
| Test 2 | HMGCS1 | 0.365100839 | 0.246563533 | UP |
| Test 2 | HMOX2 | 0.417111371 | 0.079852349 | UP |
| Test 2 | HSP90AA1 | 0.33731324 | 0.228358936 | UP |
| Test 2 | HSPB1 | 0.370513163 | 0.20793407 | UP |
| Test 2 | IVL | 1.089468005 | 0.001079959 | UP |
| Test 2 | KLF5 | 0.637821501 | 0.203917134 | UP |
| Test 2 | KLK13 | 0.693077306 | 0.09306963 | UP |
| Test 2 | KLK7 | 0.637766385 | 0.102583472 | UP |

**[Table 4-10]**

| | | | | |
|---|---|---|---|---|
| Test 2 | KRT10 | 0.786915063 | 0.214896999 | UP |
| Test 2 | KRT14 | 0.556501136 | 0.094970832 | UP |
| Test 2 | **KRT16** | **0.398735989** | **0.203917134** | **UP** |
| Test 2 | KRT25 | 1.240192706 | 0.001079959 | UP |
| Test 2 | KRT27 | 1.042441735 | 0.007811119 | UP |
| Test 2 | KRT5 | 1.059130956 | 0.035341619 | UP |
| Test 2 | KRT6A | 0.539579298 | 0.094970832 | UP |
| Test 2 | KRT71 | 1.005044058 | 0.009445737 | UP |
| Test 2 | KRT72 | 0.953782057 | 0.019401367 | UP |
| Test 2 | KRT74 | 0.942811431 | 0.110496509 | UP |
| Test 2 | KRTAP5-3 | 0.864276264 | 0.240103288 | UP |
| Test 2 | KRTDAP | 0.532072112 | 0.094970832 | UP |
| Test 2 | LCE2C | 0.468549536 | 0.192091234 | UP |
| Test 2 | LCE2D | 0.445193874 | 0.230255192 | UP |
| Test 2 | LCE3D | 0.583545316 | 0.093863146 | UP |
| Test 2 | LCE3E | 0.589208528 | 0.087587726 | UP |
| Test 2 | LCN2 | 0.716728817 | 0.032990086 | UP |
| Test 2 | LNX1 | 0.777864317 | 0.019217132 | UP |
| Test 2 | LRRC15 | 0.63618229 | 0.127005018 | UP |
| Test 2 | NDRG2 | 0.34352027 | 0.204723138 | UP |
| Test 2 | NDUFA4L2 | 0.939272871 | 0.02821497 | UP |
| Test 2 | NDUFB11 | 0.372680705 | 0.176688503 | UP |
| Test 2 | NDUFB2 | 0.621006658 | 0.079217394 | UP |
| Test 2 | NDUFB8 | 0.382724687 | 0.137176161 | UP |
| Test 2 | NDUFS5 | 0.475805215 | 0.070897185 | UP |
| Test 2 | NSFL1C | 0.305386997 | 0.225295536 | UP |
| Test 2 | NUMA1 | 0.361671318 | 0.101888953 | UP |
| Test 2 | PDZK1IP1 | 0.714851787 | 0.203917134 | UP |
| Test 2 | PINLYP | 0.705842244 | 0.157749295 | UP |
| Test 2 | PKP1 | 0.464155403 | 0.180898511 | UP |
| Test 2 | PNP | 0.323977868 | 0.203917134 | UP |
| Test 2 | **POLR2L** | **0.388253793** | **0.070687016** | **UP** |
| Test 2 | PPL | 1.076945835 | 0.035341619 | UP |
| Test 2 | PPP2R2A | 0.337886437 | 0.236569609 | UP |
| Test 2 | PRR9 | 0.834659426 | 0.019217132 | UP |
| Test 2 | PRSS3 | 0.616978345 | 0.094970832 | UP |
| Test 2 | PSMC2 | 0.289510565 | 0.240103288 | UP |
| Test 2 | RBBP4 | 0.438634443 | 0.203917134 | UP |
| Test 2 | RMRP | 0.530656521 | 0.034346406 | UP |
| Test 2 | ROMO1 | 0.278856205 | 0.213337813 | UP |
| Test 2 | RPL10A | 0.267591219 | 0.19331656 | UP |

**[Table 4-11]**

| | | | | |
|---|---|---|---|---|
| Test 2 | RPL11 | 0.272049357 | 0.188973375 | UP |
| Test 2 | RPL12 | 0.272231447 | 0.203917134 | UP |
| Test 2 | RPL13A | 0.332882836 | 0.087496195 | UP |
| Test 2 | RPL18 | 0.23631901 | 0.203917134 | UP |
| Test 2 | RPL21 | 0.234057222 | 0.228358936 | UP |
| Test 2 | RPL26 | 0.275178488 | 0.203917134 | UP |
| Test 2 | RPL27 | 0.25361932 | 0.203917134 | UP |
| Test 2 | RPL27A | 0.286064033 | 0.203917134 | UP |
| Test 2 | RPL29 | 0.227323201 | 0.202573111 | UP |
| Test 2 | RPL3 | 0.267937405 | 0.185751097 | UP |
| Test 2 | RPL30 | 0.22303117 | 0.208405568 | UP |
| Test 2 | RPL32 | 0.366698061 | 0.05120094 | UP |
| Test 2 | RPL35 | 0.353161175 | 0.075426886 | UP |
| Test 2 | RPL36 | 0.321842862 | 0.084037369 | UP |
| Test 2 | RPL36A | 0.338476857 | 0.089104057 | UP |
| Test 2 | RPL37A | 0.429627149 | 0.035341619 | UP |
| Test 2 | RPL38 | 0.361505145 | 0.069516286 | UP |
| Test 2 | RPL7 | 0.407722145 | 0.013807457 | UP |
| Test 2 | RPL7A | 0.3865274 | 0.033308231 | UP |
| Test 2 | RPLP0 | 0.475836697 | 0.00985997 | UP |
| Test 2 | RPLP1 | 0.466227457 | 0.019217132 | UP |
| Test 2 | RPLP2 | 0.351641372 | 0.151691535 | UP |
| Test 2 | RPS10 | 0.278100919 | 0.129200547 | UP |
| Test 2 | RPS12 | 0.557211765 | 0.001079959 | UP |
| Test 2 | RPS15 | 0.343773569 | 0.061406696 | UP |
| Test 2 | RPS15A | 0.241359539 | 0.204723138 | UP |
| Test 2 | RPS18 | 0.545456358 | 0.003707257 | UP |
| Test 2 | RPS19 | 0.304856429 | 0.188426153 | UP |
| Test 2 | RPS21 | 0.299295052 | 0.230255192 | UP |
| Test 2 | RPS26 | 0.495375545 | 0.056702789 | UP |
| Test 2 | RPS28 | 0.346942763 | 0.045753193 | UP |
| Test 2 | RPS3 | 0.467901737 | 0.121272078 | UP |
| Test 2 | RPS4X | 0.403982139 | 0.053047353 | UP |
| Test 2 | RPS5 | 0.360449408 | 0.079217394 | UP |
| Test 2 | RPS6 | 0.323863058 | 0.083321514 | UP |
| Test 2 | RPS8 | 0.276154805 | 0.156533343 | UP |
| Test 2 | S100A14 | 0.77672344 | 0.035341619 | UP |
| Test 2 | S100A7 | 0.49351568 | 0.203917134 | UP |
| Test 2 | S100A7A | 0.82107465 | 0.057323067 | UP |
| Test 2 | S100A9 | 0.415203898 | 0.204723138 | UP |
| Test 2 | SBDS | 0.433629133 | 0.094970832 | UP |

**[Table 4-12]**

| | | | | |
|---|---|---|---|---|
| Test 2 | SBSN | 0.400950246 | 0.248266371 | UP |
| Test 2 | **SERPINB4** | **0.673429357** | **0.142882428** | **UP** |
| Test 2 | SERPINB5 | 0.437096337 | 0.185215103 | UP |
| Test 2 | SFN | 1.052956601 | 0.013115227 | UP |
| Test 2 | SLURP1 | 0.772094195 | 0.246563533 | UP |
| Test 2 | SNORA16A | 0.61798567 | 0.035341619 | UP |
| Test 2 | **SNORA24** | **0.379856346** | **0.249405298** | **UP** |
| Test 2 | SNORA52 | 0.527159643 | 0.109351493 | UP |
| Test 2 | SNORA63 | 0.384997588 | 0.248489765 | UP |
| Test 2 | SNORA68 | 0.601608716 | 0.045753193 | UP |
| Test 2 | SNORA71A | 0.545397641 | 0.114065157 | UP |
| Test 2 | SNORD15B | 0.48371262 | 0.126316838 | UP |
| Test 2 | SPRR1A | 0.428636075 | 0.19331656 | UP |
| Test 2 | SPRR1B | 0.495978239 | 0.101888953 | UP |
| Test 2 | SPRR2D | 0.974665073 | 0.001096741 | UP |
| Test 2 | SPRR2E | 0.723183713 | 0.019217132 | UP |
| Test 2 | SPRR2F | 0.886789503 | 0.029464953 | UP |
| Test 2 | TCHH | 1.050322557 | 0.004197223 | UP |
| Test 2 | TCHHL1 | 1.1133749 | 0.019217132 | UP |
| Test 2 | TMOD3 | 0.466106975 | 0.180087728 | UP |
| Test 2 | TMPRSS11E | 0.464901468 | 0.240103288 | UP |
| Test 2 | UBE2L3 | 0.495979301 | 0.003707257 | UP |
| Test 2 | UBL3 | 0.384648798 | 0.129200547 | UP |
| Test 2 | UQCR11 | 0.316274231 | 0.127005018 | UP |
| Test 2 | UQCRH | 0.330479444 | 0.156533343 | UP |
| Test 2 | UXT | 0.285545238 | 0.240103288 | UP |
| Test 2 | WWC1 | 0.509668226 | 0.241275153 | UP |
| Test 2 | WWTR1 | 0.5963163 | 0.101888953 | UP |

**[Table 4-13]**

| | | | | |
|---|---|---|---|---|
| Test 2 | A2M | -0.718075308 | 0.148123279 | DOWN |
| Test 2 | AADACL3 | -0.58202357 | 0.213337813 | DOWN |
| Test 2 | ABHD5 | -0.384116317 | 0.248266371 | DOWN |
| Test 2 | ABTB1 | -0.48310785 | 0.207332199 | DOWN |
| Test 2 | ACSL5 | -0.962971251 | 0.127005018 | DOWN |
| Test 2 | ADAM8 | -0.628191368 | 0.184292206 | DOWN |
| Test 2 | ADORA2A | -1.177098212 | 0.018559854 | DOWN |
| Test 2 | AGTRAP | -0.841095761 | 0.10645836 | DOWN |
| Test 2 | AKR7A2 | -0.599763384 | 0.178842249 | DOWN |
| Test 2 | ALPL | -0.832944054 | 0.240103288 | DOWN |
| Test 2 | AMPD2 | -0.618352376 | 0.240103288 | DOWN |
| Test 2 | ANKRD22 | -0.356210317 | 0.240103288 | DOWN |
| Test 2 | AP5B1 | -0.586537694 | 0.213337813 | DOWN |
| Test 2 | ARF1 | -0.211680435 | 0.097540633 | DOWN |
| Test 2 | ARF5 | -0.266734474 | 0.203917134 | DOWN |
| Test 2 | ARHGAP1 | -0.422844029 | 0.143273824 | DOWN |
| Test 2 | ARHGAP30 | -0.800282483 | 0.083321514 | DOWN |
| Test 2 | ARHGEF2 | -0.553024402 | 0.203917134 | DOWN |
| Test 2 | ARID3A | -0.972422942 | 0.122046617 | DOWN |
| Test 2 | ARL5B | -0.502970468 | 0.203976387 | DOWN |
| Test 2 | ARRB2 | -0.503495008 | 0.156533343 | DOWN |
| Test 2 | ASAH1 | -0.573971415 | 0.228358936 | DOWN |
| Test 2 | ATG2A | -0.506283174 | 0.036440805 | DOWN |
| Test 2 | ATHL1 | -1.056342135 | 0.050301518 | DOWN |
| Test 2 | **ATP6V0C** | **-0.454978704** | **0.029798738** | **DOWN** |
| Test 2 | BASP1 | -0.555927115 | 0.185751097 | DOWN |
| Test 2 | BCKDK | -0.31920705 | 0.203917134 | DOWN |
| Test 2 | BCL2L1 | -0.328248615 | 0.235721155 | DOWN |
| Test 2 | **BHLHE40** | **-0.401373324** | **0.189293656** | **DOWN** |
| Test 2 | BRD4 | -0.592405451 | 0.185751097 | DOWN |
| Test 2 | C17orf107 | -0.743835661 | 0.213337813 | DOWN |
| Test 2 | C1orf43 | -0.386791488 | 0.034346406 | DOWN |
| Test 2 | C22orf13 | -0.651369541 | 0.073352416 | DOWN |
| Test 2 | C2CD2 | -0.578876548 | 0.248266371 | DOWN |
| Test 2 | C6orf106 | -0.657660723 | 0.019217132 | DOWN |
| Test 2 | CANT1 | -0.715521843 | 0.191525939 | DOWN |
| Test 2 | CCDC86 | -0.492588982 | 0.240103288 | DOWN |
| Test 2 | **CCL3** | **-1.013989016** | **0.019217132** | **DOWN** |
| Test 2 | CCL3L3 | -1.008726691 | 0.019217132 | DOWN |
| Test 2 | CCL4 | -0.756395613 | 0.087496195 | DOWN |
| Test 2 | **CCNI** | **-0.297462333** | **0.191525939** | **DOWN** |

**[Table 4-14]**

| | | | | |
|---|---|---|---|---|
| Test 2 | CCNY | -0.412042019 | 0.127005018 | DOWN |
| Test 2 | CCRL2 | -0.793390941 | 0.12802663 | DOWN |
| Test 2 | CCSAP | -0.460516843 | 0.248489765 | DOWN |
| Test 2 | CD300A | -0.684472303 | 0.203917134 | DOWN |
| Test 2 | CD36 | -0.533682671 | 0.204049804 | DOWN |
| Test 2 | CD63 | -0.379385495 | 0.194315694 | DOWN |
| Test 2 | CD82 | -0.716610607 | 0.090458126 | DOWN |
| Test 2 | CD83 | -0.481612361 | 0.203917134 | DOWN |
| Test 2 | CD97 | -0.845340799 | 0.045753193 | DOWN |
| Test 2 | CDC14A | -0.646668837 | 0.101888953 | DOWN |
| Test 2 | CDC37 | -0.382406309 | 0.240103288 | DOWN |
| Test 2 | CDC42EP3 | -0.6817877 | 0.19331656 | DOWN |
| Test 2 | CDC42SE1 | -0.378168521 | 0.191525939 | DOWN |
| Test 2 | CDKN1A | -0.387560594 | 0.035341619 | DOWN |
| Test 2 | CEP76 | -0.945072617 | 0.073143922 | DOWN |
| Test 2 | CHD2 | -0.690227101 | 0.069311378 | DOWN |
| Test 2 | CHMP4B | -0.287164907 | 0.094913279 | DOWN |
| Test 2 | CHP1 | -0.291208979 | 0.238519536 | DOWN |
| Test 2 | CLMP | -0.729937574 | 0.225295536 | DOWN |
| Test 2 | CNN2 | -0.512609072 | 0.19331656 | DOWN |
| Test 2 | COTL1 | -0.455952536 | 0.203917134 | DOWN |
| Test 2 | CRKL | -0.30261375 | 0.248133155 | DOWN |
| Test 2 | CSF2RB | -0.678104076 | 0.155367959 | DOWN |
| Test 2 | CSF3R | -0.569585968 | 0.223914394 | DOWN |
| Test 2 | CSNK1G2 | -0.599862624 | 0.204723138 | DOWN |
| Test 2 | CSRNP1 | -0.781500951 | 0.014394956 | DOWN |
| Test 2 | CTSA | -0.33123967 | 0.248133155 | DOWN |
| Test 2 | CTSD | -0.379023691 | 0.240103288 | DOWN |
| Test 2 | CXCL16 | -0.548865027 | 0.188426153 | DOWN |
| Test 2 | **CXCR4** | **-0.655969209** | **0.097540633** | **DOWN** |
| Test 2 | CYTH4 | -0.700576961 | 0.184292206 | DOWN |
| Test 2 | DBNL | -0.423838322 | 0.160228921 | DOWN |
| Test 2 | DCAF11 | -0.617731883 | 0.203917134 | DOWN |
| Test 2 | DDX60L | -0.683358582 | 0.227224075 | DOWN |
| Test 2 | DENND5A | -0.668319578 | 0.148123279 | DOWN |
| Test 2 | DGAT2 | -0.360390084 | 0.236147723 | DOWN |
| Test 2 | DHCR24 | -0.591071219 | 0.154704386 | DOWN |
| Test 2 | DIRC2 | -0.516298908 | 0.236575098 | DOWN |
| Test 2 | DSCR3 | -0.542042929 | 0.211019733 | DOWN |
| Test 2 | DUSP1 | -0.541740484 | 0.188426153 | DOWN |
| Test 2 | DUSP2 | -0.689003628 | 0.03912317 | DOWN |

**[Table 4-15]**

| | | | | |
|---|---|---|---|---|
| Test 2 | DUSP3 | -0.59012828 | 0.122393306 | DOWN |
| Test 2 | DUSP4 | -0.634642504 | 0.235243914 | DOWN |
| Test 2 | ECE1 | -0.65379167 | 0.156533343 | DOWN |
| Test 2 | EFHD2 | -0.617790089 | 0.098631765 | DOWN |
| Test 2 | EFR3A | -0.501652521 | 0.187412757 | DOWN |
| Test 2 | **EGR2** | **-0.387465028** | **0.179929185** | **DOWN** |
| Test 2 | EGR3 | -0.721696305 | 0.035341619 | DOWN |
| Test 2 | EHBP1L1 | -0.581774222 | 0.130446509 | DOWN |
| Test 2 | EHD1 | -0.456876838 | 0.240103288 | DOWN |
| Test 2 | EID3 | -0.828295686 | 0.155367959 | DOWN |
| Test 2 | EIF1 | -0.193856817 | 0.240103288 | DOWN |
| Test 2 | EIF4EBP2 | -0.594264701 | 0.02821497 | DOWN |
| Test 2 | EIF4EBP3 | -0.641006049 | 0.240103288 | DOWN |
| Test 2 | ELL | -0.552384988 | 0.184292206 | DOWN |
| Test 2 | EMP3 | -0.54637512 | 0.127634739 | DOWN |
| Test 2 | EPS15L1 | -0.75105164 | 0.088334668 | DOWN |
| Test 2 | FADS2 | -0.634238349 | 0.188426153 | DOWN |
| Test 2 | FAM100B | -0.434221338 | 0.045753193 | DOWN |
| Test 2 | FAM193B | -1.065452843 | 0.039812849 | DOWN |
| Test 2 | FAM213A | -0.619927264 | 0.192977197 | DOWN |
| Test 2 | FAM32A | -0.451599652 | 0.038297933 | DOWN |
| Test 2 | FAM46C | -0.401691761 | 0.203917134 | DOWN |
| Test 2 | FFAR2 | -0.834337354 | 0.129055508 | DOWN |
| Test 2 | FGR | -0.714071655 | 0.074376997 | DOWN |
| Test 2 | FLNA | -0.501907163 | 0.205001407 | DOWN |
| Test 2 | FMNL1 | -0.568342039 | 0.191525939 | DOWN |
| Test 2 | FNIP1 | -0.534504704 | 0.225031103 | DOWN |
| Test 2 | FOSB | -1.497165708 | 0.001079959 | DOWN |
| Test 2 | FOSL2 | -0.650897973 | 0.038524973 | DOWN |
| Test 2 | FURIN | -0.387559873 | 0.083321514 | DOWN |
| Test 2 | **GABARAPL1** | **-0.427119307** | **0.02821497** | **DOWN** |
| Test 2 | GADD45B | -0.59479857 | 0.03912317 | DOWN |
| Test 2 | GAL | -0.457031303 | 0.246563533 | DOWN |
| Test 2 | GAS7 | -0.431198926 | 0.183527457 | DOWN |
| Test 2 | GDE1 | -0.423089167 | 0.240103288 | DOWN |
| Test 2 | GPR108 | -0.777015122 | 0.093471839 | DOWN |
| Test 2 | GPR157 | -0.527189631 | 0.101888953 | DOWN |
| Test 2 | GPSM3 | -0.592430722 | 0.191525939 | DOWN |
| Test 2 | GRAMD1A | -0.951769694 | 0.014394956 | DOWN |
| Test 2 | GRINA | -0.595457147 | 0.156533343 | DOWN |
| Test 2 | GRK6 | -0.83349196 | 0.054079361 | DOWN |

**[Table 4-16]**

| | | | | |
|---|---|---|---|---|
| Test 2 | GRN | -0.535715253 | 0.191525939 | DOWN |
| Test 2 | GTPBP1 | -0.46308194 | 0.148123279 | DOWN |
| Test 2 | HDAC7 | -0.490842046 | 0.248266371 | DOWN |
| Test 2 | HLA-A | -1.104833425 | 0.203917134 | DOWN |
| Test 2 | HPCAL1 | -0.447792645 | 0.216748647 | DOWN |
| Test 2 | HS3ST6 | -0.501663196 | 0.207332199 | DOWN |
| Test 2 | HSPA4 | -0.906581783 | 0.092289404 | DOWN |
| Test 2 | IDS | -0.255674167 | 0.191525939 | DOWN |
| Test 2 | IER3 | -0.441738596 | 0.034346406 | DOWN |
| Test 2 | IMPDH1 | -0.618750853 | 0.202573111 | DOWN |
| Test 2 | INPP5K | -0.39152519 | 0.179929185 | DOWN |
| Test 2 | IRAK2 | -0.941310019 | 0.041102123 | DOWN |
| Test 2 | IRF1 | -0.633328723 | 0.219872648 | DOWN |
| Test 2 | ITGA5 | -0.614100521 | 0.148123279 | DOWN |
| Test 2 | ITGAX | -0.584382378 | 0.191525939 | DOWN |
| Test 2 | ITPK1 | -0.681600843 | 0.167922188 | DOWN |
| Test 2 | JUNB | -0.405633107 | 0.174126215 | DOWN |
| Test 2 | KIAA0247 | -0.43354089 | 0.185751097 | DOWN |
| Test 2 | KIAA0368 | -0.386851905 | 0.149347833 | DOWN |
| Test 2 | KIAA0494 | -0.375961313 | 0.203917134 | DOWN |
| Test 2 | KIAA1191 | -0.759570647 | 0.07612678 | DOWN |
| Test 2 | KLF2 | -0.829622672 | 0.095805056 | DOWN |
| Test 2 | KLF6 | -0.615237069 | 0.032647959 | DOWN |
| Test 2 | LARP1 | -0.371691928 | 0.191525939 | DOWN |
| Test 2 | LGALS3 | -0.33660704 | 0.228358936 | DOWN |
| Test 2 | LILRB2 | -0.713217829 | 0.141991209 | DOWN |
| Test 2 | LILRB3 | -0.586252086 | 0.203917134 | DOWN |
| Test 2 | LIMK2 | -0.693871145 | 0.19331656 | DOWN |
| Test 2 | LITAF | -0.472582053 | 0.129200547 | DOWN |
| Test 2 | LOC146880 | -0.596953376 | 0.248133155 | DOWN |
| Test 2 | LOC729737 | -0.641814422 | 0.204723138 | DOWN |
| Test 2 | LPCAT1 | -1.010875742 | 0.013807457 | DOWN |
| Test 2 | LPIN1 | -0.520806257 | 0.196547493 | DOWN |
| Test 2 | LSP1 | -0.669555347 | 0.066340963 | DOWN |
| Test 2 | LTBR | -0.715104448 | 0.155367959 | DOWN |
| Test 2 | MAF1 | -0.475268842 | 0.232304966 | DOWN |
| Test 2 | MAP4K4 | -0.517139843 | 0.204723138 | DOWN |
| Test 2 | MAP7D1 | -0.449559995 | 0.189823772 | DOWN |
| Test 2 | MAPKAPK2 | -0.475983818 | 0.191525939 | DOWN |
| Test 2 | MARCKS | -0.564491232 | 0.18041519 | DOWN |
| Test 2 | MBOAT7 | -0.751160266 | 0.145937048 | DOWN |

**[Table 4-17]**

| | | | | |
|---|---|---|---|---|
| Test 2 | MEF2D | -0.626428106 | 0.045753193 | DOWN |
| Test 2 | MEGF9 | -0.362999714 | 0.203917134 | DOWN |
| Test 2 | MEPCE | -0.864615229 | 0.073987946 | DOWN |
| Test 2 | METRNL | -0.281506863 | 0.183513502 | DOWN |
| Test 2 | MGEA5 | -0.345655087 | 0.180032429 | DOWN |
| Test 2 | MKNK2 | -0.410364719 | 0.126316838 | DOWN |
| Test 2 | MLF2 | -0.387393069 | 0.075659228 | DOWN |
| Test 2 | MLLT6 | -0.882491218 | 0.041252415 | DOWN |
| Test 2 | MMP25 | -0.743721149 | 0.204723138 | DOWN |
| Test 2 | MSRB1 | -0.384733834 | 0.160228921 | DOWN |
| Test 2 | MTHFS | -0.57838818 | 0.191525939 | DOWN |
| Test 2 | MTMR14 | -0.690369051 | 0.156533343 | DOWN |
| Test 2 | MYO9B | -0.607502615 | 0.191525939 | DOWN |
| Test 2 | NAA50 | -0.444730906 | 0.019217132 | DOWN |
| Test 2 | NBEAL2 | -0.535965413 | 0.2421585 | DOWN |
| Test 2 | NCF1B | -0.929517474 | 0.094970832 | DOWN |
| Test 2 | NFKB2 | -0.926577772 | 0.023042585 | DOWN |
| Test 2 | NFKBIA | -0.494604251 | 0.189823772 | DOWN |
| Test 2 | NFKBIB | -0.580266689 | 0.221139649 | DOWN |
| Test 2 | NFKBID | -0.861315902 | 0.050427226 | DOWN |
| Test 2 | NFKBIE | -0.730971195 | 0.083321514 | DOWN |
| Test 2 | NINJ1 | -0.82050845 | 0.035341619 | DOWN |
| Test 2 | NIPBL | -0.390839696 | 0.188426153 | DOWN |
| Test 2 | NLRC5 | -0.794366743 | 0.185751097 | DOWN |
| Test 2 | NOTCH2NL | -0.334354965 | 0.094970832 | DOWN |
| Test 2 | NR4A3 | -0.589158721 | 0.249272492 | DOWN |
| Test 2 | NTAN1 | -0.620825777 | 0.126316838 | DOWN |
| Test 2 | OGDH | -0.409053089 | 0.156533343 | DOWN |
| Test 2 | OSM | -0.609522522 | 0.240103288 | DOWN |
| Test 2 | P2RY4 | -0.830041687 | 0.204723138 | DOWN |
| Test 2 | PACSIN2 | -0.430359253 | 0.196505966 | DOWN |
| Test 2 | PDHX | -0.681406483 | 0.24513842 | DOWN |
| Test 2 | PDLIM7 | -0.646265774 | 0.236147723 | DOWN |
| Test 2 | PER1 | -0.715031562 | 0.129200547 | DOWN |
| Test 2 | PFKL | -0.472924949 | 0.189087808 | DOWN |
| Test 2 | PHF1 | -0.616090955 | 0.203917134 | DOWN |
| Test 2 | PIK3AP1 | -0.754938139 | 0.131526721 | DOWN |
| Test 2 | PIK3R5 | -0.699813238 | 0.141991209 | DOWN |
| Test 2 | PILRA | -0.578293645 | 0.221139649 | DOWN |
| Test 2 | PIM2 | -0.543863209 | 0.248133155 | DOWN |
| Test 2 | PIM3 | -0.602922387 | 0.032647959 | DOWN |

**[Table 4-18]**

| | | | | |
|---|---|---|---|---|
| Test 2 | PITPNA | -0.666509912 | 0.026318959 | DOWN |
| Test 2 | PLAU | -0.978710234 | 0.035341619 | DOWN |
| Test 2 | PLEKHO2 | -0.55687838 | 0.224160094 | DOWN |
| Test 2 | POU5F1P3 | -0.893422599 | 0.204723138 | DOWN |
| Test 2 | PPP1CB | -0.242710496 | 0.184292206 | DOWN |
| Test 2 | PPP1R15A | -0.687500599 | 0.014394956 | DOWN |
| Test 2 | PPP1R18 | -0.70890583 | 0.185751097 | DOWN |
| Test 2 | PPP4R1 | -0.613615567 | 0.130446509 | DOWN |
| Test 2 | PSMF1 | -0.38322124 | 0.228358936 | DOWN |
| Test 2 | PTGER4 | -0.572972237 | 0.179929185 | DOWN |
| Test 2 | PTK2B | -0.43711103 | 0.126316838 | DOWN |
| Test 2 | PTPN6 | -0.506301773 | 0.200987694 | DOWN |
| Test 2 | PTTG1IP | -0.590740401 | 0.185751097 | DOWN |
| Test 2 | RAB11FIP1 | -0.25222007 | 0.17055456 | DOWN |
| Test 2 | RAB20 | -1.092595824 | 0.058926254 | DOWN |
| Test 2 | RAB27A | -0.398774147 | 0.151691535 | DOWN |
| Test 2 | RAB5B | -0.202685445 | 0.229460189 | DOWN |
| Test 2 | RAB5C | -0.423663208 | 0.130446509 | DOWN |
| Test 2 | RALGDS | -1.217008231 | 0.001079959 | DOWN |
| Test 2 | RANGAP1 | -0.552917543 | 0.087496195 | DOWN |
| Test 2 | RAP2A | -0.736519183 | 0.046019666 | DOWN |
| Test 2 | RBCK1 | -0.695750187 | 0.240103288 | DOWN |
| Test 2 | RBM39 | -0.384671412 | 0.207377895 | DOWN |
| Test 2 | RELA | -0.553248888 | 0.10472235 | DOWN |
| Test 2 | RHEB | -0.430417385 | 0.023042585 | DOWN |
| Test 2 | RHOA | -0.306833302 | 0.114612949 | DOWN |
| Test 2 | RHOB | -0.530555714 | 0.138694251 | DOWN |
| Test 2 | RILPL2 | -0.839108918 | 0.094970832 | DOWN |
| Test 2 | RIT1 | -0.681872442 | 0.155367959 | DOWN |
| Test 2 | **RNASEK** | **-0.263726846** | **0.189823772** | **DOWN** |
| Test 2 | RNF213 | -0.587786601 | 0.203917134 | DOWN |
| Test 2 | RTN4 | -0.471653936 | 0.045753193 | DOWN |
| Test 2 | RXRA | -0.462010218 | 0.094970832 | DOWN |
| Test 2 | RYBP | -0.469450448 | 0.203917134 | DOWN |
| Test 2 | SBNO2 | -0.639964031 | 0.121272078 | DOWN |
| Test 2 | SCARF1 | -0.891605529 | 0.082241574 | DOWN |
| Test 2 | SCD | -0.581730024 | 0.18328675 | DOWN |
| Test 2 | SCYL1 | -0.489185457 | 0.191525939 | DOWN |
| Test 2 | **SERINC1** | **-0.436066431** | **0.233336584** | **DOWN** |
| Test 2 | SH2B2 | -0.791605737 | 0.184132179 | DOWN |
| Test 2 | SH3BP5 | -0.614289416 | 0.118903158 | DOWN |

**[Table 4-19]**

| | | | | |
|---|---|---|---|---|
| Test 2 | SHISA5 | -0.70313086 | 0.200987694 | DOWN |
| Test 2 | SHKBP1 | -0.610047261 | 0.203917134 | DOWN |
| Test 2 | SIRPA | -0.512022506 | 0.03912317 | DOWN |
| Test 2 | SLC11A1 | -0.656308616 | 0.199786172 | DOWN |
| Test 2 | SLC15A3 | -0.637280648 | 0.205001407 | DOWN |
| Test 2 | SLC15A4 | -0.716205845 | 0.130446509 | DOWN |
| Test 2 | SLC31A1 | -0.451679847 | 0.159048563 | DOWN |
| Test 2 | SLC3A2 | -0.828553543 | 0.079852349 | DOWN |
| Test 2 | SLC41A1 | -1.010783773 | 0.079217394 | DOWN |
| Test 2 | SLC43A2 | -0.940136909 | 0.032647959 | DOWN |
| Test 2 | SLC43A3 | -0.690613412 | 0.225295536 | DOWN |
| Test 2 | SLC45A4 | -0.749047969 | 0.093863146 | DOWN |
| Test 2 | SLC6A6 | -0.758400864 | 0.073352416 | DOWN |
| Test 2 | SMG1P1 | -0.693226977 | 0.094970832 | DOWN |
| Test 2 | SNORA8 | -0.519845806 | 0.211019733 | DOWN |
| Test 2 | SORT1 | -0.623031599 | 0.050123349 | DOWN |
| Test 2 | SPHK1 | -1.108424501 | 0.016355202 | DOWN |
| Test 2 | SPINT2 | -0.339670123 | 0.203917134 | DOWN |
| Test 2 | SQSTM 1 | -0.276887228 | 0.240103288 | DOWN |
| Test 2 | SREBF2 | -1.227441548 | 0.007293838 | DOWN |
| Test 2 | SRP54 | -0.319771058 | 0.248266371 | DOWN |
| Test 2 | SRRM2 | -0.418303881 | 0.180898511 | DOWN |
| Test 2 | SRXN1 | -0.582979776 | 0.038524973 | DOWN |
| Test 2 | STK40 | -0.488001779 | 0.0381434 | DOWN |
| Test 2 | STX11 | -0.658724054 | 0.205349428 | DOWN |
| Test 2 | STX6 | -0.513619131 | 0.230255192 | DOWN |
| Test 2 | STXBP2 | -0.508585693 | 0.130446509 | DOWN |
| Test 2 | TAGAP | -0.801415208 | 0.118903158 | DOWN |
| Test 2 | TAP1 | -0.654126047 | 0.218710004 | DOWN |
| Test 2 | TCF25 | -0.461070498 | 0.230255192 | DOWN |
| Test 2 | TCIRG1 | -0.804126136 | 0.079217394 | DOWN |
| Test 2 | TECPR2 | -0.825139388 | 0.170236917 | DOWN |
| Test 2 | TEX264 | -0.505490645 | 0.227224075 | DOWN |
| Test 2 | TLE3 | -0.466144984 | 0.203917134 | DOWN |
| Test 2 | TMBIM6 | -0.291260917 | 0.207332199 | DOWN |
| Test 2 | TMEM123 | -0.395242663 | 0.05837947 | DOWN |
| Test 2 | TMEM134 | -0.472439908 | 0.204723138 | DOWN |
| Test 2 | TMEM189 | -0.563615464 | 0.204723138 | DOWN |
| Test 2 | TNFAIP2 | -0.891574563 | 0.035341619 | DOWN |
| Test 2 | TNFRSF14 | -0.722611949 | 0.19331656 | DOWN |
| Test 2 | TNIP1 | -0.425719633 | 0.122393306 | DOWN |

**[Table 4-20]**

| | | | | |
|---|---|---|---|---|
| Test 2 | TOM1 | -0.505066513 | 0.035341619 | DOWN |
| Test 2 | TPD52L2 | -0.276554324 | 0.248133155 | DOWN |
| Test 2 | TRIB1 | -0.555648849 | 0.183527457 | DOWN |
| Test 2 | TRIM25 | -0.486286342 | 0.216748647 | DOWN |
| Test 2 | TRPC4AP | -0.425236454 | 0.203917134 | DOWN |
| Test 2 | UBAP2L | -0.698597784 | 0.190692011 | DOWN |
| Test 2 | UBE2D3 | -0.400068279 | 0.016355202 | DOWN |
| Test 2 | UBIAD1 | -0.472019436 | 0.203917134 | DOWN |
| Test 2 | UBR4 | -0.790770601 | 0.068265083 | DOWN |
| Test 2 | UCP2 | -0.469130629 | 0.219872648 | DOWN |
| Test 2 | USF2 | -0.455624975 | 0.191525939 | DOWN |
| Test 2 | VOPP1 | -0.479532379 | 0.180898511 | DOWN |
| Test 2 | WBP2 | -0.508317102 | 0.036236726 | DOWN |
| Test 2 | WSB2 | -0.373814864 | 0.183776073 | DOWN |
| Test 2 | XPO6 | -0.62485095 | 0.248489765 | DOWN |
| Test 2 | YKT6 | -0.273245456 | 0.234837927 | DOWN |
| Test 2 | ZC3H12A | -0.776257578 | 0.019217132 | DOWN |
| Test 2 | ZFP36 | -0.505429212 | 0.197195688 | DOWN |
| Test 2 | ZFP36L1 | -0.618017539 | 0.155367959 | DOWN |
| Test 2 | ZHX2 | -0.7205355 | 0.16157383 | DOWN |
| Test 2 | ZMIZ1 | -0.802337523 | 0.079217394 | DOWN |

A biological process (BP) and a KEGG pathway were searched for by gene ontology (GO) enrichment analysis by using the public database STRING. As a result, 30 and 28 KEGG pathways related to the gene group with increased or decreased expression in the PD patients were obtained in Test 1 and Test 2, respectively, and the term hsa05012 (Parkinson's disease) which indicates Parkinson's disease was found to be included in both the tests (Tables 5-1 and 5-2) .

**[Table 5-1]**

| Test | Regulation | ID | Description | FDR |
|---|---|---|---|---|
| Test 1 | UP | hsa05016 | Huntington's disease | 0.0039 |
| Test 1 | UP | hsa04714 | Thermogenesis | 0.0048 |
| Test 1 | UP | hsa00190 | Oxidative phosphorylation | 0.0302 |
| Test 1 | UP | hsa04932 | Non-alcoholic fatty liver disease (NAFL D) | 0.0303 |
| Test 1 | UP | hsa05012 | Parkinson's disease | 0.0303 |
| Test 1 | UP | hsa04260 | Cardiac muscle contraction | 0.035 |
| Test 1 | UP | hsa05010 | Alzheimer's disease | 0.035 |
| Test 1 | UP | hsa01040 | Biosynthesis of unsaturated fatty acids | 0.0374 |
| Test 1 | UP | hsa05169 | Epstein-Barr virus infection | 0.0409 |
| Test 1 | UP | hsa04066 | HIF-1 signaling pathway | 0.0422 |
| Test 1 | UP | hsa03020 | RNA polymerase | 0.0472 |
| Test 1 | DOWN | hsa03010 | Ribosome | 0.00000294 |
| Test 1 | DOWN | hsa04144 | Endocytosis | 0.00022 |
| Test 1 | DOWN | hsa05203 | Viral carcinogenesis | 0.00024 |
| Test 1 | DOWN | hsa04670 | Leukocyte transendothelial migration | 0.00066 |
| Test 1 | DOWN | hsa05130 | Pathogenic Escherichia coli infection | 0.0026 |
| Test 1 | DOWN | hsa05323 | Rheumatoid arthritis | 0.0032 |
| Test 1 | DOWN | hsa04141 | Protein processing in endoplasmic retic ulum | 0.0053 |
| Test 1 | DOWN | hsa04068 | FoxO signaling pathway | 0.0057 |
| Test 1 | DOWN | hsa05211 | Renal cell carcinoma | 0.0057 |
| Test 1 | DOWN | hsa05168 | Herpes simplex infection | 0.0085 |
| Test 1 | DOWN | hsa05206 | MicroRNAs in cancer | 0.0098 |
| Test 1 | DOWN | hsa04621 | NOD-like receptor signaling pathway | 0.0176 |
| Test 1 | DOWN | hsa03040 | Spliceosome | 0.018 |
| Test 1 | DOWN | hsa04062 | Chemokine signaling pathway | 0.0255 |
| Test 1 | DOWN | hsa05100 | Bacterial invasion of epithelial cells | 0.0277 |
| Test 1 | DOWN | hsa05169 | Epstein-Barr virus infection | 0.0337 |
| Test 1 | DOWN | hsa04919 | Thyroid hormone signaling pathway | 0.0355 |
| Test 1 | DOWN | hsa04966 | Collecting duct acid secretion | 0.0453 |
| Test 1 | DOWN | hsa04140 | Autophagy - animal | 0.0469 |

**[Table 5-2]**

| | | | | |
|---|---|---|---|---|
| Test 2 | UP | hsa03010 | Ribosome | 1.12E-43 |
| Test 2 | UP | hsa00190 | Oxidative phosphorylation | 1.42E-09 |
| Test 2 | UP | hsa05010 | Alzheimer's disease | 0.000000186 |
| Test 2 | UP | hsa05012 | Parkinson's disease | 0.000000281 |
| Test 2 | UP | hsa04714 | Thermogenesis | 0.000000324 |
| Test 2 | UP | hsa05016 | Huntington's disease | 0.000000399 |
| Test 2 | U P | hsa04932 | Non-alcoholic fatty liver disease (NAFL D) | 0.0000257 |
| Test 2 | UP | hsa04915 | Estrogen signaling pathway | 0.00075 |
| Test 2 | UP | hsa04260 | Cardiac muscle contraction | 0.027 |
| Test 2 | UP | hsa04657 | IL-17 signaling pathway | 0.0453 |
| Test 2 | UP | hsa04723 | Retrograde endocannabinoid signaling | 0.0453 |
| Test 2 | DOWN | hsa04062 | Chemokine signaling pathway | 0.0015 |
| Test 2 | DOWN | hsa04064 | NF-kappa B signaling pathway | 0.0023 |
| Test 2 | DOWN | hsa04144 | Endocytosis | 0.0023 |
| Test 2 | DOWN | hsa04380 | Osteoclast differentiation | 0.0023 |
| Test 2 | DOWN | hsa04722 | Neurotrophin signaling pathway | 0.0041 |
| Test 2 | DOWN | hsa04920 | Adipocytokine signaling pathway | 0.0041 |
| Test 2 | DOWN | hsa05152 | Tuberculosis | 0.0041 |
| Test 2 | DOWN | hsa04142 | Lysosome | 0.0042 |
| Test 2 | DOWN | hsa04662 | B cell receptor signaling pathway | 0.0042 |
| Test 2 | DOWN | hsa05203 | Viral carcinogenesis | 0.0042 |
| Test 2 | DOWN | hsa04218 | Cellular senescence | 0.016 |
| Test 2 | DOWN | hsa04010 | MAPK signaling pathway | 0.037 |
| Test 2 | DOWN | hsa04060 | Cytokine-cytokine receptor interaction | 0.037 |
| Test 2 | DOWN | hsa04072 | Phospholipase D signaling pathway | 0.037 |
| Test 2 | DOWN | hsa04145 | Phagosome | 0.037 |
| Test 2 | DOWN | hsa05168 | Herpes simplex infection | 0.037 |
| Test 2 | DOWN | hsa05222 | Small cell lung cancer | 0.043 |

Previously reported literatures were checked about the relation to Parkinson's disease of the genes shown in Tables 4-1 to 4-20 described above which were differentially expressed in at least either Test 1 or Test 2. As a result, 19 genes shown in Table 6-1 among the genes differentially expressed in Test 1 and 30 genes shown in Table 6-2 among the genes differentially expressed in Test 2 had not been reported so far on their relation to Parkinson's disease, demonstrating that these genes are capable of serving as novel markers for detecting Parkinson's disease. Genes indicated by boldface in the tables are common genes between Test 1 and Test 2.

**[Table 6-1]**

| Test | Symbol | Regulation |
|---|---|---|
| Test 1 | LOC100288069 | UP |
| Test 1 | MESDC1 | UP |
| Test 1 | POLR2J3 | UP |
| Test 1 | PQLC1 | UP |
| Test 1 | **SNORA24** | UP |
| Test 1 | SNORA50 | UP |
| Test 1 | SNORA57 | UP |
| Test 1 | SNORA9 | UP |
| Test 1 | TRMT44 | UP |
| Test 1 | C14orf178 | DOWN |
| Test 1 | FAM100A | DOWN |
| Test 1 | FYTTD1 | DOWN |
| Test 1 | LGALSL | DOWN |
| Test 1 | NSFP1 | DOWN |
| Test 1 | SLMO2 | DOWN |
| Test 1 | SNORA53 | DOWN |
| Test 1 | SREK1IP1 | DOWN |
| Test 1 | SSU72 | DOWN |
| Test 1 | TMEM167B | DOWN |

**[Table 6-2]**

| Test | Symbol | Regulation |
|---|---|---|
| Test 2 | KRTAP5-3 | UP |
| Test 2 | LRRC15 | UP |
| Test 2 | PINLYP | UP |
| Test 2 | SNORA16A | UP |
| Test 2 | **SNORA24** | UP |
| Test 2 | SNORA52 | UP |
| Test 2 | SNORA63 | UP |
| Test 2 | SNORA68 | UP |
| Test 2 | SNORA71A | UP |
| Test 2 | SNORD15B | UP |
| Test 2 | AADACL3 | DOWN |
| Test 2 | ARHGAP30 | DOWN |
| Test 2 | C17orf107 | DOWN |
| Test 2 | C1orf43 | DOWN |
| Test 2 | C22orf13 | DOWN |
| Test 2 | CCDC86 | DOWN |
| Test 2 | CCSAP | DOWN |
| Test 2 | CYTH4 | DOWN |
| Test 2 | FAM100B | DOWN |
| Test 2 | FAM193B | DOWN |
| Test 2 | GPR108 | DOWN |
| Test 2 | GRAMD1A | DOWN |
| Test 2 | KIAA0494 | DOWN |
| Test 2 | KIAA1191 | DOWN |
| Test 2 | LOC729737 | DOWN |
| Test 2 | MAP7D1 | DOWN |
| Test 2 | MLLT6 | DOWN |
| Test 2 | NCF1B | DOWN |
| Test 2 | POU5F1P3 | DOWN |
| Test 2 | SMG1P1 | DOWN |

### Example 2 Preparation and verification of discriminant model - 1

### 1) Data used

In the data (read count values) on the expression level of SSL-derived RNA from the test subjects, data with a read count of less than 10 was treated as missing values, as in RNA expression analysis - 1 in Example 1. After conversion to RPM values which normalized the read count values for difference in the total number of reads among samples, the missing values were compensated for by use of an approach called singular value decomposition (SVD) imputation. However, only genes which produced expression level data without missing values in 80% or more samples in all the samples were used in analysis given below. In the construction of machine learning models, converted RPM values, logarithmic values of RPM value to base 2 (Log₂RPM values) were used in order to approximate the RPM values, which followed negative binominal distribution, to normal distribution.

### 2) Data set partitioning

In the RNA profile data set obtained from the test subjects of Test 1, RNA profile data from a total of 20 subjects (10 healthy subjects and 10 PD) was used as training data for PD prediction models, and RNA profile data from the remaining 10 subjects was used as test data for use in the evaluation of model precision. In the RNA profile data set obtained from the test subjects of Test 2, RNA profile data from a total of 80 subjects (40 healthy subjects and 40 PD) was used as training data for PD prediction models, and RNA profile data from the remaining 20 subjects was used as test data for use in the evaluation of model precision.

### 3) Selection of feature gene

18 RNAs whose expression was increased in common between Test 1 and Test 2 and 15 RNAs whose expression was decreased in common between Test 1 and Test 2, in the PD patients compared with the healthy subjects in RNA expression analysis - 1 in Example 1 (genes indicated by boldface in Tables 1-1 to 1-27) were selected as feature genes. Their expression level data was converted to principal components by principal component analysis. Then, the first to tenth principal components were used as explanatory variables. Among the 18 RNAs whose expression was increased in common between Test 1 and Test 2 and the 15 RNAs whose expression was decreased in common between Test 1 and Test 2 in the PD patients, 4 genes SNORA16A, SNORA24, SNORA50, and REXO1L2P were selected as feature genes. Their expression level data was converted to principal components by principal component analysis. Then, the first to fourth principal components were used as explanatory variables.

### 4) Model construction

Prediction model construction was carried out by using a value of each principal component obtained from expression level data (Log₂RPM values) on the feature genes selected as training data from SSL-derived RNA as an explanatory variable, and the healthy subjects (HL) and PD as objective variables. The prediction models were learned by 10-fold cross validation by using 7 algorithms random forest, linear kernel support vector machine (SVM linear), rbf kernel support vector machine (SVM rbf), neural network, generalized linear model, regularized linear discriminant analysis, and regularized logistic regression for each item to be predicted. As for each algorithm, the value of each principal component obtained from the feature gene expression levels (Log2RPM value) of the test data was input to the models thus learned to calculate a target predictive value for each prediction item. Recall, precision, and an F value which is a harmonic mean thereof are calculated from a predictive value and an actually measured value, and a model having the largest F value was selected as the optimum prediction model.

### 5) Results

Table 7 shows the algorithm used, the recall, the precision, and the F value of each item to be predicted. Figure 1 shows confusion matrix in which predictive values in the optimum prediction model and actually measured values were plotted in test data. Numeric values in the drawing represent the number of samples of each quadrant.

Table 8 shows results of calculating the variable importance of each feature gene when random forest was used in model construction.

F1 of the model obtained by using 4 genes SNORA16A, SNORA24, SNORA50, and REXO1L2P was 0.67 in Test 1, 0.75 in Test 2, and 0.76 in integrated Test 1 + Test 2, indicating that PD was predictable with this model. F1 of the model obtained by using a total of 33 genes including 18 RNAs with increased expression and 15 RNAs with decreased expression in the PD patients was 0.91 in Test 1, 0.80 in Test 2, and 0.82 in integrated Test 1 + Test 2, indicating that PD was more highly accurately predictable with this model.

**[Table 7]**

| | | | The number of RNA: 4 | | | | | | | The number of RNA: 33 | | | | | | |
|---|---|---|---|---|---|---|---|---|---|---|---|---|---|---|---|---|
| | | | Rf | SMVlinear | SVMrbf | Nnet | GLM | rLDA | rLogistic | Rf | SMVlinear | SVMrbf | Nnet | GLM | rLDA | rLogistic |
| Test 1 | Test data | Precision | 0.75 | 0.6 | 0.6 | 0.5 | 0.5 | 0.67 | 0.5 | 0.83 | 0.83 | 0.83 | 0.83 | 0.83 | 0.83 | 0.83 |
| | | Recall | 0.6 | 0.6 | 0.6 | 0.2 | 0.2 | 0.4 | 0.2 | 1 | 1 | 1 | 1 | 1 | 1 | 1 |
| | | F-measure | 0.67 | 0.6 | 0.6 | 0.29 | 0.29 | 0.5 | 0.29 | 0.91 | 0.91 | 0.91 | 0.91 | 0.91 | 0.91 | 0.91 |
| | Training data | Precision | 1 | 0.83 | 0.91 | 0.91 | 0.91 | 0.82 | 0.9 | 1 | 1 | 1 | 1 | 1 | 1 | 1 |
| | | Recall | 1 | 1 | 1 | 1 | 1 | 0.9 | 0.9 | 1 | 1 | 1 | 1 | 1 | 1 | 1 |
| | | F-measure | 1 | 0.91 | 0.95 | 0.95 | 0.95 | 0.86 | 0.9 | 1 | 1 | 1 | 1 | 1 | 1 | 1 |
| Test 2 | Test data | Precision | 0.64 | 0.64 | 0.58 | 0.64 | 0.7 | 0.67 | 0.7 | 0.78 | 0.6 | 0.69 | 0.8 | 0.67 | 0.6 | 0.69 |
| | | Recall | 0.9 | 0.7 | 0.7 | 0.7 | 0.7 | 0.8 | 0.7 | 0.7 | 0.6 | 0.9 | 0.8 | 0.6 | 0.6 | 0.9 |
| | | F-measure | 0.75 | 0.67 | 0.64 | 0.67 | 0.7 | 0.73 | 0.7 | 0.74 | 0.6 | 0.78 | 0.8 | 0.63 | 0.6 | 0.78 |
| | Training data | Precision | 1 | 0.76 | 0.79 | 0.78 | 0.77 | 0.74 | 0.71 | 1 | 0.74 | 0.97 | 1 | 0.76 | 0.77 | 0.73 |
| | | Recall | 1 | 0.78 | 0.83 | 0.88 | 0.75 | 0.78 | 0.63 | 1 | 0.65 | 0.95 | 1 | 0.73 | 0.68 | 0.75 |
| | | F-measure | 1 | 0.77 | 0.8 | 0.82 | 0.76 | 0.76 | 0.67 | 1 | 0.69 | 0.96 | 1 | 0.74 | 0.72 | 0.74 |
| Test 1 + Test 2 | Test data | Precision | 0.53 | 0.68 | 0.61 | 0.63 | 0.71 | 0.72 | 0.54 | 0.74 | 0.78 | 0.78 | 0.74 | 0.78 | 0.78 | 0.76 |
| | | Recall | 0.56 | 0.81 | 0.69 | 0.75 | 0.75 | 0.81 | 0.44 | 0.88 | 0.88 | 0.88 | 0.88 | 0.88 | 0.88 | 0.81 |
| | | F-measure | 0.55 | 0.74 | 0.65 | 0.69 | 0.73 | 0.76 | 0.48 | 0.8 | 0.82 | 0.82 | 0.8 | 0.82 | 0.82 | 0.79 |
| | Training data | Precision | 1 | 0.66 | 0.77 | 0.71 | 0.69 | 0.68 | 0.53 | 1 | 0.84 | 0.92 | 1 | 0.82 | 0.8 | 0.78 |
| | | Recall | 1 | 0.71 | 0.82 | 0.92 | 0.67 | 0.65 | 0.37 | 1 | 0.86 | 0.94 | 1 | 0.82 | 0.84 | 0.82 |
| | | F-measure | 1 | 0.69 | 0.79 | 0.8 | 0.68 | 0.67 | 0.43 | 1 | 0.85 | 0.93 | 1 | 0.82 | 0.82 | 0.8 |

| | | | | | | | | | | | | | | | | |
|---|---|---|---|---|---|---|---|---|---|---|---|---|---|---|---|---|
| *Rf, random forest; SVMlinear, linear kernel support vector machine; SVMrbf, rbf kernel support vector machine; Nnet, neural network; GLM, generalized linear model; rLDA, regularized linear discriminant analysis; rLogistic, regularized 1 ogistic regression | | | | | | | | | | | | | | | | |

**[Table 8]**

| The number of feature RNA: 4 | | The number of feature RNA: 33 | |
|---|---|---|---|
| Gene | Importance | Gene | Importance |
| SNORA16A | 0.280469095 | EGR2 | 0.121039691 |
| SNORA24 | 0.274323927 | RHOA | 0.113763948 |
| SNORA50 | 0.24669339 | CCNI | 0.093092191 |
| REXO1L2P | 0.198513588 | RNASEK | 0.063837117 |
| | | CSF2RB | 0.048802707 |
| | | SERP1 | 0.048409696 |
| | | ANKRD12 | 0.045938856 |
| | | SLC25A3 | 0.041588563 |
| | | SNORA16A | 0.039001187 |
| | | CD83 | 0.030624415 |
| | | CXCR4 | 0.027441137 |
| | | ITGAX | 0.026515533 |
| | | UQCRH | 0.024491485 |
| | | SNORA24 | 0.024265663 |
| | | KCNQ1OT1 | 0.022758123 |
| | | CCL3 | 0.022737515 |
| | | C10orf116 | 0.018907367 |
| | | SERPINB4 | 0.018665702 |
| | | LCE3D | 0.01686108 |
| | | CNFN | 0.016538758 |
| | | SNORA50 | 0.015782887 |
| | | CNN2 | 0.013610312 |
| | | SNRPG | 0.012844074 |
| | | SRRM2 | 0.012694083 |
| | | RPL7A | 0.012650305 |
| | | NDUFA4L2 | 0.012282458 |
| | | RPS26 | 0.011473664 |
| | | REXO1L2P | 0.007799926 |
| | | EMP1 | 0.007547062 |
| | | POLR2L | 0.00754434 |
| | | SERINC1 | 0.007300344 |
| | | NDUFS5 | 0.006761863 |
| | | LITAF | 0.006427944 |

### Example 3 Preparation and verification of discriminant model - 2

### 1) Data used

Data (read count values) on the expression level of SSL-derived RNA from the test subjects was normalized by use of an approach called DESeq2, as in RNA expression analysis -2 in Example 1. However, a sample in which 4161 or more genes were not detected was excluded, and only genes which produced expression level data without missing values in 90% or more sample test subjects in the expression level data on the test subjects in all the samples after exclusion were used in analysis given below. In the analysis, normalized count values obtained by use of an approach called DESeq2 were used.

### 2) Data set partitioning

In the RNA profile data set obtained from the test subjects of Test 1, RNA profile data from a total of 15 subjects (9 healthy subjects and 6 PD) was used as training data for PD prediction models, and RNA profile data from a total of 5 subjects (the remaining 4 healthy subjects and 1 PD) was used as test data for use in the evaluation of model precision. In the RNA profile data set obtained from the test subjects of Test 2, RNA profile data from a total of 72 subjects (37 healthy subjects and 35 PD) was used as training data for PD prediction models, and RNA profile data from a total of 24 subjects (the remaining 13 healthy subjects and 11 PD) was used as test data for use in the evaluation of model precision.

### 3) Selection of feature gene

17 RNAs whose expression was increased or decreased in common between Test 1 and Test 2 in the PD patients compared with the healthy subjects in RNA expression analysis - 2 in Example 1 (genes indicated by boldface in Tables 4-1 to 4-20) were selected as feature genes. Their expression level data was converted to principal components by principal component analysis. Then, the first to fourth principal components were used as explanatory variables.

### 4) Model construction

Prediction model construction was carried out by using a value of each principal component obtained from expression level data (logarithmic values to base 2 of normalized count values plus 1) on the feature genes selected as training data from SSL-derived RNA as an explanatory variable, and the healthy subjects (HL) and PD as objective variables. The prediction models were learned by 10-fold cross validation by using 7 algorithms random forest, linear kernel support vector machine (SVM linear), rbf kernel support vector machine (SVM rbf), neural network, generalized linear model, regularized linear discriminant analysis, and regularized logistic regression for each item to be predicted. As for each algorithm, the value of each principal component obtained from the feature gene expression levels (logarithmic values to base 2 of normalized count values plus 1) of the test data was input to the models thus learned to calculate a target predictive value for each prediction item. Recall, precision, and an F value which is a harmonic mean thereof are calculated from a predictive value and an actually measured value, and a model having the largest F value was selected as the optimum prediction model.

### 5) Results

Table 9 shows the algorithm used, the recall, the precision, and the F value of each item to be predicted.

The F value of the model obtained by using 17 RNAs whose expression was increased or decreased in common between Test 1 and Test 2 in results of the likelihood ratio test after normalization by DESeq2 was 1 in Test 1 and 0.87 in Test 2, indicating that PD was predictable with this model.

**[Table 9]**

| | | | The number of RNA: 17 | | | | | | |
|---|---|---|---|---|---|---|---|---|---|
| | | | Rf | SMVlinear | SVMrbf | Nnet | GLM | rLDA | rLogistic |
| Test 1 | Test data | Precision | 1 | 1 | 1 | 1 | 1 | 1 | 1 |
| | | Recall | 1 | 1 | 1 | 1 | 1 | 1 | 1 |
| | | F-measure | 1 | 1 | 1 | 1 | 1 | 1 | 1 |
| | Training data | Precision | 1 | 0.86 | 1 | 1 | 1 | 0.86 | 0.86 |
| | | Recall | 1 | 1 | 1 | 1 | 1 | 1 | 1 |
| | | F-measure | 1 | 0.92 | 1 | 1 | 1 | 0.92 | 0.92 |
| Test 2 | Test data | Precision | 0.83 | 0.57 | 0.55 | 0.71 | 0.6 | 0.6 | 0.62 |
| | | Recall | 0.91 | 0.73 | 0.55 | 0.45 | 0.82 | 0.82 | 0.73 |
| | | F-measure | 0.87 | 0.64 | 0.55 | 0.56 | 0.69 | 0.69 | 0.67 |
| | Training data | Precision | 1 | 0.77 | 0.84 | 1 | 0.74 | 0.74 | 0.77 |
| | | Recall | 1 | 0.66 | 0.77 | 1 | 0.66 | 0.66 | 0.66 |
| | | F-measure | 1 | 0.71 | 0.81 | 1 | 0.7 | 0.7 | 0.71 |

| | | | | | | | | | |
|---|---|---|---|---|---|---|---|---|---|
| *Rf, random forest; SVMlinear, linear kernel support vector machine; SVMrbf, rbf kernel support vector machine; Nnet, neural network; GLM, generalized linear model; rLDA, regularized linear discriminant analysis; rLogistic, regularized logistic regression | | | | | | | | | |

### Example 4 Preparation and verification of discriminant model - 3

### 1) Data used

Data (read count values) on the expression level of SSL-derived RNA from the test subjects was normalized by use of an approach called DESeq2, as in RNA expression analysis -2 in Example 1. However, a sample in which 4161 or more genes were not detected was excluded, and only genes which produced expression level data without missing values in 90% or more sample test subjects in the expression level data on the test subjects in all the samples after exclusion were used in analysis given below. In the analysis, normalized count values obtained by use of an approach called DESeq2 were used.

### 2) Data set partitioning

In the RNA profile data set obtained from the test subjects of Test 1, RNA profile data from a total of 15 subjects (9 healthy subjects and 6 PD) was used as training data for PD prediction models, and RNA profile data from a total of 5 subjects (the remaining 4 healthy subjects and 1 PD) was used as test data for use in the evaluation of model precision. In the RNA profile data set obtained from the test subjects of Test 2, RNA profile data from a total of 72 subjects (37 healthy subjects and 35 PD) was used as training data for PD prediction models, and RNA profile data from a total of 24 subjects (the remaining 13 healthy subjects and 11 PD) was used as test data for use in the evaluation of model precision.

### 3) Selection of feature gene

19 RNAs whose expression was increased or decreased in Test 1 in the PD patients compared with the healthy subjects (genes shown in Table 6-1) or 30 RNAs whose expression was increased or decreased in Test 2 in the PD patients compared with the healthy subjects (genes shown in Table 6-2) in RNA expression analysis - 2 in Example 1 were selected as feature genes. Their expression level data was converted to principal components by principal component analysis. Then, the first to fourth principal components were used as explanatory variables.

### 4) Model construction

Prediction model construction was carried out by using a value of each principal component obtained from expression level data (logarithmic values to base 2 of normalized count values plus 1) on the feature genes selected as training data from SSL-derived RNA as an explanatory variable, and the healthy subjects (HL) and PD as objective variables. The prediction models were learned by 10-fold cross validation by using 7 algorithms random forest, linear kernel support vector machine (SVM linear), rbf kernel support vector machine (SVM rbf), neural network, generalized linear model, regularized linear discriminant analysis, and regularized logistic regression for each item to be predicted. As for each algorithm, the value of each principal component obtained from the feature gene expression levels (logarithmic values to base 2 of normalized count values plus 1) of the test data was input to the models thus learned to calculate a target predictive value for each prediction item. Recall, precision, and an F value which is a harmonic mean thereof are calculated from a predictive value and an actually measured value, and a model having the largest F value was selected as the optimum prediction model.

### 5) Results

Tables 10 and 11 show the algorithm used, the recall, the precision, and the F value of each item to be predicted.

The F value of the model obtained by using 19 RNAs whose relation to Parkinson's disease had not been reported so far among RNAs whose expression was increased or decreased in results of the likelihood ratio test after normalization by DESeq2 in Test 1 was 1, indicating that PD was predictable with this model. The F value of the model obtained by using 30 RNAs whose relation to Parkinson's disease had not been reported so far among RNAs whose expression was increased or decreased in results of the likelihood ratio test after normalization by DESeq2 in Test 2 was 0.87, indicating that PD was predictable with this model.

**[Table 10]**

| | | | The number of RNA: 19 | | | | | | |
|---|---|---|---|---|---|---|---|---|---|
| | | | Rf | SMVlinear | SVMrbf | Nnet | GLM | rLDA | rLogistic |
| Test 1 | Test data | Precision | 1 | 1 | 1 | 1 | 1 | 1 | 1 |
| | | Recall | 1 | 1 | 1 | 1 | 1 | 1 | 1 |
| | | F-measure | 1 | 1 | 1 | 1 | 1 | 1 | 1 |
| | Training data | Precision | 1 | 1 | 1 | 1 | 1 | 1 | 1 |
| | | Recall | 1 | 1 | 1 | 1 | 1 | 1 | 0.83 |
| | | F-measure | 1 | 1 | 1 | 1 | 1 | 1 | 0.91 |

| | | | | | | | | | |
|---|---|---|---|---|---|---|---|---|---|
| *Rf, random forest; SVMlinear, linear kernel support vector machine; SVMrbf, rbf kernel support vector machine; Nnet, neural network; GLM, generalized linear model; rLDA, regularized linear discriminant analysis; rLogistic, regularized logistic regression | | | | | | | | | |

**[Table 11]**

| | | | The number of RNA: 30 | | | | | | |
|---|---|---|---|---|---|---|---|---|---|
| | | | Rf | SMVlinear | SVMrbf | Nnet | GLM | rLDA | rLogistic |
| Test 2 | Test data | Precision | 0.83 | 0.82 | 0.77 | 0.82 | 0.83 | 0.82 | 0.82 |
| | | Recall | 0.91 | 0.82 | 0.91 | 0.82 | 0.91 | 0.82 | 0.82 |
| | | F-measure | 0.87 | 0.82 | 0.83 | 0.82 | 0.87 | 0.82 | 0.82 |
| | Training data | Precision | 1 | 0.81 | 0.82 | 0.81 | 0.83 | 0.83 | 0.81 |
| | | Recall | 1 | 0.86 | 0.89 | 0.83 | 0.83 | 0.86 | 0.86 |
| | | F-measure | 1 | 0.83 | 0.85 | 0.82 | 0.83 | 0.85 | 0.83 |

| | | | | | | | | | |
|---|---|---|---|---|---|---|---|---|---|
| *Rf, random forest; SVMlinear, linear kernel support vector machine; SVMrbf, rbf kernel support vector machine; Nnet, neural network; GLM, generalized linear model; rLDA, regularized linear discriminant analysis; rLogistic, regularized logistic regression | | | | | | | | | |

### Example 5 Preparation and verification of discriminant model - 4

### 1) Data used

In the data (read count values) on the expression level of SSL-derived RNA from the test subjects, data with a read count of less than 10 was treated as missing values, as in RNA expression analysis - 1 in Example 1. After conversion to RPM values which normalized the read count values for difference in the total number of reads among samples, the missing values were compensated for by use of an approach called singular value decomposition (SVD) imputation. However, only genes which produced expression level data without missing values in 80% or more samples in all the samples were used in analysis given below. In the construction of machine learning models, converted RPM values, logarithmic values of RPM value to base 2 (Log₂RPM values) were used in order to approximate the RPM values, which followed negative binominal distribution, to normal distribution.

### 2) Data set partitioning

In the RNA profile data set obtained from the test subjects of Test 1, RNA profile data from a total of 20 subjects (10 healthy subjects and 10 PD) was used as training data for PD prediction models, and RNA profile data from the remaining 10 subjects was used as test data for use in the evaluation of model precision. In the RNA profile data set obtained from the test subjects of Test 2, RNA profile data from a total of 80 subjects (40 healthy subjects and 40 PD) was used as training data for PD prediction models, and RNA profile data from the remaining 20 subjects was used as test data for use in the evaluation of model precision.

### 3) Selection of feature gene

21 RNAs whose expression was increased or decreased in Test 1 in the PD patients compared with the healthy subjects (genes shown in Table 3-1) or 92 RNAs whose expression was increased or decreased in Test 2 in the PD patients compared with the healthy subjects (genes shown in Tables 3-2 to 3-4) in RNA expression analysis - 1 in Example 1 were selected as feature genes. Their expression level data was converted to principal components by principal component analysis. Then, the first to fourth principal components were used as explanatory variables.

### 4) Model construction

Prediction model construction was carried out by using a value of each principal component obtained from expression level data (Log₂RPM values) on the feature genes selected as training data from SSL-derived RNA as an explanatory variable, and the healthy subjects (HL) and PD as objective variables. The prediction models were learned by 10-fold cross validation by using 7 algorithms random forest, linear kernel support vector machine (SVM linear), rbf kernel support vector machine (SVM rbf), neural network, generalized linear model, regularized linear discriminant analysis, and regularized logistic regression for each item to be predicted. As for each algorithm, the value of each principal component obtained from the feature gene expression levels (Log2RPM value) of the test data was input to the models thus learned to calculate a target predictive value for each prediction item. Recall, precision, and an F value which is a harmonic mean thereof are calculated from a predictive value and an actually measured value, and a model having the largest F value was selected as the optimum prediction model.

### 5) Results

Tables 12 and 13 show the algorithm used, the recall, the precision, and the F value of each item to be predicted.

The F value of the model obtained by using 21 RNAs whose relation to Parkinson's disease had not been reported so far among RNAs whose expression was increased or decreased in results of the test after normalization by Log2RPM in Test 1 was 0.91, indicating that PD was predictable with this model. The F value of the model obtained by using 92 RNAs whose relation to Parkinson's disease had not been reported so far among RNAs whose expression was increased or decreased in results of the test after normalization by Log2RPM in Test 2 was 0.9, indicating that PD was predictable with this model.

**[Table 12]**

| | | | The number of RNA: 21 | | | | | | |
|---|---|---|---|---|---|---|---|---|---|
| | | | Rf | SMVlinear | SVMrbf | Nnet | GLM | rLDA | rLogistic |
| Test 1 | Test data | Precision | 0.83 | 0.75 | 0.71 | 0.8 | 0.75 | 0.8 | 0.8 |
| | | Recall | 1 | 0.6 | 1 | 0.8 | 0.6 | 0.8 | 0.8 |
| | | F-measure | 0.91 | 0.67 | 0.83 | 0.8 | 0.67 | 0.8 | 0.8 |
| | Training data | Precision | 1 | 1 | 1 | 1 | 1 | 1 | 1 |
| | | Recall | 1 | 0.9 | 1 | 1 | 1 | 1 | 1 |
| | | F-measure | 1 | 0.95 | 1 | 1 | 1 | 1 | 1 |

| | | | | | | | | | |
|---|---|---|---|---|---|---|---|---|---|
| *Rf, random forest; SVMlinear, linear kernel support vector machine; SVMrbf, rbf kernel support vector machine; Nnet, neural network; GLM, generalized linear model; rLDA, regularized linear discriminant analysis; rLogistic, regularized logistic regression | | | | | | | | | |

**[Table 13]**

| | | | The number of RNA: 92 | | | | | | |
|---|---|---|---|---|---|---|---|---|---|
| | | | Rf | SMVIinear | SVMrbf | Nnet | GLM | rLDA | rLogistic |
| Test 2 | Test data | Precision | 0.9 | 0.88 | 0.88 | 0.88 | 0.88 | 0.88 | 0.89 |
| | | Recall | 0.9 | 0.7 | 0.7 | 0.7 | 0.7 | 0.7 | 0.8 |
| | | F-measure | 0.9 | 0.78 | 0.78 | 0.78 | 0.78 | 0.78 | 0.84 |
| | Training data | Precision | 1 | 0.83 | 0.92 | 0.92 | 0.87 | 0.83 | 0.89 |
| | | Recall | 1 | 0.83 | 0.88 | 0.85 | 0.85 | 0.88 | 0.83 |
| | | F-measure | 1 | 0.83 | 0.9 | 0.88 | 0.86 | 0.85 | 0.86 |

| | | | | | | | | | |
|---|---|---|---|---|---|---|---|---|---|
| *Rf, random forest; SVMlinear, linear kernel support vector machine; SVMrbf, rbf kernel support vector machine; Nnet, neural network; GLM, generalized linear model; rLDA, regularized linear discriminant analysis; rLogistic, regularized logistic regression | | | | | | | | | |

## Claims

1. A method for detecting Parkinson's disease in a test subject, comprising a step of measuring an expression level of at least one gene selected from the group of 4 genes consisting of SNORA16A, SNORA24, SNORA50 and REXO1L2P or an expression product thereof in a biological sample collected from the test subject.

2. The method for detecting Parkinson's disease according to claim 1, wherein the method at least comprises measuring an expression level of SNORA24 gene or an expression product thereof.

3. The method according to claim 1 or 2, wherein the expression level of the gene or the expression product thereof is measured as an expression level of mRNA.

4. The method according to any one of claims 1 to 3, wherein the gene or the expression product thereof is RNA contained in skin surface lipids of the test subject.

5. The method according to any one of claims 1 to 4, wherein the presence or absence of Parkinson's disease is evaluated by comparing the measurement value of the expression level with a reference value of the gene or the expression product thereof.

6. The method according to any one of claims 1 to 4, wherein the presence or absence of Parkinson's disease in the test subject is evaluated by the following steps:
preparing a discriminant which discriminates between the Parkinson's disease patient and a healthy person by using measurement values of an expression level of the gene or the expression product thereof derived from a Parkinson's disease patient and an expression level of the gene or the expression product thereof derived from a healthy subject as teacher samples; substituting the measurement value of the expression level of the gene or the expression product thereof obtained from the biological sample collected from the test subject into the discriminant; and comparing the obtained results with a reference value.

7. The method according to claim 6, wherein expression levels of all the genes of the group of 4 genes or expression products thereof are measured.

8. The method according to claim 6 or 7, wherein expression levels of the at least one gene selected from the group of 4 genes as well as at least one gene selected from the following group of 29 genes or expression products thereof are measured:
ANKRD12, C10orf116, CCL3, CCNI, CD83, CNFN, CNN2, CSF2RB, CXCR4, EGR2, EMP1, ITGAX, KCNQ1OT1, LCE3D, LITAF, NDUFA4L2, NDUFS5, POLR2L, RHOA, RNASEK, RPL7A, RPS26, SERINC1, SERP1, SERPINB4, SLC25A3, SNRPG, SRRM2, and UQCRH.

9. The method according to claim 8, wherein expression levels of the at least one gene selected from the group of 4 genes as well as at least one gene selected from the following group of 10 genes or expression products thereof are measured:
CCL3, CCNI, CXCR4, EGR2, EMP1, POLR2L, RHOA, RNASEK, SERINC1, and SERPINB4.

10. The method according to claim 6 or 7, wherein expression levels of the at least one gene selected from the group of 4 genes as well as at least one gene selected from the groups of 1,005 genes shown in the following Tables 1-1 to 1-27 and 725 genes shown in the following Tables 4-1 to 4-20 except for the 4 genes, or expression products thereof are measured.
| Table 1-1 | Table 1-2 | Table 1-3 | Table 1-4 | Table 1-5 |
|---|---|---|---|---|
| ADRM1 | KCTD11 | SLC25A3 | ACSL1 | RGS2 |
| ARF5 | KIAA0930 | SNF8 | ACSL4 | RHOA |
| ARHGEF5 | KLHDC3 | SNORA16A | ANKRD12 | RNASEK |
| BCKDK | LCE3D | SNORA24 | ARPC1B | RPL10 |
| C10orf116 | LOC100093631 | SNORA43 | BRD4 | RPL15 |
| C11orf10 | LOC100506888 | SNORA50 | BTG1 | RPL19 |
| C14orf2 | LOC349196 | SNORA8 | CALM2 | RPL21 |
| CEBPA | LOC401321 | SNRPG | CCL3 | RPL26 |
| CHAC1 | LPIN1 | SPINT1 | CCNI | RPL28 |
| CHCHD2 | MAP2K2 | SQRDL | CD83 | RPL3 |
| CMIP | METRNL | SRXN1 | CDC42 | RPL30 |
| CNFN | MGLL | STAT6 | CHMP4B | RPL35 |
| COPE | NDUFA13 | STIP1 | CNBP | RPL5 |
| COPS8 | NDUFA4L2 | TALDO1 | CNN2 | RPL6 |
| COX8A | NDUFB11 | TCEB3CL | CSF2RB | RPS20 |
| CSDA | NDUFS5 | TCIRG1 | CXCR4 | RPS25 |
| CTBP2 | NR4A3 | TEX264 | DDX5 | S100A11 |
| CTDNEP1 | OAZ1 | TMEM183A | EEF1A1 | SCARNA9 |
| CYFIP1 | OR4F3 | TRMT112 | EEF1B2 | SERINC1 |
| DAD1 | PKP3 | TTC9 | EGR2 | SERP1 |
| DNASE1L2 | POLD4 | TYMP | EIF1 | SNORA53 |
| DUX4L4 | POLR2L | UQCRB | EPS15 | SRRM2 |
| EDF1 | PPA1 | UQCRC1 | GNG10 | STK24 |
| EIF3E | PQLC1 | UQCRH | GRINA | TMEM127 |
| EIF4G1 | PRELID1 | UQCRQ | H3F3A | TNIP1 |
| EMP1 | PSMB7 | USP17L5 | HIF1A | TPM4 |
| FAM129B | PSMC1 | USP17L6P | HNRNPA2B1 | TPT1 |
| FAM83G | PSMD4 | USP38 | HNRNPU | |
| FEM1B | PURB | VEGFA | IFNGR2 | |
| G6PD | RAP2B | ZNF33A | IL1RN | |
| GPBP1L1 | RASAL1 | ZNF410 | ITGAX | |
| GPR157 | REXO1L2P | | LITAF | |
| GPX3 | RPL7A | | LYN | |
| HECA | RPS26 | | NEAT1 | |
| HIPK1 | RRAD | | PABPC1 | |
| HIST2H2BE | RRAGA | | PAIP2 | |
| HLA.DQB2 | SEC61A1 | | PGK1 | |
| HMGCS1 | SERPINB4 | | PLXNC1 | |
| HSPA1A | SFXN3 | | RABGEF1 | |
| IQSEC1 | | | RAP1A | |
| KCNQ1OT1 | | | REL | |
| Table 1-6 | Table 1-7 | Table 1-8 | Table 1-9 | Table 1-10 |
|---|---|---|---|---|
| A2ML1 | C22orf32 | CRELD2 | EIF3K | GTF2A2 |
| ABRACL | C2orf49 | CRIPT | EIF4EBP1 | GTF2E2 |
| ACBD3 | C5orf43 | CRNN | ELOVL7 | GTF2H5 |
| ACOT13 | C5orf46 | CST6 | EMP1 | GTF3C5 |
| ACSS3 | C8orf33 | CSTA | ENDOD1 | GTF3C6 |
| ADAP2 | CACYBP | CUL4A | EPHB6 | H1FX |
| ADPRHL2 | CALM1 | CUTA | EPHX3 | HADH |
| ADSL | CARHSP1 | CYB5A | ERBB3 | HBEGF |
| ADSS | CASK | CYB5B | ERO1L | HDAC1 |
| AHCY | CASP14 | DANCR | EXOC4 | HDDC2 |
| AIF1L | CAST | DCAF12 | EXOC5 | HEATR5A |
| AIM1L | CCDC6 | DDRGK1 | EXOC6B | HEXB |
| AK1 | CCNE1 | DDT | F13A1 | HIBADH |
| AK4 | CCT2 | DEGS1 | FABP4 | HIBCH |
| ALDH1A3 | CCT3 | DENND2C | FABP9 | HIST1H1E |
| ALDOC | CCT4 | DHPS | FAM108B1 | HIST1H2AE |
| AMBRA1 | CCT8 | DHX29 | FAM135A | HIST1H2AG |
| ANP32B | CDC16 | DHX32 | FAM210B | HIST1H2AI |
| ANP32E | CDSN | DHX40 | FAM25B | HIST1H2AM |
| ANXA1 | CGA | DNAJA1 | FAM3C | HIST1H2BN |
| AP4S1 | CGNL1 | DNAJA4 | FAM45A | HIST1H3B |
| ARFGAP2 | CHI3L2 | DNAJC13 | FAM46B | HIST1H3I |
| ARHGAP29 | CHIC2 | DNAJC15 | FBXO45 | HIST1H4B |
| ARL1 | CHMP4A | DNAJC21 | FCHSD1 | HIST1H4E |
| ASS1 | CIZ1 | DNAJC7 | FIG4 | HIST1H4F |
| ATP5B | CKB | DNAJC9 | FKBP1A | HIST1H4H |
| ATP5E | CLIC3 | DOCK6 | FKBP3 | HMOX2 |
| ATP5G1 | CLIP1 | DOCK9 | FLG | HNRNPA0 |
| ATP5I | CNDP2 | DPH1 | FOXQ1 | HOMER1 |
| ATP5O | CNFN | DPY30 | FRMD6 | HOOK1 |
| ATPIF1 | CNIH4 | DRG1 | FTSJ1 | HPGD |
| BAG3 | CNN3 | DSG1 | FUNDC2 | HRSP12 |
| BCAS1 | CNNM4 | DUSP11 | FYN | HSD17B10 |
| BCAS2 | COA1 | DYM | GBAS | HSP90AA1 |
| BCL2L13 | COA3 | DYNC1LI1 | GGCT | HSPD1 |
| BCL7C | COMT | DYNLL1 | GHITM | HYPK |
| BMP2 | COX4I1 | DYNLRB1 | GLOD4 | IDE |
| C10orf116 | COX5B | ECHS1 | GNL3 | IDH3A |
| C11orf31 | CPEB2 | EFN B2 | GPSM2 | IFI27 |
| C1orf52 | CPNE3 | EIF1AX | GRHL3 | IL32 |
| C1orf63 | CRABP2 | EIF2S2 | GRPEL1 | IL36A |
| Table 1-11 | Table 1-12 | Table 1-13 | Table 1-14 | Table 1-15 |
|---|---|---|---|---|
| ILKAP | LCE3E | MTMR12 | PEPD | RAB38 |
| IPO5 | LCMT1 | MUT | PFDN2 | RABIF |
| IQCG | LCN2 | MYO10 | PFDN5 | RANBP1 |
| ITGB1BP1 | LEMD3 | MZT2A | PFDN6 | RANBP10 |
| ITPA | LEPROTL1 | NCBP2 | PHAX | RARRES1 |
| ITPRIPL2 | LINC00675 | NCK1 | PHF13 | RBM10 |
| IVL | LLPH | NDRG2 | PHPT1 | RBMS2 |
| KANK1 | LMBR1 | NDUFA12 | PICK1 | REXO1L2P |
| KCNQ1OT1 | LNX1 | NDUFA2 | PINLYP | RHCG |
| KIAA0240 | LOC100505738 | NDUFA4L2 | PITRM1 | RMRP |
| KIAA1143 | LOC550643 | NDUFB1 | PKP1 | RNASE7 |
| KLF5 | LOC646862 | NDUFS5 | PLCD1 | RNF121 |
| KLK13 | LRBA | NDUFS6 | PLD2 | RNF20 |
| KLK7 | LRRC15 | NEDD4L | PLS3 | ROMO1 |
| KLK8 | LSM10 | NFU1 | POF1B | RPA1 |
| KRT14 | LSM2 | NHP2 | POLR2D | RPIA |
| KRT16 | LSM7 | NIN | POLR2G | RPL10A |
| KRT25 | LTF | NIPAL3 | POLR2L | RPL18 |
| KRT26 | LY6D | NIPAL4 | POLR2M | RPL21 |
| KRT27 | LYNX1 | NOSIP | PPFIBP2 | RPL26L1 |
| KRT5 | MAFA | NRIP3 | PPID | RPL30 |
| KRT6A | MAL | NSMCE1 | PPIL4 | RPL32 |
| KRT6C | MALL | NUDC | PPL | RPL36 |
| KRT71 | MAOA | NUMA1 | PPP1R13B | RPL36A |
| KRT72 | MAP4K3 | NUP214 | PPP2R2A | RPL37A |
| KRT74 | MAP7 | NUPL1 | PPP5C | RPL38 |
| KRT78 | MCCC1 | OFD1 | PPWD1 | RPL7 |
| KRTAP1.5 | MCTS1 | OLA1 | PRDX3 | RPL7A |
| KRTAP12.1 | MICALCL | ORMDL3 | PRDX6 | RPLP0 |
| KRTAP12.2 | MNF1 | PABPN1 | PREP | RPLP1 |
| KRTAP19.1 | MPHOSPH6 | PADI1 | PRKRA | RPS12 |
| KRTAP3.1 | MPV17 | PADI3 | PROM2 | RPS15 |
| KRTAP3.3 | MRPL11 | PAK4 | PRPF40A | RPS15A |
| KRTAP5.3 | MRPL12 | PAPL | PRPF4B | RPS18 |
| KRTAP5.7 | MRPL24 | PCCB | PRR9 | RPS26 |
| KRTDAP | MRPL32 | PDCD5 | PRSS3 | RPS28 |
| KTN1 | MRPL47 | PDDC1 | PSMC2 | RPS29 |
| LCE2A | MRPS11 | PDE12 | PSORS1C2 | RPS3 |
| LCE2C | MRPS18B | PDHA1 | PTPN3 | RPS4X |
| LCE2D | MRPS24 | PDZD8 | PVRL4 | RPS5 |
| LCE3D | MT1X | PDZK1IP1 | QKI | RPS6 |
| Table 1-16 | Table 1-17 | Table 1-18 | Table 1-19 | Table 1-20 |
|---|---|---|---|---|
| RPS6KA2 | SMC3 | SNRPE | TRPT1 | ABTB1 |
| RPS6KB1 | SMEK2 | SNRPF | TSC2 | ADAM8 |
| RPTN | SMIM5 | SNRPG | TSPO | ADORA2A |
| S100A14 | SNHG1 | SOS1 | TSR1 | AGTRAP |
| S100A7 | SNHG16 | SPINK5 | TTPAL | AGXT2L2 |
| S100A7A | SNHG6 | SPINK7 | TUBB2A | AHCYL1 |
| S100A8 | SNHG9 | SPRED1 | TWF1 | ALPL |
| S100A9 | SNIP1 | SPRR1A | TXNDC17 | ANKRD12 |
| SBDS | SNORA10 | SPRR1B | TXNRD1 | ANKRD17 |
| SBF1 | SNORA14B | SPRR2D | UBE2L3 | ANKRD27 |
| SBSN | SNORA16A | SPRR2E | UBL3 | AP1G1 |
| SCARNA12 | SNORA21 | SPRR2F | UBL5 | APH1A |
| SCARNA16 | SNORA23 | SPRR3 | UCHL3 | ARF1 |
| SCARNA17 | SNORA24 | SPTLC1 | UGP2 | ARF5 |
| SCARNA6 | SNORA33 | SPTLC2 | UNC50 | ARHGAP30 |
| SCARNA7 | SNORA34 | SRD5A1 | UQCR10 | ARHGEF2 |
| SCGB2A2 | SNORA38 | SRSF10 | UQCRH | ARID3A |
| SCNN1B | SNORA49 | SSBP1 | UTP6 | ARL5B |
| SCNN1G | SNORA50 | SSBP3 | VASN | ARPC2 |
| SDR16C5 | SNORA52 | STAP2 | VPS4A | ATG2A |
| SDR9C7 | SNORA57 | SUMF2 | VSIG8 | ATHL1 |
| SEC23A | SNORA6 | SYBU | WDR60 | ATP13A3 |
| SERPINA9 | SNORA62 | TADA2B | WDR61 | ATP6V0C |
| SERPINB4 | SNORA63 | TCEA1 | WFDC12 | ATP6V0D1 |
| SERPINB5 | SNORA65 | TCHH | WFDC5 | AURKAIP1 |
| SERPINB7 | SNORA67 | TCHHL1 | WIBG | BAK1 |
| SF3B14 | SNORA68 | TFAP2C | WWTR1 | BAP1 |
| SF3B3 | SNORA71A | TFIP11 | XPOT | BMP2K |
| SH3GL3 | SNORA71B | TGM3 | YTHDF1 | BRD2 |
| SLC10A6 | SNORA71C | THOC7 | YTHDF2 | BSDC1 |
| SLC25A20 | SNORA71D | TIA1 | ZFAND2A | C15orf38 |
| SLC25A3 | SNORA74A | TM4SF1 | ZNF259 | C17orf107 |
| SLC25A5 | SNORA74B | TM4SF19 | | C22orf13 |
| SLC26A9 | SNORA7B | TMEM179B | | CAMK1D |
| SLC5A1 | SNORA84 | TMEM45B | | CANT1 |
| SLC6A14 | SNORA9 | TMEM60 | | CASP9 |
| SLC6A8 | SNORD15A | TPRG1 | | CCDC28A |
| SLFN5 | SNORD15B | TRAF4 | | CCDC9 |
| SLMO2 | SNORD17 | TRAK2 | | CCL3 |
| SLURP1 | SNORD94 | TRAPPC2L | | CCNI |
| SMAD7 | SNRPD1 | TRMT6 | | CCRL2 |
| Table 1-21 | Table 1-22 | Table 1-23 | Table 1-24 | Table 1-25 |
|---|---|---|---|---|
| CD63 | FAM53C | ISG20L2 | NAB1 | RAB20 |
| CD83 | FBXO11 | ITGA5 | NAGK | RAB5C |
| CD97 | FCGRT | ITGAM | NCF1B | RALGDS |
| CDC42SE1 | FGR | ITGAX | NCF1C | RAP2C |
| CDKN1A | FLNA | JARID2 | NCOA1 | RBCK1 |
| CFL1 | FNIP1 | JUNB | NFKB2 | RBM39 |
| CHD2 | FOSB | KAT5 | NFKBIB | RBM4 |
| CIC | FOSL2 | KDM6B | NFKBID | RELA |
| CNN2 | FOXN3 | KIAA0232 | NINJ1 | RGS19 |
| CRLF3 | FOXO4 | KIAA0513 | NLRC5 | RHBDD2 |
| CSF1 | FURIN | KLF2 | NOTCH2NL | RHEB |
| CSF2RB | FZR1 | KLF6 | NRIP1 | RHOA |
| CSRNP1 | GABARAPL1 | KLHL2 | NUMB | RHOB |
| CTBP2 | GADD45B | LATS2 | OGFR | RILPL2 |
| CTDSP2 | GAPVD1 | LILRB2 | OS9 | RNASEK |
| CXCR4 | GATAD2A | LIMS1 | PAN3 | RNF13 |
| CYTH1 | GGA1 | LITAF | PATL1 | RNF41 |
| DBNL | GLA | LOC283070 | PCBP1 | RTN4 |
| DCAF11 | GMIP | LPAR2 | PDPK1 | RXRA |
| DENND5A | GNB1 | LPCAT1 | PER1 | RYBP |
| DESI1 | GNB2 | LSP1 | PFKFB3 | SBNO2 |
| DGAT1 | GPR108 | LTBR | PHF1 | SCYL1 |
| DNM2 | GPX1 | MAF1 | PIK3AP1 | SDE2 |
| DOT1L | GRAMD1A | MAN2A1 | PIK3R5 | SEC22B |
| DUSP1 | GRK6 | MAP4K4 | PIM3 | SEMA6B |
| DUSP2 | GRN | MAP7D1 | PITPNA | SERINC1 |
| DUSP3 | GTPBP1 | MAPKAPK2 | PLAU | SERP1 |
| ECD | HEXIM1 | MECP2 | PLEKHB2 | SF3B2 |
| EFH D2 | HIPK3 | MEF2D | PLEKHM3 | SH3BP5 |
| EFR3A | HLA.A | METRNL | PLIN5 | SHISA5 |
| EGR2 | HLX | MGEA5 | PPP1R15A | SIPA1 |
| EGR3 | HSPA4 | MIDN | PPP1R18 | SIRPA |
| EIF2C4 | IDS | MKNK2 | PPP2R5C | SLC11A1 |
| EIF4EBP2 | IER3 | MLF2 | PPP4R1 | SLC15A3 |
| ELF1 | IMPDH1 | MLLT6 | PRR14 | SLC16A3 |
| EMP3 | INO80D | MMP25 | PRR24 | SLC25A6 |
| EPS15L1 | INPP5K | MTHFS | PRRC2C | SLC3A2 |
| FAM100B | IQSEC1 | MTMR14 | PTGER4 | SLC43A2 |
| FAM193B | IRAK2 | MYADM | PTK2B | SLC44A2 |
| FAM210A | IRS2 | MYO9B | PTTG1IP | SLC6A6 |
| FAM32A | ISCU | NAA50 | RAB11FIP1 | SLC9A8 |
| Table 1-26 | Table 1-27 | | | |
|---|---|---|---|---|
| SLED1 | XPO6 | | | |
| SMG1P1 | YPEL5 | | | |
| SPHK1 | ZC3H12A | | | |
| SQSTM 1 | ZFP36 | | | |
| SREBF2 | ZMIZ1 | | | |
| SRRM2 | ZNFX1 | | | |
| SRXN1 | ZZEF1 | | | |
| STK40 | | | | |
| STX11 | | | | |
| STX3 | | | | |
| STX6 | | | | |
| STXBP2 | | | | |
| SUPT6H | | | | |
| TAF10 | | | | |
| TANK | | | | |
| TCF25 | | | | |
| TCIRG1 | | | | |
| TM9SF4 | | | | |
| TMBIM6 | | | | |
| TMEM123 | | | | |
| TMEM167B | | | | |
| TMEM183A | | | | |
| TMEM66 | | | | |
| TMX4 | | | | |
| TNFAIP2 | | | | |
| TNFAIP3 | | | | |
| TNFRSF14 | | | | |
| TOM1 | | | | |
| TP53INP2 | | | | |
| TRAPPC5 | | | | |
| TSPAN13 | | | | |
| TTYH3 | | | | |
| UBAP2L | | | | |
| UBE2D3 | | | | |
| UBR4 | | | | |
| UCP2 | | | | |
| UPF1 | | | | |
| USB1 | | | | |
| USF2 | | | | |
| WBP2 | | | | |
| WDR82 | | | | |
| Table 4-1 | Table 4-2 | Table 4-3 | Table 4-4 | Table 4-5 |
|---|---|---|---|---|
| ACOT2 | PQLC1 | AATF | COPB2 | HBP1 |
| ACOX3 | PRELID1 | ADRBK2 | CPA4 | HELZ |
| ACTG1 | PRKAA1 | AHSA1 | CPM | HIF1A |
| AKT1S1 | PSMA7 | AIDA | CS | HINT1 |
| AMZ2 | PSMD4 | ANKRD12 | CSF1 | HINT3 |
| ANXA1 | PTGS2 | ANXA3 | CXCR4 | HIST1H1E |
| ANXA2 | RASAL1 | AP3B1 | CYBB | HMGN1 |
| AQP3 | RNASET2 | APH1A | DCUN1D1 | HNRNPA2B1 |
| AREG | RNF217 | API5 | DDX21 | HNRNPK |
| ARF5 | RPL13 | APLP2 | DDX5 | HNRNPU |
| ATP5E | S100A8 | ARID4B | DICER1 | IARS2 |
| BCKDK | SDC4 | ARPC1A | DLD | ICAM1 |
| BCR | SERPINB4 | ARPC3 | DNAJC15 | IDE |
| BSG | SLC25A3 | ATG12 | DNAJC3 | IER3IP1 |
| C14orf2 | SLPI | ATP2A2 | DR1 | JAK1 |
| CEBPA | SNORA24 | ATP5J2 | EEF1B2 | JMY |
| CHCHD2 | SNORA50 | ATP6AP2 | EGR2 | KAT2B |
| CHMP5 | SNORA57 | ATP6V0C | EIF2S2 | KIAA1551 |
| COPE | SNORA8 | ATP6V1G1 | EIF5A | KIF16B |
| CORO1A | SNORA9 | BAG1 | ELF1 | KLF10 |
| CSDA | SOCS3 | BHLHE40 | EML4 | KLF3 |
| DYNLT1 | TIMP1 | BTF3 | EP300 | LGALSL |
| EIF4A3 | TMCC3 | BTG1 | EPS15 | MARCH7 |
| EMP1 | TRMT44 | BUD31 | ERBB2IP | MBD2 |
| FLII | TSPO | C14orf178 | ETF1 | MBD6 |
| GPR157 | TUBA1C | CAPZA1 | ETV6 | MDM2 |
| GPX3 | UQCRB | CAPZA2 | EVL | MED13L |
| HSPA1A | UQCRC1 | CBFB | EZR | MED19 |
| KRT16 | UQCRFS1 | CCDC93 | FAM100A | MRPL15 |
| LOC100216546 | VEGFA | CCL3 | FAM126A | NAPA |
| LOC100288069 | ZFP36L2 | CCNI | FAM160A1 | NR4A2 |
| MESDC1 | ZNF410 | CDC42 | FNTA | NRBF2 |
| MIEN1 | ZSWIM6 | CHMP2A | FUBP1 | NRBP1 |
| MKNK2 | | CHMP2B | FYTTD1 | NSFP1 |
| MNDA | | CHMP3 | G3BP2 | OGFRL1 |
| NEDD8 | | CIRBP | GABARAP | P4HB |
| OTUD1 | | CLIC4 | GABARAPL1 | PAIP2 |
| PIR | | CLIP1 | GLTP | PDXK |
| PNISR | | CLK1 | GLTSCR2 | PGK1 |
| POLR2J3 | | CLNS1A | GOLGA8B | PGRMC2 |
| | | CNBP | GRB2 | PHF20L1 |
| Table 4-6 | Table 4-7 | Table 4-8 | Table 4-9 | Table 4-10 |
|---|---|---|---|---|
| PHF5A | SERTAD2 | YWHAQ | ALOX12B | KRT10 |
| PIKFYVE | SET | ZCRB1 | ANXA1 | KRT14 |
| PLA2G7 | SH3BGRL3 | ZMAT2 | AQP3 | KRT16 |
| POLR2A | SLMO2 | ZNF148 | ATP12A | KRT25 |
| PTPN12 | SMS | | ATP5B | KRT27 |
| QARS | SNAP29 | | ATP5I | KRT5 |
| RAB14 | SNORA53 | | ATP5O | KRT6A |
| RAB9A | SNX13 | | BAG3 | KRT71 |
| RABGEF1 | SNX9 | | C6orf132 | KRT72 |
| RAP1A | SREK1IP1 | | CALM1 | KRT74 |
| RAP1B | SRSF5 | | CASP14 | KRTAP5-3 |
| RHOA | SSR2 | | CAST | KRTDAP |
| RIOK3 | SSU72 | | CDSN | LCE2C |
| RMND5A | STK24 | | CLIC3 | LCE2D |
| RNASEK | STT3B | | CNFN | LCE3D |
| RPL10 | TAF10 | | COX4I1 | LCE3E |
| RPL13AP20 | TAOK1 | | COX8A | LCN2 |
| RPL15 | TERF2IP | | CRABP2 | LNX1 |
| RPL19 | TLK2 | | CST6 | LRRC15 |
| RPL24 | TMA7 | | CTSC | NDRG2 |
| RPL26 | TMEM106B | | DNAJA1 | NDUFA4L2 |
| RPL28 | TMEM127 | | DYNLL1 | NDUFB11 |
| RPL36AL | TMEM167B | | EEF1B2 | NDUFB2 |
| RPL5 | TNFSF13B | | EIF1AX | NDUFB8 |
| RPL6 | TPGS2 | | EIF3K | NDUFS5 |
| RPS20 | TRAM1 | | ELF3 | NSFL1C |
| RPS25 | TRIP12 | | EMP1 | NUMA1 |
| RPS9 | TRPM7 | | EPHX3 | PDZK1IP1 |
| S100A10 | TSG101 | | FABP9 | PINLYP |
| S100A11 | TXNL1 | | GNB2L1 | PKP1 |
| SCAF11 | UBE2A | | GRHL3 | PNP |
| SCYL2 | UBE2B | | HIST1H4E | POLR2L |
| SDF4 | UBE2H | | HIST1H4H | PPL |
| SEC11C | USMG5 | | HMGCS1 | PPP2R2A |
| SEC24A | USP22 | | HMOX2 | PRR9 |
| SEPT11 | USP53 | | HSP90AA1 | PRSS3 |
| SEPT2 | USP6NL | | HSPB1 | PSMC2 |
| SERINC1 | USP7 | | IVL | RBBP4 |
| SERINC3 | WIPF1 | | KLF5 | RMRP |
| SERPINA12 | WTAP | | KLK13 | ROMO1 |
| SERPINB9 | XBP1 | | KLK7 | RPL10A |
| Table 4-11 | Table 4-12 | Table 4-13 | Table 4-14 | Table 4-15 |
|---|---|---|---|---|
| RPL11 | SBSN | A2M | CCNY | DUSP3 |
| RPL12 | SERPINB4 | AADACL3 | CCRL2 | DUSP4 |
| RPL13A | SERPINB5 | ABHD5 | CCSAP | ECE1 |
| RPL18 | SFN | ABTB1 | CD300A | EFH D2 |
| RPL21 | SLURP1 | ACSL5 | CD36 | EFR3A |
| RPL26 | SNORA16A | ADAM8 | CD63 | EGR2 |
| RPL27 | SNORA24 | ADORA2A | CD82 | EGR3 |
| RPL27A | SNORA52 | AGTRAP | CD83 | EHBP1L1 |
| RPL29 | SNORA63 | AKR7A2 | CD97 | EHD1 |
| RPL3 | SNORA68 | ALPL | CDC14A | EID3 |
| RPL30 | SNORA71A | AMPD2 | CDC37 | EIF1 |
| RPL32 | SNORD15B | ANKRD22 | CDC42EP3 | EIF4EBP2 |
| RPL35 | SPRR1A | AP5B1 | CDC42SE1 | EIF4EBP3 |
| RPL36 | SPRR1B | ARF1 | CDKN1A | ELL |
| RPL36A | SPRR2D | ARF5 | CEP76 | EMP3 |
| RPL37A | SPRR2E | ARHGAP1 | CHD2 | EPS15L1 |
| RPL38 | SPRR2F | ARHGAP30 | CHMP4B | FADS2 |
| RPL7 | TCHH | ARHGEF2 | CHP1 | FAM100B |
| RPL7A | TCHHL1 | ARID3A | CLMP | FAM193B |
| RPLP0 | TMOD3 | ARL5B | CNN2 | FAM213A |
| RPLP1 | TMPRSS11E | ARRB2 | COTL1 | FAM32A |
| RPLP2 | UBE2L3 | ASAH1 | CRKL | FAM46C |
| RPS10 | UBL3 | ATG2A | CSF2RB | FFAR2 |
| RPS12 | UQCR11 | ATHL1 | CSF3R | FGR |
| RPS15 | UQCRH | ATP6V0C | CSNK1G2 | FLNA |
| RPS15A | UXT | BASP1 | CSRNP1 | FMNL1 |
| RPS18 | WWC1 | BCKDK | CTSA | FNIP1 |
| RPS19 | WWTR1 | BCL2L1 | CTSD | FOSB |
| RPS21 | | BHLHE40 | CXCL16 | FOSL2 |
| RPS26 | | BRD4 | CXCR4 | FURIN |
| RPS28 | | C17orf107 | CYTH4 | GABARAPL1 |
| RPS3 | | C1orf43 | DBNL | GADD45B |
| RPS4X | | C22orf13 | DCAF11 | GAL |
| RPS5 | | C2CD2 | DDX60L | GAS7 |
| RPS6 | | C6orf106 | DENND5A | GDE1 |
| RPS8 | | CANT1 | DGAT2 | GPR108 |
| S100A14 | | CCDC86 | DHCR24 | GPR157 |
| S100A7 | | CCL3 | DIRC2 | GPSM3 |
| S100A7A | | CCL3L3 | DSCR3 | GRAMD1A |
| S100A9 | | CCL4 | DUSP1 | GRINA |
| SBDS | | CCNI | DUSP2 | GRK6 |
| Table 4-16 | Table 4-17 | Table 4-18 | Table 4-19 | Table 4-20 |
|---|---|---|---|---|
| GRN | MEF2D | PITPNA | SHISA5 | TOM1 |
| GTPBP1 | MEGF9 | PLAU | SHKBP1 | TPD52L2 |
| HDAC7 | MEPCE | PLEKHO2 | SIRPA | TRIB1 |
| HLA-A | METRNL | POU5F1P3 | SLC11A1 | TRIM25 |
| HPCAL1 | MGEA5 | PPP1CB | SLC15A3 | TRPC4AP |
| HS3ST6 | MKNK2 | PPP1R15A | SLC15A4 | UBAP2L |
| HSPA4 | MLF2 | PPP1R18 | SLC31A1 | UBE2D3 |
| IDS | MLLT6 | PPP4R1 | SLC3A2 | UBIAD1 |
| IER3 | MMP25 | PSMF1 | SLC41A1 | UBR4 |
| IMPDH1 | MSRB1 | PTGER4 | SLC43A2 | UCP2 |
| INPP5K | MTHFS | PTK2B | SLC43A3 | USF2 |
| IRAK2 | MTMR14 | PTPN6 | SLC45A4 | VOPP1 |
| IRF1 | MYO9B | PTTG1IP | SLC6A6 | WBP2 |
| ITGA5 | NAA50 | RAB11FIP1 | SMG1P1 | WSB2 |
| ITGAX | NBEAL2 | RAB20 | SNORA8 | XPO6 |
| ITPK1 | NCF1B | RAB27A | SORT1 | YKT6 |
| JUNB | NFKB2 | RAB5B | SPHK1 | ZC3H12A |
| KIAA0247 | NFKBIA | RAB5C | SPINT2 | ZFP36 |
| KIAA0368 | NFKBIB | RALGDS | SQSTM 1 | ZFP36L1 |
| KIAA0494 | NFKBID | RANGAP1 | SREBF2 | ZHX2 |
| KIAA1191 | NFKBIE | RAP2A | SRP54 | ZMIZ1 |
| KLF2 | NINJ1 | RBCK1 | SRRM2 | |
| KLF6 | NIPBL | RBM39 | SRXN1 | |
| LARP1 | NLRC5 | RELA | STK40 | |
| LGALS3 | NOTCH2NL | RHEB | STX11 | |
| LILRB2 | NR4A3 | RHOA | STX6 | |
| LILRB3 | NTAN1 | RHOB | STXBP2 | |
| LIMK2 | OGDH | RILPL2 | TAGAP | |
| LITAF | OSM | RIT1 | TAP1 | |
| LOC146880 | P2RY4 | RNASEK | TCF25 | |
| LOC729737 | PACSIN2 | RNF213 | TCIRG1 | |
| LPCAT1 | PDHX | RTN4 | TECPR2 | |
| LPIN1 | PDLIM7 | RXRA | TEX264 | |
| LSP1 | PER1 | RYBP | TLE3 | |
| LTBR | PFKL | SBNO2 | TMBIM6 | |
| MAF1 | PHF1 | SCARF1 | TMEM123 | |
| MAP4K4 | PIK3AP1 | SCD | TMEM134 | |
| MAP7D1 | PIK3R5 | SCYL1 | TMEM189 | |
| MAPKAPK2 | PILRA | SERINC1 | TNFAIP2 | |
| MARCKS | PIM2 | SH2B2 | TNFRSF14 | |
| MBOAT7 | PIM3 | SH3BP5 | TNIP1 | |

11. A test kit for detecting Parkinson's disease, the kit being used in a method according to any one of claims 1 to 9, and comprising an oligonucleotide which specifically hybridizes to the gene or a nucleic acid derived therefrom, or an antibody which recognizes an expression product of the gene.

12. A marker for detecting Parkinson's disease comprising at least one gene selected from the groups of genes shown in the following Tables 3-1 to 3-4 and the following Tables 6-1 and 6-2 or an expression product thereof.
| Table 3-1 | Table 3-2 | Table 3-3 | Table 3-4 |
|---|---|---|---|
| DUX4L4 | ACSS3 | SNORA24 | INO80D |
| GPBP1L1 | C1orf52 | SNORA33 | KIAA0232 |
| KIAA0930 | C5orf43 | SNORA34 | MAP7D1 |
| LOC100093631 | COA1 | SNORA49 | MLLT6 |
| LOC100506888 | FAM210B | SNORA50 | NCF1B |
| LOC349196 | FAM25B | SNORA52 | PRR24 |
| LOC401321 | FAM45A | SNORA57 | SDE2 |
| OR4F3 | GTF3C6 | SNORA6 | SLED1 |
| PQLC1 | HEATR5A | SNORA63 | SMG1P1 |
| REXO1L2P | IQCG | SNORA65 | TMEM167B |
| SNORA16A | ITPRIPL2 | SNORA67 | |
| SNORA24 | KIAA0240 | SNORA68 | |
| SNORA43 | KIAA1143 | SNORA71A | |
| SNORA50 | KRTAP1.5 | SNORA71B | |
| SNORA8 | KRTAP12.1 | SNORA71C | |
| TCEB3CL | KRTAP12.2 | SNORA71D | |
| TTC9 | KRTAP3.1 | SNORA74B | |
| USP17L5 | KRTAP5.3 | SNORA7B | |
| USP17L6P | LINC00675 | SNORA84 | |
| ZNF33A | LOC100505738 | SNORA9 | |
| SNORA53 | LOC550643 | SNORD15B | |
| | LOC646862 | SNORD17 | |
| | LRRC15 | TM4SF19 | |
| | MICALCL | TMEM179B | |
| | PDE12 | TMEM45B | |
| | PINLYP | TRMT6 | |
| | REXO1L2P | UTP6 | |
| | SCARNA12 | VSIG8 | |
| | SCARNA16 | WDR60 | |
| | SCARNA6 | WDR61 | |
| | SCARNA7 | WFDC12 | |
| | SF3B14 | WIBG | |
| | SLFN5 | ARHGAP30 | |
| | SLMO2 | C17orf107 | |
| | SMIM5 | C22orf13 | |
| | SNHG9 | FAM100B | |
| | SNORA10 | FAM193B | |
| | SNORA14B | FAM210A | |
| | SNORA16A | FAM53C | |
| | SNORA21 | GPR108 | |
| | SNORA23 | GRAMD1A | |
| Table 6-1 | Table 6-2 | | |
|---|---|---|---|
| LOC100288069 | KRTAP5-3 | | |
| MESDC1 | LRRC15 | | |
| POLR2J3 | PINLYP | | |
| PQLC1 | SNORA16A | | |
| SNORA24 | SNORA24 | | |
| SNORA50 | SNORA52 | | |
| SNORA57 | SNORA63 | | |
| SNORA9 | SNORA68 | | |
| TRMT44 | SNORA71A | | |
| C14orf178 | SNORD15B | | |
| FAM100A | AADACL3 | | |
| FYTTD1 | ARHGAP30 | | |
| LGALSL | C17orf107 | | |
| NSFP1 | C1orf43 | | |
| SLMO2 | C22orf13 | | |
| SNORA53 | CCDC86 | | |
| SREK1IP1 | CCSAP | | |
| SSU72 | CYTH4 | | |
| TMEM167B | FAM100B | | |
| | FAM193B | | |
| | GPR108 | | |
| | GRAMD1A | | |
| | KIAA0494 | | |
| | KIAA1191 | | |
| | LOC729737 | | |
| | MAP7D1 | | |
| | MLLT6 | | |
| | NCF1B | | |
| | POU5F1P3 | | |
| | SMG1P1 | | |

13. The marker for detecting Parkinson's disease according to claim 12, wherein the marker comprises at least one gene selected from the group of 4 genes consisting of SNORA16A, SNORA24, SNORA50 and REXO1L2P or an expression product thereof.
